# EUROPEAN PATENT APPLICATION

(11) **EP 3 098 326 A1**
(43) Date of publication of application: **30.11.2016**
(21) Application number: 15305798.9
(22) Date of filing: 27.05.2015
(51) Int. Cl.: C12Q 1/70

(54) **ISOTHERMAL AMPLIFICATION ASSAY FOR RAPID AND ACCURATE DETECTION OF HEMORRHAGIC FEVER VIRUSES IN CLINICAL SAMPLES**

(71) Applicant: INSTITUT PASTEUR, 75015 Paris (FR)
(72) Inventor: Manuguerra, Jean-Claude, 75018 Paris (FR); Escadafal, Camille, 75002 Paris (FR); Kwasiborski, Aurélia, 94270 Le Kremlin Bicêtre (FR); Vanhomwegen, Jessica, 75019 Paris (FR)
(74) Representative: Gevers & Orès

(57) **Abstract**

The invention relates to a method for diagnosing a Viral Hemorrhagic Fever by isothermal amplification of a target sequence of a Hemorrhagic Fever virus nucleic acid located within a gene which codes for a protein of the viral ribonucleocapsid. The invention relates also to a kit for performing the method.

SEQUENCE LISTING

<110>  INSTITUT PASTEUR

<120>  Isothermal amplification assay for rapid and accurate detection
       of Hemorrhagic Fever Viruses  in clinical samples

<130>  F226EP208

<160>  206

<170>  PatentIn version 3.5

<210>  1
<211>  6639
<212>  DNA
<213>  Ebola virus

<400>  1
atggctacac aacataccca atacccagac gctaggttat catcaccaat tgtattggac      60

caatgtgacc tagtcactag agcttgcggg ttatattcat catactccct taatccgcaa     120

ctacgcaact gtaaactccc gaaacatatc taccgtttga aatacgatgt aactgttacc     180

aagttcttga gtgatgtacc agtggcgaca ttgcccatag atttcatagt cccagttctt     240

ctcaaggcac tgtcaggcaa tggattctgt cctgttgagc cgcggtgcca acagttctta     300

gatgaaatca ttaagtacac aatgcaagat gctctcttct tgaaatatta tctcaaaaat     360

gtgggtgctc aagaagactg tgttgatgaa cactttcaag agaaaatctt atcttcaatt     420

cagggcaatg aatttttaca tcaaatgttt ttctggtatg atctggctat tttaactcga     480

aggggtagat taaatcgagg aaactctaga tcaacatggt ttgttcatga tgatttaata     540

gacatcttag gctatgggga ctatgttttt tggaagatcc caatttcaat gttaccactg     600

aacacacaag gaatccccca tgctgctatg gactggtatc aggcatcagt attcaaagaa     660

gcggttcaag ggcatacaca cattgtttct gtttctactg ccgacgtctt gataatgtgc     720

aaagatttaa ttacatgtcg attcaacaca actctaatct caaaaatagc agagattgag     780

gatccagttt gttctgatta tcccaatttt aagattgtgt ctatgcttta ccagagcgga     840

gattacttac tctccatatt agggtctgat gggtataaaa ttattaagtt cctcgaacca     900

ttgtgcttgg ccaaaattca attatgctca aagtacactg agaggaaggg ccgattctta     960

acacaaatgc atttagctgt aaatcacacc ctagaagaaa ttacagaaat gcgtgcacta    1020

aagccttcac aggctcaaaa gatccgtgaa ttccatagaa cattgataag gctggagatg    1080

acgccacaac aactttgtga gctattttcc attcaaaaac actgggggca tcctgtgcta    1140

catagtgaaa cagcaatcca aaaagttaaa aaacatgcta cggtgctaaa agcattacgc    1200

cctatagtga ttttcgagac atactgtgtt tttaaatata gtattgccaa acattatttt    1260

gatagtcaag gatcttggta cagtgttact tcagatagga atctaacacc gggtcttaat    1320

tcttatatca aaagaaatca attccctccg ttgccaatga ttaaagaact actatgggaa 1380

ttttaccacc ttgaccaccc tccacttttc tcaaccaaaa ttattagtga cttaagtatt 1440

tttataaaag acagagctac cgcagtagaa aggacatgct gggatgcagt attcgagcct 1500

aatgttctag gatataatcc acctcacaaa tttagtacta aacgtgtacc ggaacaattt 1560

ttagagcaag aaaacttttc tattgagaat gttctttcct acgcacaaaa actcgagtat 1620

ctactaccac aatatcggaa cttttctttc tcattgaaag agaaagagtt gaatgtaggt 1680

agaaccttcg gaaaattgcc ttatccgact cgcaatgttc aaacactttg tgaagctctg 1740

ttagctgatg gtcttgctaa agcatttcct agcaatatga tggtagttac ggaacgtgag 1800

caaaaagaaa gcttattgca tcaagcatca tggcaccaca caagtgatga ttttggtgaa 1860

catgccacag ttagagggag tagctttgta actgatttag agaaatacaa tcttgcattt 1920

agatatgagt ttacagcacc ttttatagaa tattgcaacc gttgctatgg tgttaagaat 1980

gtttttaatt ggatgcatta tacaatccca cagtgttata tgcatgtcag tgattattat 2040

aatccaccac ataacctcac actggagaat cgagacaacc cccccgaagg gcctagttca 2100

tacaggggtc atatgggagg gattgaagga ctgcaacaaa aactctggac aagtatttca 2160

tgtgctcaaa tttctttagt tgaaattaag actggtttta agttacgctc agctgtgatg 2220

ggtgacaatc agtgcattac tgttttatca gtcttcccct tagagactga cgcagacgag 2280

caggaacaga gcgccgaaga caatgcagcg agggtggccg ccagcctagc aaaagttaca 2340

agtgcctgtg gaatcttttt aaaacctgat gaaacatttg tacattcagg ttttatctat 2400

tttggaaaaa aacaatattt gaatggggtc caattgcctc agtcccttaa aacggctaca 2460

agaatggcac cattgtctga tgcaattttt gatgatcttc aagggaccct ggctagtata 2520

ggcactgctt ttgagcgatc catctctgag acacgacata tctttccttg caggataacc 2580

gcagctttcc atacgttttt ttcggtgaga atcttgcaat atcatcatct cgggttcaat 2640

aaaggttttg accttggaca gttaacactc ggcaaacctc tggatttcgg aacaatatca 2700

ttggcactag cggtaccgca ggtgcttgga gggttatcct tcttgaatcc tgagaaatgt 2760

ttctaccgga atctaggaga tccagttacc tcaggcttat tccagttaaa aacttatctc 2820

cgaatgattg agatggatga tttattctta cctttaattg cgaagaaccc tgggaactgc 2880

actgccattg actttgtgct aaatcctagc ggattaaatg tccctgggtc gcaagactta 2940

acttcatttc tgcgccagat tgtacgcagg accatcaccc taagtgcgaa aaacaaactt 3000

attaatacct tatttcatgc gtcagctgac ttcgaagacg aaatggtttg taaatggcta 3060

ttatcatcaa ctcctgttat gagtcgtttt gcggccgata tcttttcacg cacgccgagc 3120

gggaagcgat tgcaaattct aggatacctg gaaggaacac gcacattatt agcctctaag 3180

atcatcaaca ataatacaga gacaccggtt ttggacagac tgaggaaaat aacattgcaa 3240

aggtggagcc tatggtttag ttatcttgat cattgtgata atatcctggc ggaggcttta 3300

acccaaataa cttgcacagt tgatttagca cagattctga gggaatattc atgggctcat 3360

attttagagg gaagacctct tattggagcc acactcccat gtatgattga gcaattcaaa 3420

gtgttttggc tgaaacccta cgaacaatgt ccgcagtgtt caaatgcaaa gcaaccaggt 3480

gggaaaccat tcgtgtcagt ggcagtcaag aaacatattg ttagtgcatg gccgaacgca 3540

tcccgaataa gctggactat cggggatgga atcccataca ttggatcaag gacagaagat 3600

aagataggac aacctgctat taaaccaaaa tgtccttccg cagccttaag agaggccatt 3660

gaattggcgt cccgtttaac atgggtaact caaggcagtt cgaacagtga cttgctaata 3720

aaaccatttt tggaagcacg agtaaattta agtgttcaag aaatacttca aatgacccct 3780

tcacattact caggaaatat tgttcacagg tacaacgatc aatacagtcc tcattctttc 3840

atggccaatc gtatgagtaa ttcagcaacg cgattgattg tttctacaaa cactttaggt 3900

gagttttcag gaggtggcca gtctgcacgc gacagcaata ttattttcca gaatgttata 3960

aattatgcag ttgcactgtt cgatattaaa tttagaaaca ctgaggctac agatatccaa 4020

tataatcgtg ctcaccttca tctaactaag tgttgcaccc gggaagtacc agctcagtat 4080

ttaacataca catctacatt ggatttagat ttaacaagat accgagaaaa cgaattgatt 4140

tatgacagta atcctctaaa aggaggactc aattgcaata tctcattcga taatccattt 4200

ttccaaggta aacggctgaa cattatagaa gatgatctta ttcgactgcc tcacttatct 4260

ggatgggagc tagccaagac catcatgcaa tcaattattt cagatagcaa caattcatct 4320

acagacccaa ttagcagtgg agaaacaaga tcattcacta cccatttctt aacttatccc 4380

aagataggac ttctgtacag ttttggggcc tttgtaagtt attatcttgg caatacaatt 4440

cttcggacta agaaattaac acttgacaat tttttatatt acttaactac tcaaattcat 4500

aatctaccac atcgctcatt gcgaatactt aagccaacat tcaaacatgc aagcgttatg 4560

tcacggttaa tgagtattga tcctcatttt tctatttaca taggcggtgc tgcaggtgac 4620

agaggactct cagatgcggc caggttattt ttgagaacgt ccatttcatc ttttcttaca 4680

tttgtaaaag aatggataat taatcgcgga acaattgtcc ctttatggat agtatatccg 4740

ctagagggtc aaaacccaac acctgtgaat aattttctct atcagatcgt agaactgctg 4800

gtgcatgatt catcaagaca acaggctttt aaaactacca taagtgatca tgtacatcct 4860

cacgacaatc ttgtttacac atgtaagagt acagccagca atttcttcca tgcatcattg 4920

gcgtactgga ggagcagaca cagaaacagc aaccgaaaat acttggcaag agactcttca 4980

actggatcaa gcacaaacaa cagtgatggt catattgaga gaagtcaaga acaaaccacc 5040

agagatccac atgatggcac tgaacggaat ctagtcctac aaatgagcca tgaaataaaa 5100

agaacgacaa ttccacaaga aaacacgcac cagggtccgt cgttccagtc ctttctaagt 5160

gactctgctt gtggtacagc aaatccaaaa ctaaatttcg atcgatcgag acacaatgtg 5220

aaatttcagg atcataactc ggcatccaag agggaaggtc atcaaataat ctcacaccgt 5280

ctagtcctac ctttctttac attatctcaa gggacacgcc aattaacgtc atccaatgag 5340

tcacaaaccc aagacgagat atcaaagtac ttacggcaat tgagatccgt cattgatacc 5400

acagtttatt gtagatttac cggtatagtc tcgtccatgc attacaaact tgatgaggtc 5460

ctttgggaaa tagagagttt caagtcggct gtgacgctag cagagggaga aggtgctggt 5520

gccttactat tgattcagaa ataccaagtt aagaccttat ttttcaacac gctagctact 5580

gagtccagta tagagtcaga aatagtatca ggaatgacta ctcctaggat gcttctacct 5640

gttatgtcaa aattccataa tgaccaaatt gagattattc ttaacaactc agcaagccaa 5700

ataacagaca taacaaatcc tacttggttt aaagaccaaa gagcaaggct acctaagcaa 5760

gtcgaggtta taaccatgga tgcagagaca acagagaata taaacagatc gaaattgtac 5820

gaagctgtat ataaattgat cttacaccat attgatccta gcgtattgaa agcagtggtc 5880

cttaaagtct ttctaagtga tactgagggt atgttatggc taaatgataa tttagccccg 5940

ttttttgcca ctggttattt aattaagcca ataacgtcaa gtgctagatc tagtgagtgg 6000

tatctttgtc tgacgaactt cttatcaact acacgtaaga tgccacacca aaaccatctc 6060

agttgtaaac aggtaatact tacggcattg caactgcaaa ttcaacgaag cccatactgg 6120

ctaagtcatt taactcagta tgctgactgt gagttacatt taagttatat ccgccttggt 6180

tttccatcat tagagaaagt actataccac aggtataacc tcgtcgattc aaaaagaggt 6240

ccactagtct ctatcactca gcacttagca catcttagag cagagattcg agaattaact 6300

aatgattata atcaacagcg acaaagtcgg actcaaacat atcactttat tcgtactgca 6360

aaaggacgaa tcacaaaact agtcaatgat tatttaaaat tctttcttat tgtgcaagca 6420

ttaaaacata atgggacatg gcaagctgag tttaagaaat taccagagtt gattagtgtg 6480

tgcaataggt tctaccatat tagagattgc aattgtgaag aacgtttctt agttcaaacc 6540

ttatatttac atagaatgca ggattctgaa gttaagctta tcgaaaggct gacagggctt 6600

ctgagtttat ttccggatgg tctctacagg tttgattga 6639

<210> 2
<211> 206
<212> DNA
<213> artificial sequence

<220>
<223> synthetic polynucleotide

<400> 2
gaatgtaggt agaaccttcg gaaaattgcc ttatccgact cgcaatgttc aaacactttg 60

tgaagctctg ttagctgatg gtcttgctaa agcatttcct agcaatatga tggtagttac 120

ggaacgtgag caaaaagaaa gcttattgca tcaagcatca tggcaccaca caagtgatga 180

ttttggtgaa catgccacag ttagag 206

<210> 3
<211> 271
<212> DNA
<213> artificial sequence

<220>
<223> synthetic polynucleotide

<400> 3
atccgactcg caatgttcaa acactttgtg aagctctgtt agctgatggt cttgctaaag 60

catttcctag caatatgatg gtagttacgg aacgtgagca aaaagaaagc ttattgcatc 120

aagcatcatg gcaccacaca agtgatgatt ttggtgaaca tgccacagtt agagggagta 180

gctttgtaac tgatttagag aaatacaatc ttgcatttag atatgagttt acagcacctt 240

ttatagaata ttgcaaccgt tgctatggtg t 271

<210> 4
<211> 303
<212> DNA
<213> artificial sequence

<220>
<223> synthetic polynucleotide

<400> 4
gaatgtaggt agaaccttcg gaaaattgcc ttatccgact cgcaatgttc aaacactttg 60

tgaagctctg ttagctgatg gtcttgctaa agcatttcct agcaatatga tggtagttac 120

ggaacgtgag caaaaagaaa gcttattgca tcaagcatca tggcaccaca caagtgatga 180

ttttggtgaa catgccacag ttagagggag tagctttgta actgatttag agaaatacaa 240

tcttgcattt agatatgagt ttacagcacc ttttatagaa tattgcaacc gttgctatgg 300

tgt 303

<210> 5
<211> 6633
<212> DNA
<213> Taï Forest Ebolavirus

<400> 5
atggctacac aacatacgca atatccagac gcaaggttat catcacctat agttttagat 60

cagtgtgatc ttgtcactcg tgcttgtgga ttgtattccg catactcctt aaatccccaa 120

ctaaagaact gtagactacc gaaacatata taccgactaa aatatgacac cactgttaca 180

gagtttttga gtgatgtgcc ggtagcaaca ttgccagcgg attttttagt acctacattt 240

cttaggactc tatcaggaaa tggttcttgt ccaattgatc caaaatgcag tcaattttta 300

gaagaaattg tcaattatac tctacaagat attcgcttcc taaactatta cctcaatcga 360

gccggagtgc ataacgatca tgtggatagg gattttggac aaaaaattcg caatctaatt 420

tgcgacaatg aggttttaca tcaaatgttt cactggtatg atcttgcaat tctagcacgt 480

agagggcgac taaatagagg gaataatcgc tcaacatggt ttgcaagtga taatttggta 540

gatatcctag gttatggaga ttatattttt tggaaaatac cattatcact actaccagtg 600

gatacacaag gcctcccaca tgcagccaag gactggtatc atgaatcggt tttcaaggag 660

gctattcaag gccatacaca catcgtgtcc atctctacag cagatgtctt aatcatgtgt 720

aaggacataa tcacctgtcg atttaatact ttactgattg ctgctgtggc aaatctagag 780

gattcagttc attcagatta ccctttacca gaaacagtgt ctgacctata caaagcagga 840

gattatttaa tctcattgct aggatcagaa ggttacaaag tcataaaatt ccttgagccg 900

ttatgcttag caaagatcca actctgctca aattacactg agaggaaagg aagattcctc 960

actcaaatgc atttagctgt aaatcataca cttgaggaac ttacagggtc ccgagaatta 1020

aggccacaac agattcggaa ggtaagggaa ttccatcaaa tgctgataaa ccttaaggca 1080

actcctcaac aactctgtga gttgttttca gtgcaaaagc attgggggca ccctgtcttg 1140

catagcgaaa aggctatcca aaaagtaaag aagcatgcaa cagtgataaa agcattgcgc 1200

ccaataataa tctttgaaac atattgtgtg tttaaataca gcattgcaaa acattatttt 1260

gatagtcagg gtacgtggta cagtgtgact tctgacagat gcttaacacc aggcctttcc 1320

tcttacatca aaagaaacca atttcctcca ctacctatga tcaaagaact tttgtgggaa 1380

ttttatcact tagatcatcc tccgttattc tccaccaaag tgattagtga tttgagtatc 1440

tttattaaag atcgtgctac tgcagtcgag aaaacatgct gggacgcagt ttttgaaccc 1500

aatgttcttg gttataaccc accgaataaa tttgctacaa aaagggtacc tgagcaattc 1560

cttgaacagg agaatttctc aatagagagt gtcctacatt atgctcaacg tctggaatat 1620

cttctcccgg agtaccggaa cttctctttt tcactcaagg agaaggagtt aaacattgga 1680

cgagcttttg ggaaattgcc atatccaaca cgcaatgttc aaactctgtg cgaagctttg 1740

ttagcagatg gtttggcgaa agcattccca agcaatatga tggttgtgac agagcgcgag 1800

caaaaagaaa gccttttgca tcaagcgtct tggcatcaca caagtgatga ttttggtgag 1860

aatgctactg ttagaggcag tagttttgta acagacttgg aaaaatacaa tttagcattc 1920

cgatatgagt ttacagctcc ttttattgaa tactgtaatc gttgttacgg tgtaagaaat 1980

ttgtttaatt ggatgcacta cactatacca cagtgttata tacatgtgag tgattattat 2040

aaccccccac atggagtctc tctcgaaaac cgagaaaatc caccagaagg tccaagctct 2100

taccgtggtc atctaggcgg gattgaggga cttcaacaaa aactctggac aagcatctca 2160

tgtgcacaga tttcattagt tgaaatcaaa accggtttta aactgcgatc tgcggtaatg 2220

ggtgacaatc aatgtataac tgtactctct gtatttcccc tcgaaactga gtctagtgag 2280

caagaattaa gttctgaaga taatgccgct agagtagctg ctagcttagc aaaagtcaca 2340

agtgcctgcg gcatcttttt aaaacctgat gaaacttttg ttcactcagg tttcatttat 2400

tttggcaaaa aacaatattt gaatggagta caattacctc aatcactgaa aactgctact 2460

agaattgcac ccttgtcaga tgctatcttt gatgatcttc aagggacact agctagcata 2520

ggcacggctt ttgaaagatc tatctccgaa actaggcacg tagtcccttg tagagtagca 2580

gctgcattcc ataccttttt ttccgtaaga atcttacaat atcatcatct tggcttcaac 2640

aagggaacag acctgggtca attgtcatta agcaagccat tagattttgg aactataact 2700

ttggccttgg cagtaccaca agtcttgggt ggcttatcat tcctaaatcc agaaaaatgt 2760

ttttatagaa atctgggtga tcctgttact tcagggctgt ttcagctcaa gacatatctt 2820

caaatgatcc acatggatga tttgttttta cctttgatcg caaagaaccc agggaactgt 2880

agcgcaattg actttgtgtt aaaccctagt gggttaaacg taccggggtc acaggatttg 2940

acatccttcc tacgtcagat agtgcgccga acaattactc taagtgctaa aaataaatta 3000

ataaacactt tgttccattc ttctgctgat ttagaagatg aaatggtttg caaatggttg 3060

ctttcttcta caccagtcat gagtaggttt gccgccgata tattttctcg cactcccagt 3120

gggaaacgtt tacagatctt aggttacctt gaagggacta gaacattgtt agcctctaaa 3180

attataaatc ataatactga gacacctatc ctagatcgat tgaggaaaat tacgctgcaa 3240

aggtggagcc tgtggtttag ttatctcgac cactgtgatc aagttctggc tgatgcccta 3300

actcagataa cctgcactgt ggacttagca cagattcttc gcgagtacac ctgggcacac 3360

atactagagg gaaggcagct cattggagca acacttcctt gtatactaga acaactaaat 3420

gtcatctggc tcaaaccata tgagcattgc cctaaatgtg caaagtcagc aaaccctaaa 3480

ggggaacctt ttgtttctat tgcaattaaa aaacatgtag taagtgcttg gcctgatcaa 3540

tcacgactta gttggacaat tggagatggc atcccttata tcggatctcg aacagaggat 3600

aagattgggc agccagccat caaaccaaaa tgcccttcag cagccttacg tgaagcaatt 3660

gagttgacat caagattgac ttgggttact caaggtggag caaacagcga cttactagtt 3720

aaacccttca tagaagcacg agtaaattta agcgtacagg aaattctcca aatgacacct 3780

tctcattact ccggcaacat tgtgcatcga tataatgatc aatatagtcc acactcattt 3840

atggcaaata ggatgagtaa ttctgctact aggttagttg tttcgacaaa cactcttgga 3900

gaattttcag gaggaggtca gtcagcaaga gatagtaata ttatcttcca gaatgtcatt 3960

aattttgctg ttgcactttt tgatctacga tttaggaacg tggctacttc ttctatacaa 4020

catcatcggg ctcatcttca tttgtcaaag tgttgcacgc gagaggttcc agcccaatat 4080

ttagtttata catcaacatt gccattggac cttacacggt atcgggataa tgagttgatt 4140

tacgatgaca atccattaag aggtggttta aattgcaatc tttcttttga taatccgctt 4200

ttcaagggcc agagacttaa cataattgaa gaagacttga ttagactacc ttacttatca 4260

ggatgggagc tagctaaaac tgttatccaa tctataattt ctgacagcaa caattcatca 4320

acggatccaa tcagtagtgg ggaaacacga tcattcacca ctcacttctt gacatatcct 4380

aagattggac tactatatag ttttggtgca ctcatcagtt attatctagg caacaccatt 4440

attagaacca aaaaattgac tcttaacaac ttcatatatt acctagctac tcaaatacat 4500

aatttacctc atcgctcgtt gagaatcctt aaacctactt tgaaacacgc tagtgttatc 4560

tcgagattaa taagtattga ctctcacttc tcaatttata ttggaggaac tgctggtgat 4620

cgaggacttt ccgatgcggc aagattgttt cttagaactg ccattactgt cttccttcaa 4680

ttcgttagaa agtggatagt tgaacgcaag acagctattc cactgtgggt catctaccct 4740

ctagaaggtc aaagtcctag tccgatcaac agttttctac accacgtcat cgcattgttg 4800

caacatgagt cctcccacga tcatgtttgt gctgcagaag cccacagtcg agtggagaca 4860

tttgataatt tagtttatat gtgtaaaagc acagcaagta acttctttca tgcttcatta 4920

gcatactgga gaagtcgatc taaaaatcaa gacaaaagag agatgacaaa gatattatct 4980

ttgacgcaaa cggaaaagaa aaattcattc ggctatacag cacatccaga aagcactgct 5040

gttcttggtt ccctccagac cagccttgct ccacctccat ctgctgacga ggctacatat 5100

gataggaaaa acaaagtttt gaaagcttcc agacctggca agtattccca gaatacaacc 5160

aaagccccac ccaaccaaac cagttgtcgc gatgtatctc ccaatatcac aggcacagat 5220

gggtgccctt ctgccaatga gggttctaac agcaataaca ataatttagt ctcgcacaga 5280

attgtactgc cgttttttac attgtctcat aattataacg aaagaccctc tatcagaaag 5340

tctgagggga caacagagat tgtaaggctt actcggcagc tgagggcaat accagacacc 5400

acaatatatt gccgcttcac gggaatagtt tcttcaatgc actataagct cgatgaagtc 5460

ctttgggaat ttgataattt taagtctgct ataacacttg ccgaaggtga aggttcgggt 5520

gcattactct tattacaaaa atataaagta gaaaccttgt tttttaatac actagccaca 5580

gaacacagca ttgaagcaga aattatttct ggaataacta caccaagaat gcttctccct 5640

attatgtcta ggttccatgg tggacaaata aaagtcactt taaacaattc tgcaagccag 5700

attaccgata ttactaatcc aagttggttg gcagaccaaa aatctaggat ccctaagcaa 5760

gtagagatta taaccatgga tgctgaaaca acagaaaaca ttaatcggtc aaaattgtac 5820

gaagcagtcc aacagctgat tgtctcacat attgatccga atgcactcaa agttgtggtt 5880

cttaaagttt tcttaagtga cattgatgga atcctatggc tgaatgataa ccttacccct 5940

ttgtttgggc tgggttactt gatcaagccg atcacctcta gcccaaaatc tagtgagtgg 6000

tacctatgtc tctcaaacct tctttcaact tcaagacgat tacctcatca gagtcatact 6060

acttgcatgc atgttattca aacagcactc cagctacaaa ttcagaggag ctcatattgg 6120

cttagccacc ttgtccagta tgccaatcat aatttgcatt tagattatat taatctcggt 6180

ttcccttcat tggagagggt tttataccat agatacaatt tagtcgattc tcagaaaggc 6240

cctttgactt ccattgtcca acatctagcg cacctgcaga ccgagattag ggagttggtt 6300

aatgactata atcaacaaag acaaagtcga acccaaacat atcatttcat taaaacaata 6360

aaaggtcgta ttacaaaatt ggtaaatgat taccttaagt tctttctaat aatacaagcc 6420

ttaaagcaca attgcacatg gcaagaggaa ctaagagctc ttccagatct aattagtgtc 6480

tgcactcgat tctatcatac tcgaaactgt tcatgtgaaa accggttcct agtacagact 6540

ttatacttat cacgcatgca ggattcggaa atcaaactaa tagatagatt gaccggcctt 6600

cttagtctat gtccaaatgg tttttttcgg taa 6633

<210> 6
<211> 215
<212> DNA
<213> artificial sequence

<220>
<223> synthetic polynucleotide

<400> 6
gcctgatcaa tcacgactta gttggacaat tggagatggc atcccttata tcggatctcg 60

aacagaggat aagattgggc agccagccat caaaccaaaa tgcccttcag cagccttacg 120

tgaagcaatt gagttgacat caagattgac ttgggttact caaggtggag caaacagcga 180

cttactagtt aaacccttca tagaagcacg agtaa 215

<210> 7
<211> 287
<212> DNA
<213> artificial sequence

<220>
<223> synthetic polynucleotide

<400> 7
agaactgcca ttactgtctt ccttcaattc gttagaaagt ggatagttga acgcaagaca 60

gctattccac tgtgggtcat ctaccctcta gaaggtcaaa gtcctagtcc gatcaacagt 120

tttctacacc acgtcatcgc attgttgcaa catgagtcct cccacgatca tgtttgtgct 180

gcagaagccc acagtcgagt ggagacattt gataatttag tttatatgtg taaaagcaca 240

gcaagtaact tctttcatgc ttcattagca tactggagaa gtcgatc 287

<210>  8
<211>  329
<212>  DNA
<213>  artificial sequence

<220>
<223>  synthetic polynucleotide

<400>  8
tctcgcacag aattgtactg ccgtttttta cattgtctca taattataac gaaagaccct      60

ctatcagaaa gtctgagggg acaacagaga ttgtaaggct tactcggcag ctgagggcaa     120

taccagacac cacaatatat tgccgcttca cgggaatagt ttcttcaatg cactataagc     180

tcgatgaagt cctttgggaa tttgataatt ttaagtctgc tataacactt gccgaaggtg     240

aaggttcggg tgcattactc ttattacaaa aatataaagt agaaaccttg ttttttaata     300

cactagccac agaacacagc attgaagca                                      329


<210>  9
<211>  6633
<212>  DNA
<213>  Sudan Ebolavirus

<400>  9
atgatggcta cccaacatac acaatatcct gatgcaagat tgtcttcccc aattgtctta      60

gaccaatgtg acctagtgac aagagcatgt ggactttact ctgagtattc gctgaaccct     120

aaactaaaga catgccgttt accgaaacat atctatagat taaaatatga cactattgtt     180

ttacgattta ttagtgatgt ccctgtagct acaatcccaa tagactacat tgctccgatg     240

ttaataaatg ttctggcaga tagtaaaaat gtaccattgg aacctccctg cttgagtttc     300

ttggatgaaa tagtcaatta taccgtgcag gatgcagcct tccttaatta ttacatgaat     360

cagattaaaa cacaggaagg agtaattaca gatcaattaa aacagaacat tcgtagggtc     420

attcacaaaa acagatatct atctgctcta ttcttctggc atgatcttgc catcctcacc     480

cgtcgaggga gaatgaaccg aggaaatgtg cgctccactt ggtttgtaac gaatgaggtt     540

gttgacattc taggatatgg tgattatatc ttctggaaga tccctattgc tctattacca     600

atgaacacag ctaatgttcc acatgcatca actgactggt accaacctaa tatcttcaag     660

gaggctattc aaggacacac acatattatt tcagtctcta cagccgaggt ccttattatg     720

tgtaaggatc ttgtcacaag tcgttttaat acccttctga ttgctgagtt agccaggttg     780

gaagatccag tgtctgctga ttatccacta gtagataata ttcaatctct gtataacgca     840

ggagactacc tgttgtccat attgggatca gaggggtaca aaataatcaa atatctcgaa     900

cctctgtgtt tggctaagat tcaactatgt tcccaatata cagaacgaaa gggcggtttt     960

ttaacccaga tgcatcttgc agttattcag acattgcgtg aactcctcct taatagaggg    1020

ttgaaaaaat cacaattgtc taaaatccgc gagtttcacc aactgttgct cagactccga 1080

tctacaccac aacaattatg tgaattattt tcaatccaaa aacactgggg ccacccagtt 1140

ctgcatagtg aaaaggccat ccaaaaggtt aaaaatcatg caacagttct aaaggcattg 1200

cggccgatta tcatctttga aacgtattgt gtattcaagt atagtgttgc aaaacatttc 1260

tttgatagtc aaggcacttg gtacagtgtg atatcagacc gatgtttaac gccgggattg 1320

aattcctaca ttaggcgaaa tcaattccct ccacttccaa tgatcaaaga tcttttatgg 1380

gaattttacc atttggatca tcctccatta ttctccacga agatcattag tgacctcagc 1440

attttcatta aagaccgcgc aacagcagtt gaacaaacct gttgggatgc agtttttgag 1500

cctaacgttt tgggctacag tccaccttat cgattcaata ccaaacgtgt acctgaacaa 1560

ttcctggagc aagaggattt ttctattgag agtgtcttac aatacgccca agaacttagg 1620

tacttattgc cccagaatcg aaatttttct ttttcattga aggaaaaaga attaaatgtt 1680

ggtaggacat ttggaaaatt gccttattta accaggaatg tccaaaccct ctgcgaagca 1740

ttacttgcag atggtttggc taaagccttt ccaagcaata tgatggttgt cacagagagg 1800

gaacaaaagg agagcctcct tcaccaagca tcctggcacc atacaagtga tgatttcgga 1860

gagcatgcca cagttcgtgg aagtagtttt gtcacagacc tggaaaaata caatctggcc 1920

ttcaggtatg aattcacagc tcccttcatc aaatattgca accaatgcta tggggttcgc 1980

aatgtctttg attggatgca cttcctaatt ccgcaatgtt acatgcatgt tagtgattat 2040

tataacccac cacataatgt aaccttagag aatagggaat atccccccga aggaccaagt 2100

gcttatagag gccaccttgg cggtattgag gggcttcaac aaaagttatg gactagtatc 2160

tcatgtgctc aaatctcatt ggtagagatc aagaccgggt tcaaattgcg atcagcagtc 2220

atgggggata atcaatgtat tacagtatta tcagtctttc cactagaatc tagtccgaat 2280

gagcaggaga gatgcgcaga agacaatgca gccagagtgg ctgctagctt ggccaaagtc 2340

acaagtgcct gtgggatatt cctcaagcct gatgagactt tcgtacactc aggctttatc 2400

tattttggca aaaagcaata cttgaacgga attcaattac ctcaatcact caagacagca 2460

gctaggatgg cccctctctc agatgcaatt tttgatgact tgcaaggtac acttgccagt 2520

ataggaactg cctttgagcg atcaatctcc gaaactagac atattttacc atgccgtgtt 2580

gcagctgcct ttcatacata tttctctgtt cggatcttac aacatcatca ccttggtttc 2640

cataagggtt cagaccttgg acaattggca atcaataaac ctcttgattt cgggaccatt 2700

gcactatcct tagcagttcc tcaggtattg ggtggattat ccttcctaaa tccagaaaag 2760

tgcctttatc gcaacttggg tgatcctgta acttcaggcc tatttcagtt gaagcattat 2820

ctgtcaatgg tgggtatgag tgatatcttt catgcactta ttgcaaaaag cccagggaat 2880

tgtagcgcaa ttgactttgt tctaaaccca ggcgggttaa atgtccctgg atcacaggat 2940

ttaacatctt tccttcgtca gattgtcaga aggagtatca cactttcggc aaggaacaag 3000

ttaatcaaca cgttatttca cgcttctgca gatcttgaag acgaattagt atgtaaatgg 3060

ttactttctt caacgcccgt gatgagccgt tttgcagccg atattttctc acgaacacca 3120

agcgggaaaa gattacaaat cttgggatac ctcgagggaa ccagaacttt attagcatcc 3180

aaaatgataa gcaataatgc agagacacca atcttggaga ggctcagaaa aataacactt 3240

caaagatgga atctatggtt tagttaccta gaccattgtg acccagcttt aatggaagca 3300

attcaaccaa ttaagtgtac tgttgatatt gctcaaattc ttagagaata ctcctgggct 3360

catatccttg atggtagaca gttaataggg gcaacactgc catgtatacc tgagcagttc 3420

caaaccacat ggttaaaacc ttacgagcaa tgtgtggaat gttcatccac aaacaattct 3480

agtccatatg tatcagttgc attaaaaagg aacgtggtta gtgcttggcc tgatgcatct 3540

agattggggt ggacgattgg tgatgggatt ccctacatag gctcaagaac tgaggacaaa 3600

ataggtcagc ccgctattaa gccgaggtgc ccatcagctg cattaagaga agctattgaa 3660

ttgacctcta ggttgacctg ggtcactcaa ggtagtgcaa acagcgatca gttaattcgc 3720

ccttttcttg aggcaagagt aaacttgagt gtacaagaga ttcttcaaat gacccctcca 3780

cattactccg gtaatattgt gcatcggtat aatgatcagt atagccctca ctcctttatg 3840

gctaaccgca tgagtaacac agcaacgcgc ttgatggtat ctaccaacac actaggagag 3900

ttttccggag ggggtcaggc tgcacgtgat agcaacatta tatttcaaaa tgtgattaac 3960

tttgcagtgg ccttgtatga cattaggttt cggaacactt gtacatcttc tattcaatat 4020

cacagggccc atattcacct gacgaattgt tgtacgaggg aagtaccggc ccaatactta 4080

acatacacaa ccacgctaaa tctagatttg agtaagtacc gtaataatga actgatttat 4140

gattcagatc cactaagagg aggtctcaac tgcaacttat cgattgacag tcctttgatg 4200

aagggcccac gtttaaatat tattgaggat gacttaatac ggttgccaca tttatccggc 4260

tgggaattag caaaaacagt cttgcaatca ataatctctg atagtagcaa ttcatcaaca 4320

gatcccatta gcagcggtga aacaagatcc ttcacaaccc acttcttaac gtatcccaaa 4380

atagggcttc tatacagttt tggagccctc ataagttttt atttgggtaa tactattcta 4440

tgcacgaaaa agatcggact cacagaattt ctatactatc tccagaatca gatccacaac 4500

ttatcacata gatcccttcg aatcttcaaa ccgacattta gacactcaag tgtcatgtcc 4560

aggttgatgg atatagaccc caacttctca atatatattg gtgggactgc aggtgaccgt 4620

ggattatcgg acgctgcaag attatttctc cgaattgcaa tttcaacttt cttgagcttt 4680

gttgaggagt gggttatctt taggaaggca aacatcccac tatgggttat ctatcctctc 4740

gaaggccaac gctctgatcc tcctggcgaa tttttgaacc gagtaaaatc tctaattgtt 4800

gggactgaag atgataaaaa taaaggctct atactttcaa gatctggaga gaaatgctct 4860

tcaaatctag tttataattg caagagtaca gcaagcaatt ttttccatgc atcattggct 4920

tactggagag gtcgacatag acctaagaag actataggtg caactaacgc gacaacagct 4980

ccacatatca ttttgccact gggaaattct gatcgaccgc ctggcctaga ccttaatagg 5040

aacaatgata ctttcattcc taccagaatt aaacagatag tccaaggaga ctctagaaac 5100

gacagaacga ccaccacgag atttccaccc aaaagtaggt ccactccaac atcagcaacc 5160

gagcctccta caaaaatgta tgagggttcg acaacccacc aagggaaatt aacagataca 5220

catttggatg aggatcacaa tgccaaagag ttcccatcca atccgcatcg tttagtagta 5280

ccattcttta aattaacaaa agatggggaa tacagcatcg aaccttctcc tgaagaaagc 5340

cgcagtaata taaaagggtt acttcaacat ttaagaacca tggttgatac taccatatat 5400

tgtcgcttca ctggaattgt ttcatcaatg cattataagt tagatgaagt actatgggaa 5460

tataataaat ttgaatcagc tgtaacccta gcagaagggg agggttcagg tgccttacta 5520

ctgatccaaa aatacggcgt taagaagtta tttttgaata cacttgctac tgaacatagt 5580

attgagagtg aagtgatatc aggttacacc actccaagga tgctactccc aattatgcct 5640

aaaacacatc gtggtgagct agaggtcata ttaaataact cagctagtca aataactgat 5700

attacacatc gagattggtt ttcaaatcaa aaaaatagga ttccaaatga tgctgatatt 5760

attaccatgg atgctgaaac tacagaaaac ttagatcgtt ccagattata tgaagcagta 5820

tatacgatta tttgtaatca tatcaatcct aaaactttga aagtggtcat cttaaaagtc 5880

ttcctcagcg atttggatgg gatgtgctgg attaacaatt atcttgctcc tatgtttgga 5940

tcaggatatt taatcaaacc tataacatca agtgcaaagt caagtgagtg gtatttatgc 6000

ttatctaatc tactttcaac cttgagaact actcagcatc aaacccaggc aaactgtctc 6060

catgtcgtac aatgtgctct tcaacagcaa gtacaaagag ggtcatattg gctaagtcat 6120

cttaccaaat acaccacaag tagattgcac aatagttata ttgcatttgg ttttccttca 6180

ttagagaagg tcctatatca taggtataac cttgttgatt cgagaaatgg accattagtt 6240

tctataacga gacaccttgc cctcctccaa actgagatcc gggagttggt aactgattat 6300

aatcagctgc gacaaagtcg aacccagact tatcatttca taaaaacatc caagggacgg 6360

ataactaaac tagtgaatga ttatctaaga tttgagttgg ttatacgggc tcttaaaaat 6420

aattctacat ggcaccatga gttatacttg ctaccagaac ttataggtgt ttgccatcga 6480

tttaatcata cacgtaactg tacatgcagt gaaaggttcc tggttcaaac tttatatcta 6540

caccgaatga gtgatgctga gataaaactt atggaccggc tcaccagcct agtcaatatg 6600

tttcctgaag gtttcaggtc tagttcagtc taa 6633

<210>  10
<211>  320
<212>  DNA
<213>  artificial sequence

<220>
<223>  synthetic polynucleotide

<400>  10
aacgaatgag gttgttgaca ttctaggata tggtgattat atcttctgga agatccctat      60

tgctctatta ccaatgaaca cagctaatgt tccacatgca tcaactgact ggtaccaacc     120

taatatcttc aaggaggcta ttcaaggaca cacacatatt atttcagtct ctacagccga     180

ggtccttatt atgtgtaagg atcttgtcac aagtcgtttt aatacccttc tgattgctga     240

gttagccagg ttggaagatc cagtgtctgc tgattatcca ctagtagata atattcaatc     300

tctgtataac gcaggagact                                                 320


<210>  11
<211>  298
<212>  DNA
<213>  artificial sequence

<220>
<223>  artificial sequence

<400>  11
cctctgcgaa gcattacttg cagatggttt ggctaaagcc tttccaagca atatgatggt      60

tgtcacagag agggaacaaa aggagagcct ccttcaccaa gcatcctggc accatacaag     120

tgatgatttc ggagagcatg ccacagttcg tggaagtagt tttgtcacag acctggaaaa     180

atacaatctg gccttcaggt atgaattcac agctcccttc atcaaatatt gcaaccaatg     240

ctatggggtt cgcaatgtct ttgattggat gcacttccta attccgcaat gttacatg       298


<210>  12
<211>  257
<212>  DNA
<213>  artificial sequence

<220>
<223>  synthetic polynucleotide

<400>  12
   aattgcgatc agcagtcatg ggggataatc aatgtattac agtattatca gtctttccac      60
   tagaatctag tccgaatgag caggagagat gcgcagaaga caatgcagcc agagtggctg     120
   ctagcttggc caaagtcaca agtgcctgtg ggatattcct caagcctgat gagactttcg     180
   tacactcagg ctttatctat tttggcaaaa agcaatactt gaacggaatt caattacctc     240
   aatcactcaa gacagca                                                    257

<210> 13
<211> 6633
<212> DNA
<213> Bundibugyo Ebolavirus

<400> 13
atggcaactc aacatacaca atatccagat gcaagattat cttcacccat tgtcttagat 60

caatgtgatc ttgtcacccg tgcttgcggt ctgtattctt catactcatt aaatcctcag 120

ttgaaaaatt gtagactacc aaaacatatt taccgcctca aatttgatgc tacggttaca 180

aaatttttaa gcgatgttcc aatagttaca ttgccgatag attacttgac ccctttactt 240

ttacgaactt tatccgggga gggcttatgc cctgtcgaac caaagtgcag ccaattctta 300

gatgaaatag taagttatgt tttgcaggat gcacgttttt taagatacta ttttaggcat 360

gttggagtac acgatgacaa tgttggaaaa aattttgagc caaagattaa ggctttgatt 420

tatgataatg aatttctgca acaattgttt tattggtacg atttagcaat cctaacgcgt 480

agagggcgcc tgaatcgagg gaataaccgt tcaacatggt ttgcaaatga cgatttaata 540

gacattctcg ggtacggtga ttatattttc tggaaaatac cgttgtcatt gttgtcactc 600

aacacagagg ggattcctca tgcagctaag gactggtatc acgcatcaat cttcaaagaa 660

gcggttcaag gtcacacaca tatcgtgtca gtttccactg cagatgtttt aattatgtgt 720

aaggacatca taacctgtcg tttcaatacc acactcattg cagcattggc aaatttagaa 780

gattctatct gttctgacta tccacaacct gaaacaatct ctaatctgta taaggcaggg 840

gattacttaa tctcgatact gggttcagaa ggttataagg tcataaagtt tttagaacca 900

ctatgtttag ctaagatcca attgtgctca aattacactg agaggaaagg gagattcctt 960

actcaaatgc atttggccgt taatcacaca cttgaagaac ttattgaggg ccggggattg 1020

aagtcacaac aagactggaa gatgagggaa tttcaccgaa tcttagtaaa tttaaagtca 1080

acaccacaac aactctgtga attgttttca gtgcaaaagc attgggggca tcctgtgcta 1140

catagcgaga aggctattca gaaagtaaag aaacatgcaa ccgtaataaa agcattgcgt 1200

cccgtaatca tctttgagac atattgtgtg ttcaagtaca gcattgccaa acattatttt 1260

gatagccaag ggtcatggta tagtgtaatc tcagataaac atctaacacc aggtttacac 1320

tcttacatta agaggaacca atttccgcca ctgcctatga ttaaagactt attgtgggaa 1380

ttctatcacc ttgatcatcc tcccttattt tccaccaaga ttattagtga cttgagtatt 1440

ttcattaagg atcgcgctac cgcagtggaa aaaacatgtt gggatgcagt tttcgagcct 1500

aatgttcttg gatatagtcc tccaaacaag ttctcaacta agagggttcc tgaacagttt 1560

cttgaacaag aaaatttctc gattgatagt gttctcactt atgcccagcg cctggattat 1620

ctacttccac aataccggaa tttttctttc tcacttaagg aaaaagaatt aaatgtagga 1680

cgagcttttg gtaagctacc ttatcctaca cgtaatgttc aaactttatg tgaagcctta 1740

ttggcagatg gattagctaa agcctttcct agtaacatga tggttgtaac agagcgtgag 1800

cagaaggaaa gcctcttgca ccaggcgtcg tggcaccaca caagtgacga tttcggtgag 1860

aatgccactg ttagaggcag cagttttgtt accgacctag aaaaatacaa cttggcattt 1920

agatatgagt ttacagctcc atttattgaa tactgtaatc gatgttatgg tgtaaaaaat 1980

ttattcaatt ggatgcatta tacgataccg caatgttata tacatgtaag tgattattat 2040

aatccccctc atggagtttc gctagaaaat cgggaagatc ccccggaagg ccctagctct 2100

taccgtggtc atcttggggg aattgaggga ctccaacaga aactctggac cagcatttca 2160

tgtgcacaaa tctcattagt tgagatcaag actggtttca aattgagatc tgcggtaatg 2220

ggtgataatc aatgcatcac agttctttcc gtatttcctc tagagacaga ttccaatgag 2280

caagagcata gctccgagga caatgctgct cgcgtagcag ccagtttagc caaagtcacg 2340

agtgcctgtg gcatcttcct aaaaccagat gagacttttg tgcattcagg ctttatttat 2400

ttcggtaaga agcaatattt aaatggcgtt caattgccac aatcactcaa gactgctacc 2460

aggattgctc ccttgtcaga tgcaatcttt gatgaccttc agggaactct ggctagtata 2520

ggaacggcat ttgagagatc tatatccgag actagacatg tatacccttg ccgggtggtt 2580

gccgcattcc atacattctt ctccgttagg atcctccaat accaccacct tggtttcaac 2640

aaaggaaccg atctaggtca actatcacta agcaaaccgt tggatttcgg aactatcact 2700

cttgctttag cggtacctca agttctagga ggtttatcgt ttttaaaccc agagaaatgt 2760

ttttatcgca accttggaga ccccgtgacc tccggcctat tccaacttag gacttacctg 2820

caaatgatca acatggacga cttatttcta cctttaattg ccaagaaccc cgggaactgt 2880

agtgcaattg actttgtact caacccaagc ggattgaatg tccctgggtc acaagaccta 2940

acatcttttt tacgtcagat agtgcgtaga acaatcacat tgagtgcaaa aaataagcta 3000

ataaacacat tgtttcactc ctcagccgat ttagaagatg agatggtatg taaatggcta 3060

ctttcttcaa cacctgtaat gagtcggttt gctgctgata tattctctcg tactccgagt 3120

gggaagcgct tgcagatcct aggttattta gaagggacta gaaccttgct agcctccaaa 3180

gtcatcaata acaatgcaga gactcctatt ttagataggt tgaggaaaat cacactgcag 3240

agatggagtt tgtggtttag ctacctagac cactgtgatc aggttctagc agatgcttta 3300

ataaaagttt cttgtacagt tgatttggcg caaattttac gtgaatatac ctgggcacac 3360

atactagagg gaagacagct cattggtgca acacttcctt gcatgttaga acaatttaat 3420

gtgttttggc tcaaatcgta cgaacaatgc cctaaatgtg caaaatctag aaatccaaaa 3480

ggagagccat ttgtgtcaat tgcaattaag aaacaagttg tgagtgcatg gccgaatcag 3540

tcacggttaa attggaccat tggggacggt gtaccttaca tcgggtctcg aacagaggac 3600

aagattgggc agccagcaat caagcctaag tgtccctctg ctgccttacg tgaagcaata 3660
gagttgacat ctagactaac atgggttacc caaggtggtg ccaatagtga tttgctagtt 3720
aaaccttttg tagaggcacg agtaaacctg agtgtgcagg agatccttca aatgacgcct 3780
tctcattatt cagggaacat cgtacatcgg tataatgacc aatacagccc tcattctttc 3840
atggcaaata gaatgagtaa ttccgcgacg agattggtgg tgtcgacaaa tactctcggg 3900
gagttctcag gtggggggca atcagcaagg gacagcaata tcatctttca aaatgtaatc 3960
aatttttcgg ttgccctatt tgatttacga tttcggaaca ccgaaacatc ctccattcag 4020
cataatcgtg cccatctcca tctttcacag tgttgcacac gggaagtccc agctcaatac 4080
ctaacctaca cgtctacgct ttccttggat ctcacaaggt accgagagaa tgagttaatt 4140
tatgataaca atccgttaaa aggtggactt aattgcaacc tatcctttga taatccactt 4200
ttcaagggcc aaaggctcaa tatcatagag gaggatttga ttagatttcc tcatctatct 4260
gggtgggaac ttgcgaaaac catcattcag tccattatct cagacagcaa taactcatcc 4320
acagacccca ttagcagtgg agaaacacga tcattcacaa ctcactttct cacatatcct 4380
aaggttgggc tcctctatag tttcggcgcc atcgtgagtt attacttagg gaataccatt 4440
attaggacca aaaagctaga cctcagtcat tttatgtatt acttaacaac tcaaatccat 4500
aatttgccac atcgctcgtt gaggatactt aagcccacct ttaaacatgt tagtgtgata 4560
tcaagactaa tgagtattga tcctcatttt tcaatctaca tcgggggtac ggcaggtgat 4620
cgagggcttt cggatgctac cagactattc cttcgagtgg ccatttcttc cttccttcaa 4680
tttatcaaaa aatggatcgt ggaatacaag acagctattc ctctgtgggt tatataccct 4740
ttggagggac aaaatccaga tccaattaat agctttctac atctgattat agccttactg 4800
caaaatgaat cccctcaaaa caacatccaa ttccaagaag acagaaataa tcaacagttg 4860
tccgataatc tagtttacat gtgcaagagc actgccagta atttcttcca tgcatcactt 4920
gcctattgga ggagccggca caaaggacgg cccaaaaatc gatcgaccga agaacagaca 4980
gttaaaccca taccatatga taattttcat tctgttaaat gtgcctcaaa cccaccaagc 5040
atccccaaat ctaagtcagg aactcaaggt tcaagcgcat tttttgagaa acttgaatat 5100
gataaagaaa gagaattgcc aacagcttcc acaccagccg aacaatccaa gacctatatc 5160
aaggccctat ccagccgaat ttatcatggt aaaacaccat ccaatgccgc aaaagatgat 5220
tcaacaacct ccaagggctg cgattccaaa gaagaaaatg ccgttcaagc ttcacaccga 5280
attgtcctac cattttttac attgtcacag aacgactaca gaactccctc agctaaaaag 5340
tcagagtata taactgaaat caccaaacta attcgacaat taaaggcaat tccagatacc 5400
actgtatact gtcgctttac aggggttgta tcttcaatgc attataagct tgatgaggtt 5460
ctctgggaat tcgatagttt caaaactgct gtgactctag ctgaaggaga agggtcaggt 5520

gccttattac tactacaaaa atataaggtc agaacaatct tttttaacac tttagctaca 5580

gagcatagca tcgaggcaga aatagtttct gggacaacca cacctcgaat gctccttcct 5640

gtaatggcca aacttcatga tgatcaaata aatgtaatat taaacaattc tgctagccag 5700

gttactgata tcactaaccc tgcatggttc actgaccaga aatctagaat ccccacacaa 5760

gttgagatta tgactatgga tgctgaaacg acagaaaata ttaatcggtc aaaattatat 5820

gaggctattc agcaattaat tgtttcacac attgatacaa gggtgctaaa gattgttatt 5880

ataaaggttt ttttaagtga tattgaaggt ctcctgtggc ttaatgacca tcttgcccct 5940

ttattcggat ccggctattt aattaaacct attacttcga gtccaaagtc aagcgaatgg 6000

tacttatgtc tttcaaattt cctttcagcc tctcgacgac ggcctcatca gggtcatgct 6060

acctgtatgc aagtcatcca aacagcgcta cgactccaag ttcaaaggag ttcatactgg 6120

cttagccatt tagtgcaata tgctgatatt aatttgcact tgagttatgt taatttgggt 6180

ttcccttcat tggaaaaggt tctttaccat cgatataacc tagttgattc acggaagggt 6240

ccactggtct cgatccttta ccatttaaca cacttgcaag cagagattag agaattagtg 6300

tgtgactata atcagcaacg acaaagtcga acccaaacat accacttcat caaaacgaca 6360

aagggccgga ttacaaaatt agtcaatgac taccttaaat tttatctcgt agtgcaagca 6420

ctgaagcata attgtctttg gcaggaagaa ctcagaacac ttcctgactt aatcaatgtt 6480

tgcaatcgat tttaccatat aagggactgc tcatgtgaag atcgattttt aattcaaact 6540

ctttacttaa cccgtatgca agactcagaa gcaaaattaa tggagagatt aaccgggttt 6600

ctaggattgt atcctaatgg tattaacgct taa 6633

<210> 14
<211> 245
<212> DNA
<213> artificial sequence

<220>
<223> synthetic polynucleotide

<400> 14
accgttgtca ttgttgtcac tcaacacaga ggggattcct catgcagcta aggactggta 60

tcacgcatca atcttcaaag aagcggttca aggtcacaca catatcgtgt cagtttccac 120

tgcagatgtt ttaattatgt gtaaggacat cataacctgt cgtttcaata ccacactcat 180

tgcagcattg gcaaatttag aagattctat ctgttctgac tatccacaac ctgaaacaat 240

ctcta 245

<210> 15
<211> 229
<212> DNA

<213>  artificial sequence

<220>
<223>  synthetic polynucleotide

<400>  15
attgccacaa tcactcaaga ctgctaccag gattgctccc ttgtcagatg caatctttga      60

tgaccttcag ggaactctgg ctagtatagg aacggcattt gagagatcta tatccgagac     120

tagacatgta tacccttgcc gggtggttgc cgcattccat acattcttct ccgttaggat     180

cctccaatac caccaccttg gtttcaacaa aggaaccgat ctaggtcaa                 229


<210>  16
<211>  259
<212>  DNA
<213>  artificial sequence

<220>
<223>  synthetic polynucleotide

<400>  16
tgtaccttac atcgggtctc gaacagagga caagattggg cagccagcaa tcaagcctaa      60

gtgtccctct gctgccttac gtgaagcaat agagttgaca tctagactaa catgggttac     120

ccaaggtggt gccaatagtg atttgctagt taaacctttt gtagaggcac gagtaaacct     180

gagtgtgcag gagatccttc aaatgacgcc ttctcattat tcagggaaca tcgtacatcg     240

gtataatgac caatacagc                                                  259


<210>  17
<211>  6639
<212>  DNA
<213>  Reston Ebolavirus

<400>  17
atggctaccc agcataccca gtacccggat gcacgtttat cctcacctat agtcctggat      60

caatgtgatt tggtaactcg agcatgtggg ttatattcat cttattctct aaatcctcaa     120

ctaaggcaat gtaaattacc aaaacatata tatcgactta agttcgacac aatagtatcc     180

aaattcctaa gtgatacacc tgtagcaaca ctgccgatag actatttagt accaattctc     240

ctgcgttccc taacggggca cggtgatagg ccattgaccc cgacttgcaa tcaattcctt     300

gatgaaatta ttaattacac tcttcatgat gcagcctttc ttgattacta tctcaaggca     360

acaggtgcac aggaccattt gacaaacatt gcaactagag agaagcttaa aaacgaaatt     420

ctaaacaatg attatgtcca tcaattgttc ttctggcatg acctttctat tttggctcga     480

cgtgggcgtc tgaatcgcgg gaacaaccgt tcaacctggt ttgttcatga tgaattcatt     540

gatattttag gatatggcga ttatattttt tggaaaatac ctttatcatt attaccagtt     600

actatagacg gggtcccaca cgcagcaact gactggtatc aaccgactct ttttaaagaa     660

tccatcctag ggcatagcca aatcctatct gtgtcaacag ctgaaatact aattatgtgt 720

aaagatatta tcacctgtag gtttaataca tcactgattg catccattgc aaaattagag 780

gatgtagatg tgtctgatta tcctgacccg agtgatattc ttaagatata caatgctgga 840

gactatgtaa tatctattct tggctcagag ggttataaga taataaagta ccttgaacca 900

ctttgtttgg ccaaaatcca actttgctct aaattcacag aaagaaaagg tcgtttcctc 960

acacagatgc atttatcagt aataaatgat cttcgggagt tgatttctaa ccgcaggtta 1020

aaggactatc agcaagagaa gattagagat tttcacaaaa tattattaca attgcaatta 1080

tctcctcaac agttttgtga attattctct gttcaaaaac attgggggca tccaatttta 1140

catagtgaga aagctataca aaaagtaaaa cggcatgcaa ccatccttaa ggctctcaga 1200

cctaatgtca tctttgagac atattgtgta ttcaagtaca atattgccaa gcactatttc 1260

gacagccaag gaacttggta cagtgtaatc tcagacagga atttaactcc aggactcaac 1320

tccttcataa aacgtaatca ctttccttca ctacccatga ttaaggatct tctatgggaa 1380

ttctatcatc ttaatcaccc tccgttattc tctacaaagg tgattagtga cttaagtatt 1440

ttcatcaaag atagggccac agctgttgaa cagacatgtt gggatgcagt ctttgaaccc 1500

aatgtgctag gttacaatcc tccaaacaaa ttctccacta aaagggtgcc ggaacaattt 1560

ctagaacaag aggatttttc aatcgaaagt gtcctgaatt atgcacagga attacattat 1620

ttattaccac agaataggaa tttttccttt tctctcaaag aaaaggaatt aaatattgga 1680

cgaacatttg ggaagctacc atatctcaca cggaatgtcc aaactttatg tgaggctctg 1740

ttagcagatg gactggccaa ggccttcccc agtaacatga tggtagtaac tgaacgtgaa 1800

caaaaagaga gccttcttca tcaggcatca tggcaccaca ccagtgatga ttttggagag 1860

aatgctaccg ttcgagggag tagttttgta actgatttag agaagtacaa tcttgcattt 1920

cgctatgagt tcactgcacc atttattgag tactgcaacc attgctatgg tgtgcgtaat 1980

gtctttaatt ggatgcatta tttaatcccg cagtgttaca tgcatgtaag tgattattat 2040

aatccgcctc acaatgttaa tcttagcaat cgagaatatc ctcctgaagg cccgagttcg 2100

taccgagggc acttaggagg catagaggga ttacaacaaa aactgtggac gagtatatcc 2160

tgtgcacaaa tctccttagt ggaaattaaa actggtttta agttacgatc agcggtcatg 2220

ggagacaatc agtgtataac cgtattgtct gtttttccac ttaaaacaga ccctgaagag 2280

caggagcaaa gcgccgaaga caatgctgca agagtagcag caagtcttgc aaaagtaacc 2340

agtgcatgtg ggatctttct taaaccagat gagacatttg tacactcagg tttcatttat 2400

ttcggaaaaa aacaatatct caatggtgta caattaccgc aatcactcaa aacagcagca 2460

agaatggcac cactctctga tgctatattc gatgatctac aaggaacact tgccagtatt 2520

ggaactgcct tcgaacgtgc tatatcggaa acgcgacata tcctcccatg tcgtattgta 2580

gcagctttcc atacgtattt cgccgttcgg attttacaat atcaccatct tggatttaat 2640
aaaggcatcg atttaggaca gttgtcactt agtaaaccat tagactatgg gactattact 2700
ctaacattgg cggttccaca agtccttggg ggattgtctt ttctaaatcc agaaaagtgt 2760
ttttatcgaa acttcggaga tcctgtgact tctggacttt tccagctacg ggtgtaccta 2820
gaaatggtta acatgaaaga cctattttgt ccattaatat cgaaaaatcc aggaaattgt 2880
agtgccattg atttgtcttt aaatccatcc ggattaaatg ttccaggatc acaagacttg 2940
acatcctttt tgcgacaaat cgttaggcgt agtattaccc taactgctag aaataagtta 3000
attaacactc tcttccatgc ctctgctgat ttggaagatg agatggtttg taagtggctc 3060
ctttcatcaa accctgtcat gagtcgcttt gcagcggata ttttttccag gacaccgagt 3120
ggtaaacgtc tccaaatatt aggttatctt gaaggaacca ggactctatt ggcctccaaa 3180
atcataaaca acaacagtga gacacctgta cttgataagc tgaggaagat caccctacaa 3240
agatggaatc tgtggttcag ttatttggac cattgtgacc aattactagc agatgctcta 3300
cagaaaatta gttgcacggt ggatttggcc cagattttgc gtgagtatac atggtcacac 3360
atcttagagg gtagatcatt gatcggagcg acattaccat gtatggtgga gcaattcaaa 3420
gttaagtggc taggacaata tgaaccttgt ccagaatgtc tcaacaaaaa aggctcaaat 3480
gcttatgtct cagttgcagt caaagatcaa gtggtcagtg cttggcctaa tacttctcga 3540
ataagttgga caatagggag tggtgtcccc tatatagggt caagaaccga ggataaaatc 3600
ggacagcctg ctatcaagcc gcgatgccct tcatctgccc tcaaggaggc tatagaatta 3660
gcatcaaggc tcacttgggt tacacaagga ggttctaata gtgaacaatt aatccggcct 3720
ttcttggaag cgagagtcaa ccttagtgtc agtgaagtcc tgcaaatgac accatcacat 3780
tattcaggaa atattgtcca tcgatataac gaccaataca gcccgcactc atttatggcg 3840
aatcgcatga gcaatactgc gacccgtctc atagtgtcaa ctaatacact tggagaattt 3900
tcaggtggag ggcaggccgc cagggatagc aatataattt tccagaatgt tataaattta 3960
gcagttgccc tttatgatat tagattccgg aatacaaaca cctctgatat aaggcataat 4020
agggctcatc ttcacctgac agagtgctgt actaaagagg tcccggccca gtatttgaca 4080
tatacaagtg cacttaatct ggatttaagc cgttatcgtg ataatgaact aatatatgac 4140
tcaaatccac tgaagggagg attgaactgc aatttaacaa tagatagtcc tttagtgaag 4200
ggtcctaggc ttaacatgat tgaagatgat cttctccgct ttccacacct ttctggatgg 4260
gagttagcga aaacggtggt acaatccatc atctcagaca atagcaactc atcaacagat 4320
ccaatcagta gcggagaaac acgctctttc acaactcatt ttctcactta ccctcagatt 4380
ggccttcttt acagtttcgg agcagtatta tgcttttatc taggcaatac tatcctatgg 4440

actaaaaaac ttgattacga acagtttcta tattatttgc ataaccagct gcacaactta 4500
cctcatcgag cactccgtgt ttttaaacca acatttaagc atgccagtgt gatgtcccga 4560
ttaatggaaa ttgattctaa cttctcaatt tatattggcg ggacatctgg agatcgaggg 4620
ctgtctgatg ctgctcgact gtttcttcgg acagcaatcg cgagtttttt acaatttctt 4680
aaaagctgga tcatcgatcg ccaaaagaca attcctttat ggatagtata tccgcttgaa 4740
ggtcaacagc cggaatccat caatgaattt ctacataaaa ttttgggtct gctcaaacaa 4800
ggccccaaaa gtattccaaa ggaggtcagc atccaaaatg atggacattt ggatttggca 4860
gaaaataatt atgtttacaa tagtaagagc actgctagta atttcttcca tgcatcctta 4920
gcttactgga gaagtaggaa atctcggaaa actcaagacc ataatgattt ctcaagaggg 4980
gatggaacac ttacagaacc cgtgcgtaag ttttcaagca atcatcagtc agatgaaaag 5040
tactacaatg tgacatgtgg aaagtcaccg aagccgcaag aacgcaaaga cttctcgcaa 5100
tacagactca gcaataacgg gcaaacaatg agtaatcatc gtaagaaagg gaagttccac 5160
aagtggaatc cctgcaaaat gttaatggag agtcaaaggg gaactgttct aacagagggt 5220
gactactttc aaaacaatac tccaccaaca gatgatgtat caagtcctca ccgactcatt 5280
ctaccatttt ttaaattggg aaatcacaac catgcacatg atcaagatgc ccaagaattg 5340
atgaatcaaa atattaaaca gtacctacat cagctaaggt ctatgttgga caccactata 5400
tattgtagat tcacagggat tgtctcatcc atgcattaca aattggacga agttcttcta 5460
gaatacaata gtttcgattc agctatcaca ttagctgaag gtgaggggtc aggggctcta 5520
ttacttttgc aaaaatatag tacaaggtta ttatttttga acacattggc aacagaacac 5580
agtatagaat cagaagttgt atcaggtttt tctactccga gaatgttgtt accaataatg 5640
caaaaggttc atgaaggaca agtcactgtt atcttaaata attcagcaag tcagataact 5700
gacataacta gctcaatgtg gttaagtaat caaaaatata atctaccttg tcaagttgaa 5760
atcattatga tggatgctga aacaacagag aacttaaaca ggtcccaact ctaccgagca 5820
gtatataact taatacttga tcacattgat ccgcagtatc tcaaggtggt ggtactcaaa 5880
gtatttctga gtgatataga aggaatatta tggattaatg attacttggc tccattattc 5940
ggggctggtt acttgattaa accgattaca tcaagtgccc ggtcaagtga atggtacctt 6000
tgcttatcaa atttgatatc tactaacagg agatcggccc atcagactca caaggcatgt 6060
cttggtgtta tcagagatgc tttgcaagca caagtccagc gaggcgtgta ctggttgagt 6120
cacatcgcac agtatgctac aaagaatctc cattgtgaat acataggcct tggtttccca 6180
tctctagaaa aggtcctata tcacaggtat aatctagttg atactggact cggtccattg 6240
tcgtcagtta ttagacattt aactaacctc caggcagaga tacgagactt agtattagat 6300
tataacctga tgagggagag tcgcactcaa acgtaccatt ttattaagac tgcaaaaggc 6360

agaatcacaa agttagtcaa tgactttctg aagttttctt taattgtcca ggcactcaaa 6420

aataattctt cttggtatac tgagcttaaa aaattacctg aggttattaa tgtgtgtaat 6480

cgattttatc atactcacaa ttgcgaatgt caggaaaaat tctttgtcca gacgctttat 6540

ttacaacgcc tacgcgatgc agaaatcaag ctaattgaac gccttaccgg gttaatgcga 6600

ttttatccag aagggttaat atattccaat cacacatag 6639

<210> 18
<211> 286
<212> DNA
<213> artificial sequence

<220>
<223> synthetic polynucleotide

<400> 18
acatgatggt agtaactgaa cgtgaacaaa aagagagcct tcttcatcag gcatcatggc 60

accacaccag tgatgatttt ggagagaatg ctaccgttcg agggagtagt tttgtaactg 120

atttagagaa gtacaatctt gcatttcgct atgagttcac tgcaccattt attgagtact 180

gcaaccattg ctatggtgtg cgtaatgtct ttaattggat gcattattta atcccgcagt 240

gttacatgca tgtaagtgat tattataatc cgcctcacaa tgttaa 286

<210> 19
<211> 321
<212> DNA
<213> artificial sequence

<220>
<223> synthetic polynucleotide

<400> 19
aggcaatact atcctatgga ctaaaaaact tgattacgaa cagtttctat attatttgca 60

taaccagctg cacaacttac ctcatcgagc actccgtgtt tttaaaccaa catttaagca 120

tgccagtgtg atgtcccgat taatggaaat tgattctaac ttctcaattt atattggcgg 180

gacatctgga gatcgagggc tgtctgatgc tgctcgactg tttcttcgga cagcaatcgc 240

gagtttttta caatttctta aaagctggat catcgatcgc caaaagacaa ttcctttatg 300

gatagtatat ccgcttgaag g 321

<210> 20
<211> 219
<212> DNA
<213> artificial sequence

<220>
<223> synthetic polynucleotide

<400> 20

ttacttggct ccattattcg gggctggtta cttgattaaa ccgattacat caagtgcccg 60

gtcaagtgaa tggtaccttt gcttatcaaa tttgatatct actaacagga gatcggccca 120

tcagactcac aaggcatgtc ttggtgttat cagagatgct ttgcaagcac aagtccagcg 180

aggcgtgtac tggttgagtc acatcgcaca gtatgctac 219

<210> 21
<211> 6996
<212> DNA
<213> Marburg virus

<400> 21
atgcagcatc caactcaata tcctgatgca aggttgtcct ctcctataat cctagaccag 60

tgtgacttat tagccagaag tttagggttg tatagtcatt attcacataa tccgaaattg 120

cgtaattgta ggattccaca tcacatttac cgtttaagga attcgacagc attaaagaca 180

tttcttcaga attgttcgat actcaccgtt ccttttcatt caatttggga tcatatttta 240

acttccattc aatatgatgc aattaatcat gttgatgatt ttaaatacct attgccatct 300

gagctagtca agtatgcgaa ttgggacaac gagttcttaa aagcatatct taataagatc 360

ttaggacttg accatgtttt ttcagcttct gcaaggtcac agtgtgagga tttttctcct 420

aaggaaaatc cttattattg ggggatgtta ttactcgtgc atctatctca acttgccagg 480

aggataaaag gacaaagagg gtcattaaga agtaactgga aatttatagg aacagatttg 540

gagctgtttg gaatagcaga ttttgttatt tttaaggttc cagtaaaaac aataatccga 600

aatgctgtaa gcttacaagc ttcaaaaccg ggattaagaa tatggtaccg tgaccaaaac 660

ttgacccctt atctatgcga cgatgagttt attgtaagcg tcgctagtta tgaatgtttt 720

atcatgatta aagacgtctt cattgagagg tataacacat gggaaatctg tgcccgcgct 780

tggctcgaag acagtgatgg agctgattat cctcctcttg atgtgttagg tgaattatac 840

aaccagggag accaaattat tgccatgtac ctggaggacg gtttcaaatt gattaaacac 900

ttagaaccct tgtgtgtcag ctgtatacaa acacatggca tctttacacc aagaaaatac 960

tggttccaat cacagatgat taagtcatat tatgatgaac tccatgatct caatttgaaa 1020

cttcaaattt cagacaataa ggctgagtgt gcccaaaact ttattaaaac tatagttcag 1080

gcgaaattga ctcctcaaca atactgtgaa ttattctccc tacaaaagca ttggggtcat 1140

cccgttttat acaatgatgt tgcactagat aaggttaaaa aacatgcgca atcgacaaaa 1200

atcttaaaac ctaaagtcat gtttgaaact tttgtgtttt tcaaatttat agtagcaaag 1260

aatcattatc attctcaagg atcatggtat aaaaccacac atgatttgca tttgactcca 1320

tatcttagac aacatattgt gtcaaattca tttccatcac aagccgaaat ttatcagcat 1380

ctttgggagt ggtatttcgt ggagcatgaa cctcttttct caactaaaat aataagtgat 1440

ttaagtatct ttataaaaga cagggctacc gctgtgaacc aggagtgttg ggacagtgtc 1500

ttcgatagaa gtgtattagg atataaccct cctgttagat ttcaatcaaa gagagtgcca 1560

gagcaatttt tgggtcaagc agacttttcc ttgaatcaaa tattagagtt tgctgaaaag 1620

ttagagtatt tggctccttc ttataggaat ttttccttct cattaaaaga aaaagagttg 1680

aatataggaa gaactttggg gaagttaccg tatcgtgtca gaaatgtcca aacactcgca 1740

gaagccctgc tagcagatgg actggcaaaa gcattcccta gtaacatgat ggttgtcact 1800

gagagggaac agaaagaagc attattacat caggcttctt ggcaccacaa ttcagcaagc 1860

ataggggaga acgctatagt gaggggtgca agttttgtta ctgatcttga gaaatacaac 1920

ctcgccttcc gatatgaatt tacacggcat ttcatagact actgtaatcg atgttatggt 1980

gtgaagaatt tatttgattg gatgcatttt ttaataccac tatgttatat gcatgtcagt 2040

gacttttata gcccaccaca ttgtgtgaca gaagataatc gaaataaccc acctgattgt 2100

gctaatgctt atcattatca cttaggaggt atagagggac ttcagcagaa attgtggaca 2160

tgcatatcat gtgcccaaat cacccttgtg gagttaaaaa ctaaattaaa attgaagtcc 2220

agcgtcatgg gtgataatca atgtataaca actctaagtc tttttccaat tgatgctccc 2280

aacgattatc aagagaacga ggctgaattg aatgcggcac gagttgctgt cgaattagct 2340

attactacag gttatagtgg tatattttta aagcctgagg aaacatttgt ccattcaggg 2400

ttcatttatt ttggtaaaaa gcaatatctc aacggtgttc aactgccgca atcattgaaa 2460

acaatggcaa ggtgtggacc cttatctgac tctattttcg atgatcttca aggttctctg 2520

gccagtattg gtacatcctt tgagagagga acaagcgaga cacggcacat ttttccgagc 2580

cgttggatag cttcattcca ttcaatgtta gcaataaatt tattaaatca gaatcacctt 2640

gggtttcctc tagggttcaa tattgatatt tcttgtttca aaaagcctct taccttctcg 2700

gaaaaattaa ttgctctcat aacgccccaa gttttaggag ggttatcatt tttaaatcca 2760

gaaaaattgt tctaccggaa cataagtgat cctctcactt caggtctatt tcaactcaag 2820

aatgcattgg aatttcttga aaaggaagaa ttattctata tcttgatttc taaaaaacct 2880

ggtttagcag atgcctcaga ttttgtcatg aatccattag gcttaaatgt accaggatca 2940

aaggaaataa taacgttcct tagacaaaca gttcgcgaaa atatcacgat cacgtcacaa 3000

aatagaataa taaattctct tttccacata ggttctgatt tagaggacca aagagtgtgt 3060

gagtggcttt tatcatcaaa ccctgtaatg agccgatttg ctgctgacat cttttcaaga 3120

acacctagtg gaaaacggct tcaagtctta ggctatctag aaggaacaag aacattacta 3180

gcttctcgga caatcagttt aactacagaa ggaacaatgt tgatgaaatt aagagaatta 3240

acgagaaacc gatggaaaag ttggttttct tatattgatg cactggacga tgatttatct 3300

gagtccttgg aaaagttcac atgtactgtt gatgtggcta atttcttgag ggcatattca 3360

tggtctgacg tcttaaaagg gaaaaggcta attggtgcca cactgccatg tttactagag 3420
caatttgagg taaagtggat taatttatct gaggatttaa gggaacaatt taatctatct 3480
tcagactcaa aatcaactat aaacttgttg ccgtatgact gtaaggaact gcgacttgaa 3540
ggaagcaatg acacagagtt aaattatgtc agttgtgctc ttgaccggaa agttgtccag 3600
aaacatccct ctgttaatcg tctagcttgg acgataggaa atcgagcacc gtatattggc 3660
tcacggacag aagataagat cggttatcct cccttaagag taaattgccc atcagcagca 3720
cttaaagaag ctattgagat ggtttctaga ttgttatggg tgactcaagg cactgcagac 3780
cgagaaaaat tgcttattcc tcttctcaat tcaagagtaa atctggacta tcagacggtg 3840
cttaactttt tacctacaca ctactcaggc aacatagttc atagatataa tgatcaatat 3900
ggacaacatt cctttatggc aaacaggatg agtaatacat ctacacgtgc aattatatca 3960
actaacacac tgggtaaata tgctggggga ggtcaagctg ctattgatag taatataatc 4020
tttcaaaata ctattaattt aggagttgca gttttagata ttgcattgtc tcttgctaaa 4080
ttgtcgtcag catcaaatgt cactttccgt ttaatgttaa ataagtgctg cacgcggcat 4140
gtaccgtccg aatacctata ttttgataaa cctttagatg tggatttgaa caagtatatg 4200
gacaatgagt tggtttatga caatgaccct ctttgcagtg ggattaaagg gagattaggc 4260
agagtatctc gatcaacact cacattgagt ttgaatgtca gtgacattgg ttcttatgac 4320
tttccaacta ttgctgcatg gacactagga gaaactatag tcggaagcat tttttctgat 4380
gaatcttctc aaagtacgga tccaataagc tcaggttgca caaaaacttt cgtcacacat 4440
ttccttgtgt atccagttga gagtatattt tatgcattcg gagctaactt aatagttgaa 4500
agtttaagtc taagtaggat caaatcaatt aagaacctct cagatttgac attccttata 4560
tcatccacaa tcaggaattt atcacataga tcacttcgga ttcttcaatc tactttccga 4620
catgaattgg tgctcacccg actagcccac cacataccgt taatttcttt aatgttaggg 4680
ggctctgcag gagagaagag ttcatcagat gctgttcggc tatttcttac agcaagttat 4740
cagaatttta tcaataattt cagttgtctg atgaagaagg gtcagtcatc gctaccggtt 4800
tggctttact ttcctagtga agggcaacaa ttaaagccta tattaaaaat cttacagaga 4860
ttatcagact tgttatcacc tgacaaaatt caaaagcgta aaattttggc tgacacctgt 4920
tgtccaattg gcagcttttg ggtctatcca agcaagtcca caaggactaa ccattattat 4980
gcaagcctta attattggag agacaaagct aataaggtta agaatactcc tttttcacac 5040
ttgataaatt gttcatttcc tgaattttct tcacatacca gttcagtctc ctctaatcaa 5100
caagtgacca attcgaagta tattgtttat ccagaaaata tcactgaaat aaatgcaaga 5160
accagattaa taaattatgg atcaacagct ctacagggga tggacaccaa gatgccactc 5220

tcagagcaaa atctagtcga aaattgtcga ccatcagagg gcatcagatt caaggacaat 5280

caaaaaataa caaaacatga ccagagatgt gagagggagg aatcttcacc gcaacagatg 5340

ttccctgaag ataacatgca gactcctgcg cacatacata gttcctcccc atttcaaatc 5400

cttataaaat cactagatgc acatgaggac tttgatgcct cgaagataat cttaaattct 5460

gaaataaata atcttaacct tacggagtat actcttaata caaagttatt gacaactcct 5520

accaggacag aaattttaga tacaagtccg ttacaatcct ctagatattc atcaacttcc 5580

agggaacggt ctctactatc cagagaacaa gcttcatatt tgtacgttga ttgcagtaat 5640

attccttcta tctctctaga cccaggtttt cggagtatgt ctgatcagaa tcaagttcaa 5700

atgttaatca atacctacaa acgtgattta catgcttgtt ttgatagcaa tcaattctgt 5760

cggtttacag gggtagtctc atcaatgcat tacaagcttt atgatctttt gcctccaggt 5820

aaattgaaaa aggcaatttg tttggccgaa ggggaaggaa gtggtgctcg gttacttttg 5880

aagtggaagg aaacggatta tttattcttc aacactttgg ctacggattc acaacaagaa 5940

gccgagattt tgagtggccg ggtaataccg agaatgttgt ataacataga cagattaagt 6000

gctttgcttg aatcaaggag actaatattg aacaacctaa ctatccaaat tacagatatt 6060

acaaatccat tatggctaga ttctgtaata caatatttac ctgaagatag tgacattctt 6120

acaatggacg cagagaccac caaggatgaa acaagggaac agctttataa aactattgtg 6180

aatatttgga cacgtacttc tcctaatatc ccaaaaatta gcatcatcaa ggtattttta 6240

ttagactatg aagggacttt attcttaatg aagaatgcta ttcagtatta tgggcaagtt 6300

caactcaaga aaccatatag ctcaaatgca aaaaactcag aatggtactt gtgttgcggt 6360

aaacgaagaa ttcaacggct ccaaattgat ttctcagacc aggtgggaat tttctgattt 6420

tgtaaagcaa tgtcacgcca aagacaagca attccttact ggttaaaaca tatagaaaag 6480

aattatcctg cttcattaca cgagtttttc ctaactttgg gtttcccttc tttagagtca 6540

tctttctgcc atcgttatac tattccattc agtgaaggaa aggctctttt tcacaaggtc 6600

cagtcttatg ttcgtcaagg caaacaacat ttacattctc ttatgttgga ttatgaaaac 6660

aattcacctc tactagactt gagaaatcac tttatttgct cattaagggg aaagataact 6720

aaatattaca atgatatatt aaagttaaat ctagtcatca aggcagtaga aaaaggtaaa 6780

aattggtcac aacttgttga gatccttcct aatatgcatt cagtatgcat agtgcacgtg 6840

gatcatgagt gttctggatg cgagaaacgg ttattactta aattggattt tatcagaaat 6900

acaaagatcg cagaacaaaa attacttaac agagtaatcg ggtatatcct attctttcca 6960

ttcggtctgt ttaaatctgg atcattaagg gcataa 6996

<210> 22
<211> 264

<212>  DNA
<213>  artificial sequence

<220>
<223>  synthetic polynucleotide

<400>  22
cgaagacagt gatggagctg attatcctcc tcttgatgtg ttaggtgaat tatacaacca      60

gggagaccaa attattgcca tgtacctgga ggacggtttc aaattgatta aacacttaga     120

acccttgtgt gtcagctgta tacaaacaca tggcatcttt acaccaagaa aatactggtt     180

ccaatcacag atgattaagt catattatga tgaactccat gatctcaatt tgaaacttca     240

aatttcagac aataaggctg agtg                                            264


<210>  23
<211>  235
<212>  DNA
<213>  artificial sequence

<220>
<223>  synthetic polynucleotide

<400>  23
agcaatcaat tctgtcggtt tacaggggta gtctcatcaa tgcattacaa gctttatgat      60

cttttgcctc caggtaaatt gaaaaaggca atttgtttgg ccgaagggga aggaagtggt     120

gctcggttac ttttgaagtg gaaggaaacg gattatttat tcttcaacac tttggctacg     180

gattcacaac aagaagccga gattttgagt ggccgggtaa taccgagaat gttgt          235


<210>  24
<211>  243
<212>  DNA
<213>  artificial sequence

<220>
<223>  synthetic polynucleotide

<400>  24
agacaagcaa ttccttactg gttaaaacat atagaaaaga attatcctgc ttcattacac      60

gagtttttcc taactttggg tttcccttct ttagagtcat ctttctgcca tcgttatact     120

attccattca gtgaaggaaa ggctcttttt cacaaggtcc agtcttatgt tcgtcaaggc     180

aaacaacatt tacattctct tatgttggat tatgaaaaca attcacctct actagacttg     240

aga                                                                   243


<210>  25
<211>  2088
<212>  DNA
<213>  Marburg virus

<400>  25
atggatttac acagtttgtt ggagttgggt acaaaaccca ctgcccctca tgttcgtaat      60

aagaaagtga tattatttga cacaaatcat caggttagta tctgtaatca gataatagat 120

gcaataaact cagggattga tcttggagat ctcctagaag ggggtttgct gacgttgtgt 180

gttgagcatt actataattc tgataaggat aaattcaaca caagtcctat cgcgaagtac 240

ttacgtgatg cgggctatga atttgatgtc atcaagaatg cagatgcaac ccgctttctg 300

gatgtgattc ctaatgaacc tcattacagc cctttaattc tagcccttaa gacattggaa 360

agtactgaat ctcagagggg gagaattggg ctctttttat cattttgcag tcttttcctc 420

ccaaaacttg tcgtcggaga ccgagctagt atcgaaaagg ctttaagaca agtaacagtg 480

catcaagaac aggggatcgt cacataccct aatcattggc ttaccacagg ccacatgaaa 540

gtaattttcg ggattttgag gtccagcttc attttaaagt ttgtgttgat tcatcaagga 600

gtaaatttgg tgacaggtca tgatgcctat gacagtatca ttagtaattc agtaggtcaa 660

actagattct caggacttct tatcgtgaaa acagttctcg agttcatctt gcaaaaaact 720

gattcagggg tgacactaca tcctttggtg cggacctcca aagtaaaaaa tgaagttgct 780

agtttcaagc aggcgttgag caacctagcc cgacatgggg aatacgcacc atttgcacgg 840

gttctgaatt tatcagggat taacaacctc gaacatggac tctatcctca gctttcagca 900

attgcgctgg gtgtggcaac agcacacggc agtacattgg ctggtgtcaa tgttggcgaa 960

caatatcaac aactacgaga ggcggcacat gatgcggaag taaaactaca aaggcgacat 1020

gaacatcagg aaattcaagc tattgccgag gatgacgagg aaaggaagat attagaacaa 1080

ttccaccttc agaaaactga aatcacacac agtcagacac tagccgtcct cagccagaaa 1140

cgagaaaaat tagctcgtct cgctgcagaa attgaaaaca atattgtgga agatcaggga 1200

tttaagcaat cacagaatcg ggtgtcacag tcgtttttga atgaccctac acctgtggaa 1260

gtaacggttc aagccaggcc catgaatcga ccaactgctc tgcctccccc agttgacgac 1320

aagattgagc atgaatctac agaagatagc tcttcttcaa gtagctttgt tgacttgaat 1380

gatccatttg cactgctgaa tgaggacgag gatactcttg atgacagtgt catgatcccg 1440

ggcacaacat cgagagaatt tcaaggattt cctgaaccgc caagacaatc ccaagacctc 1500

aataacagcc aaggaaagca ggaagatgaa tccacaaatc cgattaagaa acagtttctg 1560

agatatcaag aattgcctcc tgttcaagag gatgatgaat cggaatacac aactgactct 1620

caagaaagca tcgaccaacc aggatccgac aatgaacaag gagttgatct tccacctcct 1680

ccgttgtacg ctcaggaaaa aagacaggac ccaatacagc acccagcagc aaaccctcag 1740

gatcccttcg gcagtattgg tgatgtaaat ggtgatatct tagaacctat aagatcacct 1800

tcttcaccat ctgctcctca ggaagacaca aggatgaggg aagcctatga attgtcgcct 1860

gatttcacaa atgatgagga taatcagcag aattggccac aaagagtggt gacaaagaag 1920

ggtagaactt tcctttatcc taatgatctt ctgcaaacaa atcctccaga gtcacttata 1980

acagccctcg ttgaggaata ccaaaatcct gtctcagcta aggagcttca agcagattgg 2040

cccgacatgt catttgatga aaggagacat gttgcgatga acttgtag 2088

<210> 26
<211> 267
<212> DNA
<213> artificial sequence

<220>
<223> synthetic polynucleotide

<400> 26
aacaacctcg aacatggact ctatcctcag ctttcagcaa ttgcgctggg tgtggcaaca 60

gcacacggca gtacattggc tggtgtcaat gttggcgaac aatatcaaca actacgagag 120

gcggcacatg atgcggaagt aaaactacaa aggcgacatg aacatcagga aattcaagct 180

attgccgagg atgacgagga aaggaagata ttagaacaat tccaccttca gaaaactgaa 240

atcacacaca gtcagacact agccgtc 267

<210> 27
<211> 290
<212> DNA
<213> artificial sequence

<220>
<223> synthetic polynucleotide

<400> 27
cggcagtatt ggtgatgtaa atggtgatat cttagaacct ataagatcac cttcttcacc 60

atctgctcct caggaagaca caaggatgag ggaagcctat gaattgtcgc ctgatttcac 120

aaatgatgag gataatcagc agaattggcc acaaagagtg gtgacaaaga agggtagaac 180

tttcctttat cctaatgatc ttctgcaaac aaatcctcca gagtcactta taacagccct 240

cgttgaggaa taccaaaatc ctgtctcagc taaggagctt caagcagatt 290

<210> 28
<211> 1710
<212> DNA
<213> Lassa virus

<400> 28
atgagtgcct caaaggaaat aaaatccttt ttgtggacac aatctttgag gagggaatta 60

tctggttact gctccaacat caaactacag gtggtgaaag atgcccaggc tcttttacat 120

ggacttgact tctccgaagt cagtaatgtt caacggttga tgcgcaagga gagaagggat 180

gacaatgatt tgaaacggtt gagggaccta aatcaagcgg tcaacaatct tgttgaatta 240

aaatcaactc aacaaaagag tatactgaga gttgggactc taacctcaga tgacttatta 300

atcttagccg ctgatctaga gaagttaaag tcaaaggtga tcagaacaga aaggccatta 360

agtgcaggtg tctatatggg caacctaagc tcacagcaac ttgaccaaag aagagctctc 420

ctgaatatga taggaatgag tggtggtaat caaggggctc gggctgggag agatggagtg 480

gtgagagttt gggatgtgaa aaatgcagag ttgctcaata atcagttcgg gaccatgcca 540

agtctgacac tggcatgtct gacaaaacag gggcaggttg acttgaatga tgcagtacaa 600

gcattgacag atttgggttt gatctacaca gcaaagtatc ccaacacttc agacttagac 660

aggctgactc aaagtcatcc catcctaaat atgattgaca ccaagaaaag ctctttgaat 720

atctcaggtt ataattttag cttgggtgca gctgtgaagg caggagcttg catgctggat 780

ggtggcaata tgttggagac aatcaaggtg tcacctcaga caatggatgg tatcctcaaa 840

tccattttaa aggtcaagaa ggctcttgga atgttcattt cagacacccc tggtgaaagg 900

aatccttatg aaaacatact ctacaagatt tgtttgtcag gagatggatg gccatatatt 960

gcatcaagaa cctcaataac aggaagggcc tgggaaaaca ctgtcgttga tctggaatca 1020

gatgggaagc cacagaaagc tgacagcaac aattccagta aatccctgca gtcggcaggg 1080

tttaccgctg ggcttaccta ttctcagctg atgaccctca aggatgcaat gctgcaactt 1140

gacccaaatg ctaagacctg gatggacatt gaaggaagac ctgaagatcc agtggaaatt 1200

gccctctatc aaccaagttc aggctgctac atacacttct tccgtgaacc tactgattta 1260

aagcagttca agcaggatgc taagtactca catgggattg atgtcacaga cctcttcgct 1320

acacaaccgg gcttgaccag tgctgtcatt gatgcactcc cccggaatat ggtcattacc 1380

tgtcaggggt ccgatgacat aaggaaactc cttgaatcac aaggaagaaa agacattaaa 1440

ctaattgata ttgccctcag caaaactgat tccaggaagt atgaaaatgc agtctgggac 1500

cagtataaag acttatgcca catgcacaca ggtgtcgttg ttgaaaagaa gaaaagaggc 1560

ggtaaagagg aaataacccc tcactgtgca ctaatggact gcatcatgtt tgatgcagca 1620

gtgtcaggag gactgaacac atcggttttg agagcagtgc tgcccagaga tatggtgttc 1680

agaacatcga cacctagagt cgttctgtaa 1710

<210> 29
<211> 239
<212> DNA
<213> artificial sequence

<220>
<223> synthetic polynucleotide

<400> 29
gaattatctg gttactgctc caacatcaaa ctacaggtgg tgaaagatgc ccaggctctt 60

ttacatggac ttgacttctc cgaagtcagt aatgttcaac ggttgatgcg caaggagaga 120

agggatgaca atgatttgaa acggttgagg gacctaaatc aagcggtcaa caatcttgtt 180

gaattaaaat caactcaaca aaagagtata ctgagagttg ggactctaac ctcagatga 239

<210> 30
<211> 188
<212> DNA
<213> artificial sequence

<220>
<223> synthetic polynucleotide

<400> 30
aaagctgaca gcaacaattc cagtaaatcc ctgcagtcgg cagggtttac cgctgggctt 60

acctattctc agctgatgac cctcaaggat gcaatgctgc aacttgaccc aaatgctaag 120

acctggatgg acattgaagg aagacctgaa gatccagtgg aaattgccct ctatcaacca 180

agttcagg 188

<210> 31
<211> 18
<212> DNA
<213> artificial sequence

<220>
<223> synthetic oligonucleotide

<400> 31
atccgactcg caatgttc 18

<210> 32
<211> 18
<212> DNA
<213> artificial sequence

<220>
<223> syntehtic oligonucleotide

<400> 32
acaccatagc aacggttg 18

<210> 33
<211> 42
<212> DNA
<213> artificial sequence

<220>
<223> synthetic oligonucleotide

<400> 33
ttgctcacgt tccgtaacta ccaacacttt gtgaagctct gt 42

<210> 34
<211> 42
<212> DNA
<213> artificial sequence

<220>
<223>  synthetic oligonucleotide

<400>  34
tcaagcatca tggcaccaca aatcagttac aaagctactc cc                          42


<210>  35
<211>  20
<212>  DNA
<213>  artificial sequence

<220>
<223>  synthetic oligonucleotide

<400>  35
gctttagcaa gaccatcagc                                                   20


<210>  36
<211>  20
<212>  DNA
<213>  artificial sequence

<220>
<223>  syntehtic oligonucleotide

<400>  36
tggtgaacat gccacagtta                                                   20


<210>  37
<211>  21
<212>  DNA
<213>  artificial sequence

<220>
<223>  synthetic oligonucleotide

<400>  37
gaatgtaggt agaaccttcg g                                                 21


<210>  38
<211>  18
<212>  DNA
<213>  artificial sequence

<220>
<223>  synthetic oligonucleotide

<400>  38
acaccatagc aacggttg                                                     18


<210>  39
<211>  42
<212>  DNA
<213>  artificial sequence

<220>
<223>  synthetic oligonucleotide

<400>  39
ttgctcacgt tccgtaacta ccttgtgaag ctctgttagc tg                          42


<210>  40
<211>  42
<212>  DNA
<213>  artificial sequence

<220>
<223>  synthetic oligonucleotide

<400>  40
tcaagcatca tggcaccaca aatcagttac aaagctactc cc                          42


<210>  41
<211>  22
<212>  DNA
<213>  artificial sequence

<220>
<223>  synthetic oligonucleotide

<400>  41
ggaaatgctt tagcaagacc at                                                22


<210>  42
<211>  20
<212>  DNA
<213>  artificial sequence

<220>
<223>  synthetic oligonucleotide

<400>  42
tggtgaacat gccacagtta                                                   20


<210>  43
<211>  21
<212>  DNA
<213>  artificial sequence

<220>
<223>  synthetic oligonucleotide

<400>  43
gaatgtaggt agaaccttcg g                                                 21


<210>  44
<211>  20
<212>  DNA
<213>  artificial sequence

<220>
<223>  synthetic oligonucleotide

<400>  44
ctctaactgt ggcatgttca                                                   20

<210>  45
<211>  41
<212>  DNA
<213>  artificial sequence

<220>
<223>  synthetic oligonucleotide

<400>  45
tgctttagca agaccatcag ctcttatccg actcgcaatg t                          41


<210>  46
<211>  43
<212>  DNA
<213>  artificial sequence

<220>
<223>  synthetic oligonucleotide

<400>  46
ggtagttacg gaacgtgagc aaccaaaatc atcacttgtg tgg                        43


<210>  47
<211>  22
<212>  DNA
<213>  artificial sequence

<220>
<223>  synthetic oligonucleotide

<400>  47
acagagcttc acaaagtgtt tg                                               22


<210>  48
<211>  20
<212>  DNA
<213>  artificial sequence

<220>
<223>  synthetic oligonucleotide

<400>  48
attgcatcaa gcatcatggc                                                  20


<210>  49
<211>  20
<212>  DNA
<213>  artificial sequence

<220>
<223>  synthetic oligonucleotide

<400>  49
tctcgcacag aattgtactg                                                  20


<210>  50
<211>  19

<212>  DNA
<213>  artificial sequence

<220>
<223>  synthetic oligonucleotide

<400>  50
tgcttcaatg ctgtgttct                                                    19


<210>  51
<211>  43
<212>  DNA
<213>  artificial sequence

<220>
<223>  synthetic oligonucleotide

<400>  51
ctattcccgt gaagcggcaa tagacaacag agattgtaag gct                         43


<210>  52
<211>  40
<212>  DNA
<213>  artificial sequence

<220>
<223>  synthetic oligonucleotide

<400>  52
gctcgatgaa gtcctttggg aagtaatgca cccgaacctt                             40


<210>  53
<211>  19
<212>  DNA
<213>  artificial sequence

<220>
<223>  synthetic oligoucleotide

<400>  53
tgtggtgtct ggtattgcc                                                    19


<210>  54
<211>  21
<212>  DNA
<213>  artificial sequence

<220>
<223>  synthetic oligonucleotide

<400>  54
tctgctataa cacttgccga a                                                 21


<210>  55
<211>  19
<212>  DNA
<213>  artificial sequence

<220>
<223>  synthetic oligonucleotide

<400>  55
gcctgatcaa tcacgactt                                                    19


<210>  56
<211>  21
<212>  DNA
<213>  artificial sequence

<220>
<223>  synthetic oligonucleotide

<400>  56
ttactcgtgc ttctatgaag g                                                 21


<210>  57
<211>  38
<212>  DNA
<213>  artificial sequence

<220>
<223>  synthetic oligonucleotide

<400>  57
ggctggctgc ccaatcttag gacaattgga gatggcat                               38


<210>  58
<211>  39
<212>  DNA
<213>  artificial sequence

<220>
<223>  synthetic oligonucleotide

<400>  58
aaatgccctt cagcagcctt agctccacct tgagtaacc                              39


<210>  59
<211>  21
<212>  DNA
<213>  artificial sequence

<220>
<223>  synthetic oligonucleotide

<400>  59
tcctctgttc gagatccgat a                                                 21


<210>  60
<211>  22
<212>  DNA
<213>  artificial sequence

<220>
<223>  synthetic oligonucleotide

<400>  60
cgtgaagcaa ttgagttgac at                                                22

<210>  61
<211>  21
<212>  DNA
<213>  artificial sequence

<220>
<223>  synthetic oligonucleotide

<400>  61
agaactgcca ttactgtctt c                                                 21

<210>  62
<211>  20
<212>  DNA
<213>  artificial sequence

<220>
<223>  synthetic oligonucleotide

<400>  62
gatcgacttc tccagtatgc                                                   20

<210>  63
<211>  40
<212>  DNA
<213>  artificial sequence

<220>
<223>  synthetic oligonucleotide

<400>  63
aacaatgcga tgacgtggtg tattccactg tgggtcatct                             40

<210>  64
<211>  41
<212>  DNA
<213>  artificial sequence

<220>
<223>  synthetic oligonucleotide

<400>  64
caacatgagt cctcccacga tcaatgtctc cactcgactg t                           41

<210>  65
<211>  21
<212>  DNA
<213>  artificial sequence

<220>
<223>  synthetic oligonucleotide

<400>  65
actgttgatc ggactaggac t                                                 21

<210>  66
<211>  18
<212>  DNA
<213>  artificial sequence

<220>
<223>  synthetic oligonucleotide

<400>  66
gtttgtgctg cagaagcc                                                    18


<210>  67
<211>  19
<212>  DNA
<213>  artificial sequence

<220>
<223>  synthetic oligonucleotide

<400>  67
cctctgcgaa gcattactt                                                   19


<210>  68
<211>  22
<212>  DNA
<213>  artificial sequence

<220>
<223>  synthetic oligonucleotide

<400>  68
catgtaacat tgcggaatta gg                                               22


<210>  69
<211>  44
<212>  DNA
<213>  artificial sequence

<220>
<223>  synthetic oligonucleotide

<400>  69
tcatcacttg tatggtgcca ggcaagcaat atgatggttg tcac                       44


<210>  70
<211>  41
<212>  DNA
<213>  artificial sequence

<220>
<223>  synthetic oligonucleotide

<400>  70
gccacagttc gtggaagtag tttgaaggga gctgtgaatt c                          41


<210>  71
<211>  18

<212>  DNA
<213>  artificial sequence

<220>
<223>  synthetic oligonucleotide

<400>  71
atgcttggtg aaggaggc                                                    18


<210>  72
<211>  21
<212>  DNA
<213>  artificial sequence

<220>
<223>  synthetic oligonucleotide

<400>  72
aatacaatct ggccttcagg t                                                21


<210>  73
<211>  19
<212>  DNA
<213>  artificial sequence

<220>
<223>  synthetic oligonucleotide

<400>  73
aattgcgatc agcagtcat                                                   19


<210>  74
<211>  20
<212>  DNA
<213>  artificial sequence

<220>
<223>  synthetic oligonucleotide

<400>  74
tgctgtcttg agtgattgag                                                  20


<210>  75
<211>  40
<212>  DNA
<213>  artificial sequence

<220>
<223>  synthetic oligonucleotide

<400>  75
gccaagctag cagccactct agaatctagt ccgaatgagc                            40


<210>  76
<211>  41
<212>  DNA
<213>  artificial sequence

<220>
<223>  synthetic oligonucleotide

<400>  76
gtcacaagtg cctgtgggat gataaagcct gagtgtacga a                          41


<210>  77
<211>  18
<212>  DNA
<213>  artificial sequence

<220>
<223>  synthetic oligonucleotide

<400>  77
gctgcattgt cttctgcg                                                    18


<210>  78
<211>  21
<212>  DNA
<213>  artificial sequence

<220>
<223>  synthetic oligonucleotide

<400>  78
attcctcaag cctgatgaga c                                                21


<210>  79
<211>  20
<212>  DNA
<213>  artificial sequence

<220>
<223>  synthetic oligonucleotide

<400>  79
aacgaatgag gttgttgaca                                                  20


<210>  80
<211>  20
<212>  DNA
<213>  artificial sequence

<220>
<223>  synthetic oligonucleotide

<400>  80
agtctcctgc gttatacaga                                                  20


<210>  81
<211>  42
<212>  DNA
<213>  artificial sequence

<220>
<223>  synthetic oligonucleotide

<400>  81
tgtgtgtcct tgaatagcct ccaacacagc taatgttcca ca                          42


<210>  82
<211>  42
<212>  DNA
<213>  artificial sequence

<220>
<223>  synthetic oligonucleotide

<400>  82
gtctctacag ccgaggtcct taggctaact cagcaatcag aa                          42


<210>  83
<211>  22
<212>  DNA
<213>  artificial sequence

<220>
<223>  synthetic oligonucleotide

<400>  83
ttaggttggt accagtcagt tg                                                22


<210>  84
<211>  22
<212>  DNA
<213>  artificial sequence

<220>
<223>  synthetic oligonucleotide

<400>  84
aaggatcttg tcacaagtcg tt                                                22


<210>  85
<211>  20
<212>  DNA
<213>  artificial sequence

<220>
<223>  synthetic oligonucleotide

<400>  85
attgccacaa tcactcaaga                                                   20


<210>  86
<211>  20
<212>  DNA
<213>  artificial sequence

<220>
<223>  synthetic oligonucleotide

<400>  86
ttgacctaga tcggttcctt                                                   20

<210>  87
<211>  40
<212>  DNA
<213>  artificial sequence

<220>
<223>  synthetic oligonucleotide

<400>  87
tgccgttcct atactagcca gactgctacc aggattgctc                              40


<210>  88
<211>  39
<212>  DNA
<213>  artificial sequence

<220>
<223>  synthetic oligonucleotide

<400>  88
tatacccttg ccgggtggtt caaggtggtg gtattggag                                39


<210>  89
<211>  22
<212>  DNA
<213>  artificial sequence

<220>
<223>  synthetic oligonucleotide

<400>  89
cctgaaggtc atcaaagatt gc                                                 22


<210>  90
<211>  21
<212>  DNA
<213>  artificial sequence

<220>
<223>  synthetic oligonucleotide

<400>  90
gccgcattcc atacattctt c                                                  21


<210>  91
<211>  20
<212>  DNA
<213>  artificial sequence

<220>
<223>  synthetic oligonucleotide

<400>  91
tgtaccttac atcgggtctc                                                    20


<210>  92
<211>  22

<212>  DNA
<213>  artificial sequence

<220>
<223>  synthetic oligonucleotide

<400>  92
gctgtattgg tcattatacc ga                                                22


<210>  93
<211>  40
<212>  DNA
<213>  artificial sequence

<220>
<223>  synthetic oligonucleotide

<400>  93
gcttcacgta aggcagcaga gaacagagga caagattggg                             40


<210>  94
<211>  40
<212>  DNA
<213>  artificial sequence

<220>
<223>  synthetic oligonucleotide

<400>  94
ccaaggtggt gccaatagtg ataaggatct cctgcacact                             40


<210>  95
<211>  19
<212>  DNA
<213>  artificial sequence

<220>
<223>  synthetic oligonucleotide

<400>  95
ttaggcttga ttgctggct                                                    19


<210>  96
<211>  21
<212>  DNA
<213>  artificial sequence

<220>
<223>  synthetic oligonucleotide

<400>  96
ttgtagaggc acgagtaaac c                                                 21


<210>  97
<211>  19
<212>  DNA
<213>  artificial sequence

<220>
<223>  synthetic oligonucleotide

<400>  97
accgttgtca ttgttgtca                                                    19


<210>  98
<211>  22
<212>  DNA
<213>  artificial sequence

<220>
<223>  synthetic oligonucleotide

<400>  98
tagagattgt ttcaggttgt gg                                                22


<210>  99
<211>  41
<212>  DNA
<213>  artificial sequence

<220>
<223>  synthetic oligonucleotide

<400>  99
gtgtgacctt gaaccgcttc tgattcctca tgcagctaag g                           41


<210>  100
<211>  41
<212>  DNA
<213>  artificial sequence

<220>
<223>  synthetic oligonucleotide

<400>  100
tgtcagtttc cactgcagat gtgctgcaat gagtgtggta t                           41


<210>  101
<211>  22
<212>  DNA
<213>  artificial sequence

<220>
<223>  synthetic oligonucleotide

<400>  101
tgaagattga tgcgtgatac ca                                                22


<210>  102
<211>  22
<212>  DNA
<213>  artificial sequence

<220>
<223>  synthetic oligonucleotide

<400>  102
aaggacatca taacctgtcg tt                                                22


<210>  103
<211>  21
<212>  DNA
<213>  artificial sequence

<220>
<223>  synthetic oligonucleotide

<400>  103
ttacttggct ccattattcg g                                                 21


<210>  104
<211>  19
<212>  DNA
<213>  artificial sequence

<220>
<223>  synthetic oligonucleotide

<400>  104
gtagcatact gtgcgatgt                                                    19


<210>  105
<211>  41
<212>  DNA
<213>  artificial sequence

<220>
<223>  synthetic oligonucleotide

<400>  105
atgggccgat ctcctgttag taaccgatta catcaagtgc c                           41


<210>  106
<211>  40
<212>  DNA
<213>  artificial sequence

<220>
<223>  synthetic oligonucleotide

<400>  106
tcacaaggca tgtcttggtg ttgactcaac cagtacacgc                             40


<210>  107
<211>  20
<212>  DNA
<213>  artificial sequence

<220>
<223>  synthetic oligonucleotide

<400>  107
aggtaccatt cacttgaccg                                                   20

<210>  108
<211>  18
<212>  DNA
<213>  artificial sequence

<220>
<223>  synthetic oligonucleotide

<400>  108
aagcacaagt ccagcgag                                                    18


<210>  109
<211>  22
<212>  DNA
<213>  artificial sequence

<220>
<223>  synthetic oligonucleotide

<400>  109
aggcaatact atcctatgga ct                                               22


<210>  110
<211>  22
<212>  DNA
<213>  artificial sequence

<220>
<223>  synthetic oligonucleotide

<400>  110
ccttcaagcg gatatactat cc                                               22


<210>  111
<211>  40
<212>  DNA
<213>  artificial sequence

<220>
<223>  synthetic oligonucleotide

<400>  111
ttaatcggga catcacactg gcaccagctg cacaacttac                            40


<210>  112
<211>  36
<212>  DNA
<213>  artificial sequence

<220>
<223>  synthetic oligonucleotide

<400>  112
tggcgggaca tctggagacg attgctgtcc gaagaa                                36


<210>  113
<211>  18

<212>  DNA
<213>  artificial sequence

<220>
<223>  syntehtic oligonucleotide

<400>  113
cacggagtgc tcgatgag                                                    18


<210>  114
<211>  18
<212>  DNA
<213>  artificial sequence

<220>
<223>  synthetic oligonucleotide

<400>  114
ctgatgctgc tcgactgt                                                    18


<210>  115
<211>  22
<212>  DNA
<213>  artificial sequence

<220>
<223>  synthetic oligonucleotide

<400>  115
acatgatggt agtaactgaa cg                                               22


<210>  116
<211>  20
<212>  DNA
<213>  artificial sequence

<220>
<223>  synthetic oligonucleotide

<400>  116
ttaacattgt gaggcggatt                                                  20


<210>  117
<211>  39
<212>  DNA
<213>  artificial sequence

<220>
<223>  synthetic oligonucleotide

<400>  117
actccctcga acggtagcac cttcttcatc aggcatcat                             39


<210>  118
<211>  41
<212>  DNA
<213>  artificial sequence

<220>
<223>  synthetic oligoncleotide

<400>  118
cgctatgagt tcactgcacc ataagacatt acgcacacca t                          41


<210>  119
<211>  18
<212>  DNA
<213>  artificial sequence

<220>
<223>  synthetic oligonucleotide

<400>  119
tcatcactgg tgtggtgc                                                    18


<210>  120
<211>  20
<212>  DNA
<213>  artificial sequence

<220>
<223>  synthetic oligonucleotide

<400>  120
tgagtactgc aaccattgct                                                  20


<210>  121
<211>  18
<212>  DNA
<213>  artificial sequence

<220>
<223>  synthetic oligonucleotide

<400>  121
cgaagacagt gatggagc                                                    18


<210>  122
<211>  21
<212>  DNA
<213>  artificial sequence

<220>
<223>  synthetic oligonucleotide

<400>  122
cactcagcct tattgtctga a                                                21


<210>  123
<211>  40
<212>  DNA
<213>  artificial sequence

<220>
<223>  synthetic oligonucleotide

<400>  123
cgtcctccag gtacatggcc tcctcttgat gtgttaggtg                              40


<210>  124
<211>  43
<212>  DNA
<213>  artificial sequence

<220>
<223>  synthetic oligonucleotide

<400>  124
ttagaaccct tgtgtgtcag ctatcatctg tgattggaac cag                          43


<210>  125
<211>  22
<212>  DNA
<213>  artificial sequence

<220>
<223>  syntehtic oligonucleotide

<400>  125
aatttggtct ccctggttgt at                                                 22


<210>  126
<211>  22
<212>  DNA
<213>  artificial sequence

<220>
<223>  synthetic oligonucleotide

<400>  126
cacatggcat ctttacacca ag                                                 22


<210>  127
<211>  20
<212>  DNA
<213>  artificial sequence

<220>
<223>  synthetic oligonucleotide

<400>  127
agcaatcaat tctgtcggtt                                                    20


<210>  128
<211>  21
<212>  DNA
<213>  artificial sequence

<220>
<223>  synthetic oligonucleotide

<400>  128
acaacattct cggtattacc c                                                  21

<210>  129
<211>  43
<212>  DNA
<213>  artificial sequence

<220>
<223>  synthetic oligonucleotide

<400>  129
cccttcggcc aaacaaattg cgtctcatca atgcattaca agc                43


<210>  130
<211>  40
<212>  DNA
<213>  artificial sequence

<220>
<223>  synthetic oligonucleotide

<400>  130
aaggaagtgg tgctcggtta cgttgtgaat ccgtagccaa                    40


<210>  131
<211>  22
<212>  DNA
<213>  artificial sequence

<220>
<223>  synthetic oligonucleotide

<400>  131
tttacctgga ggcaaaagat ca                                       22


<210>  132
<211>  21
<212>  DNA
<213>  artificial sequence

<220>
<223>  synthetic oligonucleotide

<400>  132
ttgaagtgga aggaaacgga t                                        21


<210>  133
<211>  21
<212>  DNA
<213>  artificial sequence

<220>
<223>  synthetic oligonucleotide

<400>  133
agacaagcaa ttccttactg g                                        21


<210>  134
<211>  22

<212>  DNA
<213>  artificial sequence

<220>
<223>  synthetic oligonucleotide

<400>  134
tctcaagtct agtagaggtg aa                                                22


<210>  135
<211>  42
<212>  DNA
<213>  artificial sequence

<220>
<223>  synthetic oligonucleotide

<400>  135
acgatggcag aaagatgact ctatcctgct tcattacacg ag                          42


<210>  136
<211>  42
<212>  DNA
<213>  artificial sequence

<220>
<223>  synthetic oligonucleotide

<400>  136
tccattcagt gaaggaaagg ctaaatgttg tttgccttga cg                          42


<210>  137
<211>  22
<212>  DNA
<213>  artificial sequence

<220>
<223>  synthetic oligonucleotide

<400>  137
agggaaaccc aaagttagga aa                                                22


<210>  138
<211>  22
<212>  DNA
<213>  artificial sequence

<220>
<223>  synthetic oligonucleotide

<400>  138
ttcacaaggt ccagtcttat gt                                                22


<210>  139
<211>  19
<212>  DNA
<213>  artificial sequence

<220>
<223>  synthetic oligonucleotide

<400>  139
aacaacctcg aacatggac                                                    19


<210>  140
<211>  18
<212>  DNA
<213>  artificial sequence

<220>
<223>  synthetic oligonucleotide

<400>  140
gacggctagt gtctgact                                                     18


<210>  141
<211>  41
<212>  DNA
<213>  artificial sequence

<220>
<223>  synthetic oligonucleotide

<400>  141
gttcgccaac attgacacca gatcctcagc tttcagcaat t                           41


<210>  142
<211>  38
<212>  DNA
<213>  artificial sequence

<220>
<223>  synthetic oligonucleotide

<400>  142
ctacgagagg cggcacatgc ctcggcaata gcttgaat                               38


<210>  143
<211>  18
<212>  DNA
<213>  artificial sequence

<220>
<223>  synthetic oligonucleotide

<400>  143
aatgtactgc cgtgtgct                                                     18


<210>  144
<211>  19
<212>  DNA
<213>  artificial sequence

<220>
<223>  synthetic oligonucleotide

<400>  144
ggcgacatga acatcagga                                                    19


<210>  145
<211>  20
<212>  DNA
<213>  artificial sequence

<220>
<223>  synthetic oligonucleotide

<400>  145
cggcagtatt ggtgatgtaa                                                   20


<210>  146
<211>  21
<212>  DNA
<213>  artificial sequence

<220>
<223>  synthetic oligonucleotide

<400>  146
aatctgcttg aagctcctta g                                                 21


<210>  147
<211>  41
<212>  DNA
<213>  artificial sequence

<220>
<223>  synthetic oligonuleotide

<400>  147
aggcgacaat tcataggctt ccccttcttc accatctgct c                           41


<210>  148
<211>  44
<212>  DNA
<213>  artificial sequence

<220>
<223>  synthetic oligonucleotide

<400>  148
agcagaattg gccacaaaga gtgaggattt gtttgcagaa gatc                        44


<210>  149
<211>  22
<212>  DNA
<213>  artificial sequence

<220>
<223>  synthetic oligonucleotide

<400>  149
ctcatccttg tgtcttcctg ag                                                22

<210>  150
<211>  22
<212>  DNA
<213>  artificial sequence

<220>
<223>  synthetic oligonucleotide

<400>  150
ggtgacaaag aagggtagaa ct                                                22


<210>  151
<211>  20
<212>  DNA
<213>  artificial sequence

<220>
<223>  synthetic oligonucleotide

<400>  151
tcatctgagg ttagagtccc                                                   20


<210>  152
<211>  22
<212>  DNA
<213>  artificial sequence

<220>
<223>  synthetic oligonucleotide

<400>  152
gaattatctg gttactgctc ca                                                22


<210>  153
<211>  40
<212>  DNA
<213>  artificial sequence

<220>
<223>  synthetic oligonucleotide

<400>  153
cgcaaggaga gaagggatga cacaagattg ttgaccgctt                             40


<210>  154
<211>  42
<212>  DNA
<213>  artificial sequence

<220>
<223>  synthetic oligonucleotide

<400>  154
gacttcggag aagtcaagtc caacatcaaa ctacaggtgg tg                          42


<210>  155
<211>  21

<212>  DNA
<213>  artificial sequence

<220>
<223>  synthetic oligonucleotide

<400>  155
aacggttgag ggacctaaat c                                                21


<210>  156
<211>  19
<212>  DNA
<213>  artificial sequence

<220>
<223>  synthetic oligonucleotide

<400>  156
aaagagcctg ggcatcttt                                                   19


<210>  157
<211>  21
<212>  DNA
<213>  artificial sequence

<220>
<223>  synthetic oligonucleotide

<400>  157
cctgaacttg gttgatagag g                                                21


<210>  158
<211>  20
<212>  DNA
<213>  artificial sequence

<220>
<223>  synthetic oligonucleotide

<400>  158
aaagctgaca gcaacaattc                                                  20


<210>  159
<211>  42
<212>  DNA
<213>  artificial sequence

<220>
<223>  synthetic oligoucleotide

<400>  159
ggatgcaatg ctgcaacttg attcaggtct tccttcaatg tc                         42


<210>  160
<211>  40
<212>  DNA
<213>  artificial sequence

<220>
<223>  synthetic oligonucleotide

<400>  160
ttgagggtca tcagctgaga atgtaaatcc ctgcagtcgg                              40


<210>  161
<211>  20
<212>  DNA
<213>  artificial sequence

<220>
<223>  synthetic oligonucleotide

<400>  161
cccaaatgct aagacctgga                                                    20


<210>  162
<211>  18
<212>  DNA
<213>  artificial sequence

<220>
<223>  synthetic oligonucleotide

<400>  162
taagcccagc ggtaaacc                                                      18


<210>  163
<211>  1008
<212>  DNA
<213>  Homo sapiens

<400>  163
atggggaagg tgaaggtcgg agtcaacgga tttggtcgta ttgggcgcct ggtcaccagg        60

gctgctttta actctggtaa agtggatatt gttgccatca atgacccctt cattgacctc       120

aactacatgg tttacatgtt ccaatatgat tccacccatg gcaaattcca tggcaccgtc       180

aaggctgaga acgggaagct tgtcatcaat ggaaatccca tcaccatctt ccaggagcga       240

gatccctcca aaatcaagtg gggcgatgct ggcgctgagt acgtcgtgga gtccactggc       300

gtcttcacca ccatggagaa ggctggggct catttgcagg ggggagccaa aagggtcatc       360

atctctgccc cctctgctga tgcccccatg ttcgtcatgg gtgtgaacca tgagaagtat       420

gacaacagcc tcaagatcat cagcaatgcc tcctgcacca ccaactgctt agcacccctg       480

gccaaggtca tccatgacaa ctttggtatc gtggaaggac tcatgaccac agtccatgcc       540

atcactgcca cccagaagac tgtggatggc ccctccggga aactgtggcg tgatggccgc       600

ggggctctcc agaacatcat ccctgcctct actggcgctg ccaaggctgt gggcaaggtc       660

atccctgagc tgaacgggaa gctcactggc atggccttcc gtgtccccac tgccaacgtg       720

tcagtggtgg acctgacctg ccgtctagaa aaacctgcca aatatgatga catcaagaag       780

gtggtgaagc aggcgtcgga gggccccctc aagggcatcc tgggctacac tgagcaccag 840

gtggtctcct ctgacttcaa cagcgacacc cactcctcca cctttgacgc tggggctggc 900

attgccctca acgaccactt tgtcaagctc atttcctggt atgacaacga atttggctac 960

agcaacaggg tggtggacct catggcccac atggcctcca aggagtaa 1008

<210> 164
<211> 268
<212> DNA
<213> artificial sequence

<220>
<223> synthetic polynucleotide

<400> 164
acggatttgg tcgtattggg cgcctggtca ccagggctgc ttttaactct ggtaaagtgg 60

atattgttgc catcaatgac cccttcattg acctcaacta catggtttac atgttccaat 120

atgattccac ccatggcaaa ttccatggca ccgtcaaggc tgagaacggg aagcttgtca 180

tcaatggaaa tcccatcacc atcttccagg agcgagatcc ctccaaaatc aagtggggcg 240

atgctggcgc tgagtacgtc gtggagtc 268

<210> 165
<211> 272
<212> DNA
<213> artificial sequence

<220>
<223> synthetic polynucleotide

<400> 165
tcttccagga gcgagatccc tccaaaatca agtggggcga tgctggcgct gagtacgtcg 60

tggagtccac tggcgtcttc accaccatgg agaaggctgg ggctcatttg caggggggag 120

ccaaaagggt catcatctct gccccctctg ctgatgcccc catgttcgtc atgggtgtga 180

accatgagaa gtatgacaac agcctcaaga tcatcagcaa tgcctcctgc accaccaact 240

gcttagcacc cctggccaag gtcatccatg ac 272

<210> 166
<211> 235
<212> DNA
<213> artificial sequence

<220>
<223> synthetic polynucleotide

<400> 166
catgttcgtc atgggtgtga accatgagaa gtatgacaac agcctcaaga tcatcagcaa 60

tgcctcctgc accaccaact gcttagcacc cctggccaag gtcatccatg acaactttgg 120

tatcgtggaa ggactcatga ccacagtcca tgccatcact gccacccaga agactgtgga 180

tggcccctcc gggaaactgt ggcgtgatgg ccgcggggct ctccagaaca tcatc 235

<210> 167
<211> 1686
<212> DNA
<213> Sigma virus

<400> 167
atggtacatt acgagactca tattctattc atccatctct ggatgttggc actgatattt 60

atcaccacct cagtatggtt ggccgccagc caaaaaactt ttaccccgga tttggtgttc 120

cctgaaatga atcgtaatag ctcttggagt gtggcaaact atggagagat attatgccca 180

acatcctttc agtcatacga ccccaagaag caccagatcc tgacacgtgt cctagttgag 240

agaccaagcc taaacactga cacaaaggtg gagggctaca catgccataa ggtgaagtat 300

gagaccatct gtgatatgcc atggtatttc tccccaacca tttcacactc catctctccg 360

cttagggtaa aagagtctga gtgtaaagat gctatagcag agcaccaatt gggcacccat 420

gtctcgctga gctttccccc tgaggactgt tcttggaact cagtgaacac gaaggcgtac 480

gaggatatta tagttaaaga ccatcctgta atgttggatc catacactaa taattatgtt 540

gacgcaatat ttccaggtgg tatttcaagc ccagggatgg gggggaccat tcatgacgat 600

atgatgtggg tatccaaaga tctggcagtg agcccagaat gctctgggtg gcaacaaagt 660

atggggctta tttattcatc tcggctgtat ggagagcggg agccgatgct ggaagtcggc 720

tccatccaca tcgagggcca cagggacaag aacctcactt tagcctgccg gatttccttc 780

tgtggtgaga taggggtgag gtttcacgat ggggagtgga tgaaggtatc tgtaaatctt 840

gaccatccta actctgtgac attccaagta actgatttcc ctccgtgtcc tcccggtacc 900

actatccaaa ctgccgttgt ggaaaatata aacccagaga ttcaagagct cactgtcaac 960

atgatgtaca gattgaagtg tcaagagacc atctcaaaaa tggtatctgg gcttccgacc 1020

tctgcccttg atctctccta tttgattcaa gtgcaggagg gacctggtat tgtttataag 1080

agagagaagg gtgtcttgta ccaaagtgtc ggaatgtacc aatacataga cacagtaaca 1140

ctgaacacgg aggagaacca actgggtgaa aacgcgaggg ggcaaaaggt cttttggacc 1200

gaatggagcg attcaccgac acgccccgac ctccaggaag ggatcaatgg gattgttaaa 1260

tatgaggggc agataagagt gcctttagga atgtccctga gattagaggc tgcaacagaa 1320

ttaatgtggg gacaccctgt ccacaccgtg tctcacccta tacttcatgt tatcagtaat 1380

cacacagaac agtctgtaac aacttggaac aggggtgtta attccacaaa cctgataggc 1440

ctggcaactc gatcaatatc aggtttctac aatgatttga agttgtattt gatcttggca 1500

ctgatagtcg tttcaattgt cgcactggtt gtcttagatg tgattccctt taagtatatt 1560

ttatttatac tatgcccacc tctgctgttg tgtaggttca taaagtgttc acgaaggagg 1620

cctgaaactg gggacaggta tcacgtagaa tataaccgac ctggacaagt gtccagcgcg 1680

ttctaa 1686

<210> 168
<211> 314
<212> DNA
<213> artificial sequence

<220>
<223> synthetic polynucleotide

<400> 168
caagaagcac cagatcctga cacgtgtcct agttgagaga ccaagcctaa acactgacac 60

aaaggtggag ggctacacat gccataaggt gaagtatgag accatctgtg atatgccatg 120

gtatttctcc ccaaccattt cacactccat ctctccgctt agggtaaaag agtctgagtg 180

taaagatgct atagcagagc accaattggg cacccatgtc tcgctgagct ttccccctga 240

ggactgttct tggaactcag tgaacacgaa ggcgtacgag gatattatag ttaaagacca 300

tcctgtaatg ttgg 314

<210> 169
<211> 286
<212> DNA
<213> artificial sequence

<220>
<223> synthetic polynucleotide

<400> 169
ggaccattca tgacgatatg atgtgggtat ccaaagatct ggcagtgagc ccagaatgct 60

ctgggtggca acaaagtatg gggcttattt attcatctcg gctgtatgga gagcgggagc 120

cgatgctgga agtcggctcc atccacatcg agggccacag ggacaagaac ctcactttag 180

cctgccggat ttccttctgt ggtgagatag gggtgaggtt tcacgatggg gagtggatga 240

aggtatctgt aaatcttgac catcctaact ctgtgacatt ccaagt 286

<210> 170
<211> 241
<212> DNA
<213> artificial sequence

<220>
<223> synthetic polynucleotide

<400> 170
aaactgccgt tgtggaaaat ataaacccag agattcaaga gctcactgtc aacatgatgt 60

acagattgaa gtgtcaagag accatctcaa aaatggtatc tgggcttccg acctctgccc 120

ttgatctctc ctatttgatt caagtgcagg agggacctgg tattgtttat aagagagaga 180

agggtgtctt gtaccaaagt gtcggaatgt accaatacat agacacagta acactgaaca 240

c 241

<210> 171
<211> 18
<212> DNA
<213> artificial sequence

<220>
<223> synthetic oligonucleotide

<400> 171
tcttccagga gcgagatc 18

<210> 172
<211> 18
<212> DNA
<213> artificial sequence

<220>
<223> synthetic oligonucleotide

<400> 172
gtcatggatg accttggc 18

<210> 173
<211> 36
<212> DNA
<213> artificial sequence

<220>
<223> synthetic oligonucleotide

<400> 173
gcaaatgagc cccagccttg agtacgtcgt ggagtc 36

<210> 174
<211> 40
<212> DNA
<213> artificial sequence

<220>
<223> synthetic oligonucleotide

<400> 174
tgttcgtcat gggtgtgaac cgaggcattg ctgatgatct 40

<210> 175
<211> 19
<212> DNA
<213> artificial sequence

<220>
<223> synthetic oligonucleotide

<400> 175
ctccatggtg gtgaagacg 19

<210>  176
<211>  22
<212>  DNA
<213>  artificial sequence

<220>
<223>  synthetic oligonucleotide

<400>  176
tgagaagtat gacaacagcc tc                                                22


<210>  177
<211>  19
<212>  DNA
<213>  artificial sequence

<220>
<223>  synthetic oligonucleotide

<400>  177
acggatttgg tcgtattgg                                                    19


<210>  178
<211>  18
<212>  DNA
<213>  artificial sequence

<220>
<223>  synthetic oligonucleotide

<400>  178
gactccacga cgtactca                                                     18


<210>  179
<211>  39
<212>  DNA
<213>  artificial sequence

<220>
<223>  synthetic oligonucleotide

<400>  179
tggaatttgc catgggtgga atgttgccat caatgaccc                              39


<210>  180
<211>  37
<212>  DNA
<213>  artificial sequence

<220>
<223>  synthetic oligonucleotide

<400>  180
gcaccgtcaa ggctgagaag atctcgctcc tggaaga                                37


<210>  181

<211>  22
<212>  DNA
<213>  artificial sequence

<220>
<223>  synthetic oligonucleotide

<400>  181
accatgtagt tgaggtcaat ga                                                22


<210>  182
<211>  22
<212>  DNA
<213>  artificial sequence

<220>
<223>  synthetic oligonucleotide

<400>  182
atcaatggaa atcccatcac ca                                                22


<210>  183
<211>  18
<212>  DNA
<213>  artificial sequence

<220>
<223>  synthetic oligonucleotide

<400>  183
catgttcgtc atgggtgt                                                     18


<210>  184
<211>  19
<212>  DNA
<213>  artificial sequence

<220>
<223>  synthetic oligonucleotide

<400>  184
gatgatgttc tggagagcc                                                    19


<210>  185
<211>  41
<212>  DNA
<213>  artificial sequence

<220>
<223>  synthetic oligonucleotide

<400>  185
ggtgctaagc agttggtggt atgagaagta tgacaacagc c                           41


<210>  186
<211>  40
<212>  DNA
<213>  artificial sequence

<220>
<223>  synthetic oligonucleotide

<400>  186
gccaaggtca tccatgacaa ctcagtgatg gcatggactg                              40


<210>  187
<211>  21
<212>  DNA
<213>  artificial sequence

<220>
<223>  synthetic oligonucleotide

<400>  187
aggcattgct gatgatcttg a                                                  21


<210>  188
<211>  21
<212>  DNA
<213>  artificial sequence

<220>
<223>  synthetic oligonucleotide

<400>  188
ggtatcgtgg aaggactcat g                                                  21


<210>  189
<211>  22
<212>  DNA
<213>  artificial sequence

<220>
<223>  synthetic oligonucleotide

<400>  189
ggaccattca tgacgatatg at                                                 22


<210>  190
<211>  22
<212>  DNA
<213>  artificial sequence

<220>
<223>  synthetic oligonucleotide

<400>  190
acttggaatg tcacagagtt ag                                                 22


<210>  191
<211>  38
<212>  DNA
<213>  artificial sequence

<220>
<223>  synthetic oligonucleotide

<400>  191
gacttccagc atcggctccc tgggtggcaa caaagtat                                38

<210>  192
<211>  39
<212>  DNA
<213>  artificial sequence

<220>
<223>  synthetic oligonucleotide

<400>  192
ggctccatcc acatcgaggc ctatctcacc acagaagga                               39

<210>  193
<211>  20
<212>  DNA
<213>  artificial sequence

<220>
<223>  synthetic oligonucleotide

<400>  193
ctctccatac agccgagatg                                                    20

<210>  194
<211>  22
<212>  DNA
<213>  artificial sequence

<220>
<223>  synthetic oligonucleotide

<400>  194
cagggacaag aacctcactt ta                                                 22

<210>  195
<211>  18
<212>  DNA
<213>  artificial sequence

<220>
<223>  synthetic oligonucleotide

<400>  195
aaactgccgt tgtggaaa                                                      18

<210>  196
<211>  22
<212>  DNA
<213>  artificial sequence

<220>
<223>  synthetic oligonucleotide

<400>  196
gtgttcagtg ttactgtgtc ta                                                 22

<210>  197
<211>  39
<212>  DNA
<213>  artificial sequence

<220>
<223>  synthetic oligonucleotide

<400>  197
agaggtcgga agcccagata gctcactgtc aacatgatg                              39


<210>  198
<211>  40
<212>  DNA
<213>  artificial sequence

<220>
<223>  synthetic oligonucleotide

<400>  198
tcaagtgcag gagggacctg tattggtaca ttccgacact                             40


<210>  199
<211>  22
<212>  DNA
<213>  artificial sequence

<220>
<223>  synthetic oligonucleotide

<400>  199
gagatggtct cttgacactt ca                                                22


<210>  200
<211>  21
<212>  DNA
<213>  artificial sequence

<220>
<223>  synthetic oligonucleotide

<400>  200
gagagaaggg tgtcttgtac c                                                 21


<210>  201
<211>  19
<212>  DNA
<213>  artificial sequence

<220>
<223>  synthetic oligonucleotide

<400>  201
caagaagcac cagatcctg                                                    19


<210>  202

<211>  21
<212>  DNA
<213>  artificial sequence

<220>
<223>  synthetic oligonucleotide

<400>  202
ccaacattac aggatggtct t                                                21


<210>  203
<211>  44
<212>  DNA
<213>  artificial sequence

<220>
<223>  synthetic oligonucleotide

<400>  203
gcggagagat ggagtgtgaa atatgccata aggtgaagta tgag                       44


<210>  204
<211>  39
<212>  DNA
<213>  artificial sequence

<220>
<223>  synthetic oligonucleotide

<400>  204
agagcaccaa ttgggcaccg agttccaaga acagtcctc                             39


<210>  205
<211>  22
<212>  DNA
<213>  artificial sequence

<220>
<223>  synthetic oligonucleotide

<400>  205
taccatggca tatcacagat gg                                               22


<210>  206
<211>  19
<212>  DNA
<213>  artificial sequence

<220>
<223>  synthetic oligonucleotide

<400>  206
atgtctcgct gagctttcc                                                   19

## Description

The invention pertains to the field of Viral Hemorrhagic Fevers (VHFs) diagnosis. The invention relates to a method for detecting a Hemorrhagic Fever virus (HFV) by isothermal amplification of a target sequence of the HFV nucleic acid. The invention relates also to a kit for performing the method.

Viral Hemorrhagic Fevers (VHFs) are acute infections with high case fatality rates caused by fourdistinct families of RNA viruses: Arenavirus, Bunyavirus, Filovirus, and Flavivirus (Meltzer et al., Infect. Dis. Clin. N. Am., 2012, 26, 479-496). Infections by Hemorrhagic Fever Viruses (HFVs) are associated with a wide spectrum of clinical manifestations such as diarrhea, myalgia, cough, headache, pneumonia, encephalopathy, and hepatitis. Hemorrhage is the characteristic manifestation, although nonhemorrhagic infections are also common. Although nearly all VHF agents are endemic in tropical or subtropical regions, infections can also occur outside these regions, for example, in returning travelers.

The ongoing Ebola virus disease (EVD) epidemic is the largest in history, affecting multiple countries in West Africa and putting neighboring countries at risk (Dixon et al., MMWR Morb. Mortal. Wkly. Rep. 2014, Jun 27; 63(25):548-51).There are five subtypes or species of Ebola virus (or Ebolavirus), a Filovirus. Four have caused diseases in humans: *Zaire ebolavirus* (Ebola virus (EBOV) or Ebola-Zaire (ZEBOV)), *Taï Forest ebolavirus* (Taï Forest virus (TAFV) or Ebola-Taï Forest (TEBOV), formerly known as Ebola-Ivory Coast), *Sudan ebolavirus* (Sudan virus (SUDV) or Ebola-Sudan (SEBOV)), and *Bundibugyo ebolavirus* (Bundibugyo virus (BDBV) or Ebola-Bundibugyo (BEBOV). The fifth, *Reston ebolavirus* (Reston virus (RESTV) or Ebola-Reston (REBOV)), has only caused diseases in nonhuman primates. Marburg hemorrhagic fever is a rare, severe type of HF caused by a Filovirus, which affects both humans and non-humans primates. The structures of filoviruses are very similar. The virus genome is a negative-stranded RNA of 19 kb in length encoding seven viral structural proteins in the order nucleoprotein (NP), phosphoprotein (VP35), matrix protein (VP40), glycoprotein (GP), replication-transcription protein (VP30), matrix protein (VP24) and RNA-dependent RNA polymerase (L), with an additional soluble glycoprotein produced from an edited GP mRNA. The viral particle is formed of a Ribonucleocapsid (RNP) comprising the viral RNA genome and the L, NP, VP30 and VP35 proteins, surrounded by a host-membrane derived envelope comprising the glycoprotein and matrix proteins.

Lassa fever virus is caused by an Arenavirus endemic in parts of West Africa. The negative-stranded RNA genome comprises L segment (7.5 kb) and S segment (3.5 kb) encoding four viral structural proteins: nucleoprotein (NP), matrix protein (Z), glycoprotein (GP), and RNA-dependent RNA polymerase (L). The viral particle is formed of a Ribonucleocapsid (RNP) comprising the viral RNA genome and the L and NP proteins, surrounded by an envelope comprising the glycoprotein and matrix protein.

In the absence of bleeding or organ manifestation, a VHF is clinically difficult to diagnose, and the various etiologic agents can hardly be distinguished by clinical tests. Inexpensive, portable, and easy-to-use diagnostic devices are urgently needed to ensure rapid detection of HFVs at the point-of-care. Unfortunately, the availability of laboratory diagnosis in low-resource, high disease-burden areas is currently limited by cost, infrastructure, and personnel constraints (Pai et al., PLoS Med., 2012 Sep 4; 9(9):e1001306). Rapid nucleic acid testing for viral diseases can provide access to much-needed diagnostic methods in low-resource countries, especially for applications requiring fast turnaround times (Niemz et al., Trends Biotechnol., 2011 May; 29(5):240-50).

Common molecular detection methods by end point and real time Polymerase Chain Reaction (PCR) are valuable tools for pathogen detection and are widely used in clinical diagnostics because of high sensitivity and specificity. Various RT-PCR assays for the detection of VHFs are available (Grolla et al., Bull. Société Pathol. Exot., 2005 Sep; 98(3):205-9; Drosten et al., J. Clin. Microbiol., 2002 Jul; 40(7):2323-30; Ogawa et al., J. Virol. Methods, 2011, 171, 310-313). However, they are major problems in implementing these methods for near-patient testing in endemic areas, in particular, the need for trained personal as well as expensive and sophisticated equipment.

In contrast to the polymerase chain reaction (PCR) technology in which the reaction is carried out with a series of alternating temperature steps or cycles, isothermal amplification is carried out at a constant temperature, and does not require a thermal cycler. Among field-applicable isothermal nucleic acid amplification methods developed for rapid, simple, and cost-effective detection of pathogenic microorganisms in smaller size systems, the RT-LAMP (*reverse-transcription loop-mediated isothermal amplification*) appears to be promising assays, highly suited for on-site detection of EVD (Craw et al., Lab. Chip., 2012 Jun 19; 12(14):2469-86). The RT-LAMP assay uses a DNA polymerization enzyme with high strand-displacement activity and 4 primers, specifically designed to recognize 6 distinct regions on the target gene, to synthesize large amounts of target viral nucleic acids under a constant temperature, usually between 55-65°C (Notomi et al., Nucleic Acids Res., 2000 Jun 15; 28(12):e63). An additional pair of "loop primers" is used to further accelerate the reaction. The LAMP reaction yields high amount of amplification products, which can be detected either visually or by simple detectors. These large amounts of synthesized double strand DNA (dsDNA) yield large amounts of pyrophosphate ion byproducts, which combine with divalent metallic ion (such as Mg²⁺) to form an insoluble salt, resulting in a decrease of Mg²⁺ ion concentration as the LAMP reaction progresses (Goto et al., BioTechniques, 2009 Mar; 46(3):167-72). In addition, it has been reported that there is a strong correlation between the change in pH and the amplification yield during the LAMP, as hydrogen ions are released during the LAMP procedure. Due to these properties, real-time monitoring of the LAMP reaction can be achieved by: (i) fluorescence, using DNA intercaling dyes, fluorescent molecular beacon probes or a fluorescence metal indicator such as calcein; (ii) colorimetry, using a colored indicator for alkaline metal ions, such as hydroxy naphthol blue (Goto et al., BioTechniques, 2009 Mar; 46(3):167-72) or pH indicators (Tanner et al., BioTechniques, 2015 Feb; 58(2):59-68); (iii) turbidity, as the LAMP reaction produces large amounts of magnesium pyrophosphate (a white precipitate) and dsDNA, which allow visual inspection of results using a turbidimeter (Mori et al., Biochem. Biophys. Res. Commun., 2001 Nov 23; 289(1):150-4); (iv) electrochemically, using a pH meter for direct measurement of released hydrogen ions during the LAMP procedure (Xie et al., Chem. Commun., 2014 Oct 24; 50(100):15932-5), or using integrated electrodes for measuring decreases in current resulting from increasing binding of electrochemically-active DNA-binding redox reporters, such as Methylene Blue, to LAMP reaction products (Xie et al., Biosens. Bioelectron., 2014 May 15; 55:324-9); (v) enzyme-linked immunosorbent assays (ELISA) or lateral flow immunoassays based on the use of specific probes (Tsai et al., J. Virol. Methods., 2012 Apr; 181(1):117-24; Ravan H and Yazdanparast R., Anal. Biochem., 2013 Aug 15; 439(2):102-8); (vi) bioluminescence, through bioluminescent output of the coupled conversion of inorganic pyrophosphate produced stoichiometrically during nucleic acid synthesis to ATP by the enzyme ATP sulfurylase (Gandelman et al., PLoS ONE, 2010 Nov 30; 5(11)). In addition to its relative simplicity and low infrastructure costs, the RT-LAMP assay (i) has moderate incubation temperature leading to simplified heating and low power consumption, (ii) allows direct genetic amplification from target pathogens due to a superior tolerance to well-known PCR inhibitors such as blood (iii) demonstrates high specificity and sensitivity, and (iv) results in rapid detection often within 30 min (Mori Y and Notomi T., J. Infect. Chemother., 2009 Apr 1; 15(2):62-9; Kaneko et al., J. Biochem. Biophys. Methods., 2007 Apr 10; 70(3):499-501). However, RT-LAMP assays designed for filovirus diagnosis have been shown to be slightly less sensitive compared to equivalent TaqMan RT-PCR assays (Schurtleff et al., frontiers in Microbiology, 2015, 6, article 108).

The inventors have developed an RT-LAMP assay for the species-specific detection of HFVs, in particular major HFV agents including Ebola virus species, Lassa virus and Marburg virus. The assay which is based on the isothermal amplification of a specific target located within a gene coding for a protein of the viral ribonucleocapsid such as the L or NP gene, demonstrated equivalent analytical sensitivity and at least a 5 times faster time-to-result (15 minutes as compared to 80 minutes) as compared to the currently used reference real-time RT-PCR assay (RealStar® Ebolavirus RT-PCR Kit 1.0, Altona Diagnostics) as well as other published Ebola virus qRT-PCR assays (Grolla et al., Bull. Société Pathol. Exot., 2005 Sep; 98(3):205-9; Drosten et al., J. Clin. Microbiol., 2002 Jul; 40(7):2323-30) thereby showing great potential for point-of-care applications. Moreover, the RT-LAMP assay demonstrated superior clinical performances as compared to the RT-PCR assay, probably due to a higher tolerance to inhibitors, making it highly suitable for accurate and reliable diagnostic applications.

Therefore, a first aspect of the present invention relates to a method of detecting a Hemorrhagic Fever Virus (HFV) in a sample, comprising:
a) subjecting said sample to an isothermal nucleic acid amplification reaction using at least one pair of oligonucleotide primers for amplifying a target sequence of a HFV nucleic acid, wherein the target sequence is located within a gene of the HFV nucleic acid which codes for a protein of the viral ribonucleocapsid, and
b) detecting the presence of an amplification product for said target sequence.

In step a) of the method of the invention, the oligonucleotide primers which are contacted with the sample suspected of containing HFV are capable of hybridizing with the target sequence present in any HFV target nucleic acid present in said sample. The HFV target nucleic acid present in the sample is used as a template for generating an amplification product (amplicon). In step b) of the method of the invention, the detection of the presence or absence of the amplification product determines the presence or absence of an HFV in the sample.

The method of the invention is used for the detection of any HFV, in particular an RNA virus chosen from a Filovirus, Arenavirus, Bunyavirus, or Flavivirus. Filoviruses, Arenaviruses, and Bunyaviruses have a single-stranded negative sense RNA which is segmented in the case of Arenaviruses. Flaviviruses have a single-stranded positive sense RNA. A Filovirus includes Ebolavirus, Marburgvirus and Cuevavirus. Ebolavirus includes one or more of the different subtypes or species of Ebola virus including Taï Forest virus (TAFV, Ebola-Taï Forest or TEBOV, formely known as Ivory Coast ebolavirus) of *Taï Forest ebolavirus species;* Reston virus (RESTV, Ebola-Reston or REBOV) of *Reston ebolavirus species;* Sudan virus (SUDV, Ebola-Sudan or SEBOV) of *Sudan ebolavirus species;* Ebola virus (EBOV, Ebola-Zaire or ZEBOV) of *Zaire ebolavirus species* and Bundibugyo virus (BDBV, Ebola-Bundibugyo or BEBOV) of *Bundibugyo ebolavirus species.* Marburgvirus includes Marburg virus (MARV) and Ravn virus (RAVV) of Marburg marburgvirus species. Cuevavirus includes Lloviu virus (LLOV) of Lloviu cuevavirus species. An Arenavirus includes Lassa fever virus (Lassa virus), Lujo virus, Argentina, Bolivian, Brazilian and Venezuelan Hemorrhagic Fever viruses. A Bunyavirus includes Crimean-Congo Hemorrhagic Fever virus (CCHF), Hantavirus, Garissa virus, Ilesha virus and Rift Valley Fever virus. A Flavivirus includes Dengue virus, Yellow Fever virus, Kyasanur Forest Disease virus and Omsk Hemorrhagic Fever virus. The method of the invention includes the detection of any variant of the above listed viruses.

In some preferred embodiments, the HFV is selected from the group consisting of: Ebola virus, Lassa virus and Marburg virus. The Ebola virus is advantageously chosen from: Ebola-Zaire, Ebola-Taï Forest, Ebola-Sudan, Ebola-Bundibugyo and Ebola-Reston, preferably from Ebola-Zaire, Ebola-Taï Forest, Ebola-Sudan and Ebola-Bundibugyo.

The method of the invention is performed on any sample suspected of containing an HFV. The sample includes any specimen that may contain an HFV or components thereof such as nucleic acids or fragments of nucleic acids. Samples include biological samples which refer to any material derived from living or dead individual (human or animal) that may contain an HFV or target nucleic acid derived therefrom, including any tissue or body fluid. Non-limiting examples of body fluids include whole-blood, serum, plasma, urine, cerebral spinal fluid (CSF), and mucosal secretions, such as with no limitations sputa and saliva. Samples include swabs. Samples include also processed samples that have been treated to disrupt tissue or cell structure, thus releasing intracellular components into a solution which may further contain reagents (buffers, salts, detergents, enzymes and the like) which are used to prepare, using standard methods, a biological sample for analysis. In particular, processed samples include samples that have been treated by standard methods used for the isolation of nucleic acids from biological samples. In some preferred embodiments of the method of the invention, the sample is a clinical sample (*i.e.,* a sample from living or dead individual (human or animal) suspected of having a VHF). The terms "a", "an", and "the" include plural referents, unless the context clearly indicates otherwise. For example "a target" as used herein is understood to represent one or more targets. As such, the term "a" (or "an"), "one or more" or "at least one" can be used interchangeably herein.

The target nucleic acid refers to a nucleic acid comprising a target sequence to be amplified. Target nucleic acids may be DNA or RNA and may be either single-stranded or double-stranded. The target nucleic acid may include other sequences besides the target sequence, which may not be amplified.

In some preferred embodiments of the method of the invention, the target nucleic acid is viral RNA (*i.e.* HFV genomic RNA).

The target sequence refers to the particular nucleotide sequence of the target nucleic acid that is to be amplified and detected. The target sequence includes the sequences to which oligonucleotide primers hybridize during the nucleic acid amplification process. Where the target nucleic acid is originally single-stranded, the term target sequence will also refer to the sequence complementary to the target sequence as present in the target nucleic acid. Where the target nucleic acid is originally double-stranded the term target-sequence refers to both the sense (+) and antisense (-) strands.

The amplification product includes monomers and concatemers of the target sequence. According to the method of the invention, the target sequence to be amplified and detected is located within a HFV gene coding for a protein of the viral ribonucleocapsid (RNP). Filovirus detection comprises the amplification of a target sequence located within the L, NP, VP30 or VP35 gene; Arenavirus detection comprises the amplification of a target sequence located within the L or NP gene.

In some preferred embodiments of the method of the invention, the target sequence is located within the L or NP genes. Whereas Marburg virus detection comprises the amplification of a target sequence located within the L or NP gene, Ebola virus detection preferably comprises the amplification a target sequence located in the L gene and Lassa virus detection preferably comprises the amplification of a target sequence located in the NP gene.

For Ebola virus detection, the target sequence to be amplified and detected is advantageously located within a region of the L gene of Ebola virus which is selected from the group consisting of:
a) a sequence from positions 1671 to 1973 of the coding sequence (CDS) of *Zaire ebolavirus* (ZEBOV or EBOV) L gene of nucleic acid sequence SEQ ID NO: 1 (positions 11581 to 18219 of nucleic acid sequence GenBank NC_002549.1); preferably a sequence selected from positions 1671 to 1876, 1703 to 1973 and 1671 to 1973 of said sequence corresponding to SEQ ID NO: 2, 3, 4, respectively; more preferably the sequence from positions 1671 to 1876 of said sequence (SEQ ID NO:2);
b) a sequence from positions 3531 to 3745, 4654 to 4940 or 5270 to 5598 of the CDS of *Taï Forest ebolavirus* (TEBOV or TAFV) L gene of nucleic acid sequence SEQ ID NO: 5 (positions 11566 to 18198 of nucleic acid sequence GenBank NC_014372.1), corresponding to SEQ ID NO: 6, 7 and 8, respectively;
c) a sequence from positions 528-847, 1728-2025 or 2204-2460 of the CDS of *Sudan ebolavirus* (SEBOV or SUDV) L gene of nucleic acid sequence SEQ ID NO: 9 (positions 11535 to 18167 of nucleic acid sequence GenBank NC_006432.1), corresponding to SEQ ID NO: 10, 11 and 12;
d) a sequence from positions 579-823, 2433 to 2661 or 3570 to 3828 of the CDS of *Bundibugyo ebolavirus* (BEBOV or BDBV) L gene of nucleic acid sequence SEQ ID NO: 13 (positions 11567 to 18199 of nucleic acid sequence GenBank NC_014373.1), corresponding to SEQ ID NO: 14, 15 and 16; and
e) a sequence from positions 1775 to 2060, 4422 to 4742 or 5922 to 6140 of the CDS of *Reston ebolavirus* (REBOV or RESTV) L gene of nucleic acid sequence SEQ ID NO: 17 (positions 11550 to 18188 of nucleic acid sequence GenBank NC_004161.1), corresponding to SEQ ID NO: 18, 19, and 20, respectively, and
   - a sequence comprising any one of SEQ ID NO: 2-4, 6-8, 10-12, 14-16, 18-20 and up to 20 consecutive nucleotides (2, 3, 4, 5, 6, 7, 8, 9, 10, 11, 12, 13, 14 ,15, 16, 17, 18, 19 and 20), preferably up to 15 (2, 3, 4, 5, 6, 7, 8, 9, 10, 11, 12, 13, 14 ,15), up to 10 (2, 3, 4, 5, 6, 7, 8, 9, 10) or up to 5 (2, 3, 4, 5) consecutive nucleotides of 5' and/or 3' flanking sequence from said L gene.

The Ebola virus target sequences listed in a) to e) above are subtype/species specific. Therefore, when the Ebola virus species suspected of being present in the sample is known, the method of the invention comprises the amplification of a target sequence of said Ebola virus species (*i.e.* the target sequence a), b), c), d) or e), respectively for EBOV, TAFV, SUDV, BDBV and RESTV. When the Ebolavirus species suspected of being present in the sample is totally or partially unknown, the method of the invention comprises the amplification of several target sequences specific for several or all different Ebolavirus species as listed above.

According to some more advantageous embodiments, the method of the invention, where being for detecting Ebolavirus, comprises the amplification of two or more of the target sequences a) to e) as listed above, preferably the target sequences a) to d) or a) to e), as listed above.

For Marburg virus (MARV) detection, the target sequence to be amplified and detected is advantageously located within a region of the L or NP gene of MARV, which is selected from the group consisting of:
f) a sequence from positions 786 to 1049, 5746 to 5980, 6442 to 6684 of the coding sequence of Marburg virus L gene of nucleic acid sequence SEQ ID NO: 21 (positions 11481 to 18476 of nucleic acid sequence GenBank NC_001608.3), corresponding to SEQ ID NO: 22, 23 and 24 respectively;
g) a sequence from positions 862 to 1128 or 1749 to 2038 of the coding sequence of Marburg virus NP gene of nucleic acid sequence SEQ ID NO: 25 (positions 104 to 2191 of nucleic acid sequence GenBank NC_001608.3), corresponding to SEQ ID NO: 26 and 27, respectively, and
   - a sequence comprising any one of SEQ ID NO 22-24 or 26-27 and up to 20 consecutive nucleotides (2, 3, 4, 5, 6, 7, 8, 9, 10, 11, 12, 13, 14, 15, 16, 17, 18, 19 and 20), preferably up to 15 (2, 3, 4, 5, 6, 7, 8, 9, 10, 11, 12, 13, 14 ,15), up to 10 (2, 3, 4, 5, 6, 7, 8, 9, 10) or up to 5 (2, 3, 4, 5) consecutive nucleotides of 5' and/or 3' flanking sequence from said L or NP gene.

For Lassa virus (LAV) detection, the target sequence to be amplified and detected is advantageously a sequence located within the NP gene of LAV, which is selected from the group consisting of:
h) a sequence from positions 55 to 293 or 1036 to 1223 of the coding sequence Lassa virus NP gene of nucleic acid sequence SEQ ID NO: 28 (positions 101 to 1810 of GenBank NC_004296.1), corresponding to SEQ ID NO: 29 and 30, respectively, and
   - a sequence comprising any one of SEQ ID NO 29-30 and up to 20 consecutive nucleotides (2, 3, 4, 5, 6, 7, 8 ,9, 10, 11, 12, 13, 14 ,15, 16, 17, 18, 19 and 20), preferably up to 15 (2, 3, 4, 5, 6, 7, 8 ,9, 10, 11, 12, 13, 14 ,15), up to 10 (2, 3, 4, 5, 6, 7, 8 ,9, 10) or up to 5 (2, 3, 4, 5) nucleotides of 5' and/or 3' flanking sequence from said NP gene.

According to the invention, the target region of the target gene consists of any one of the sequences SEQ ID NO: 2-4, 6-8, 10-12, 14-16, 18-20, 22-24, 26-27 and 29-30 and up to 20 consecutive nucleotides of 5' and/or 3' flanking sequence from said target gene. The target sequence of the target gene that is amplified with the oligonucleotide primers consists of or is included, comprised in or contained in the target region. In some preferred embodiment the target sequence consists of or is included in one of the sequences SEQ ID NO: 2-4, 6-8, 10-12, 14-16, 18-20, 22-24, 26-27 and 29-30. The target sequence consists of at least 50 consecutive nucleotides, preferably at least 100 or 150 consecutive nucleotides from the sequence of the target region. Preferably, the target sequence consists of 150 to 350 consecutive nucleotides from the sequence of the target region, more preferably 150 to 250 consecutive nucleotides.

According to another preferred embodiment, the method of the invention comprises assaying different target sequences simultaneously on the same sample, preferably target sequences specific for different VHFs including different types of VHFs and different subtypes or species of the same type of VHF. In some more preferred embodiments, the method of the invention comprises assaying target sequences specific for different types of HFV, for example Ebola, Lassa and Marburg viruses, in order to perform a differential diagnosis of VHFs. In other more preferred embodiments, the method of the invention comprises assaying target sequences specific for different subtypes or species of a type of HFV, for example target sequences specific for different species of Ebola virus as defined above, to perform the HFV species or subtype determination. For detecting the different VHFs, the amplification of the different target sequences are performed in parallel, in separate reaction mixtures or in the same reaction mixture and detected independently.

According to the method of the invention, the target sequence is amplified by isothermal nucleic acid amplification reaction using any of the various natural or engineered enzymes available for this purpose.

These include in particular DNA polymerases having strong strand-displacement activity in isothermal conditions, thereby obviating the need for thermal cycling. Such polymerases are well-known in the art and include DNA polymerase long fragment (LF) of thermophilic bacteria such as *Bacillus stearothermophilus* (Bst), *Bacillus Smithii* (Bsm), *Geobacillus sp.* M (GspM) and *Thermodesulfatator indicus* (Tin), engineered variants therefrom as well as Taq DNA polymerase variants. Non-limiting examples of polymerase which can be used to perform the method of the invention include Bst LF DNA polymerase, GspM LF DNA polymerase, GspSSD LF DNA polymerase, Tin exo-LF DNA polymerase and SD DNA polymerase which are usually used at 50-75°C, more generally at 55 to 65°C.

According to another preferred embodiment of the method of the invention, the isothermal amplification is chosen from strand-displacement amplification (SDA), Rolling-circle amplification (RCA), Cross-priming amplification (CPA), nucleic acid sequence-based amplification (NASBA), Recombinase Polymerase Amplification (RPA) and Loop-mediated amplification (LAMP). The principle of LAMP method is disclosed in Notomi et al., Nucleic Acids Res., 2000 Jun 15; 28(12):e63.

When the nucleic acid is RNA, step a) includes a reverse transcription step prior to the isothermal amplification of the target sequence. The reverse transcription reaction is performed using any reverse transcriptase (RT). RT are well-known in the art and include for example Avian Myeloblastosis Virus (AMV) and Moloney Murine Leukemia virus (MMLV) RT. The Reverse transcription and DNA amplification can be performed in the same reaction mixture comprising the DNA polymerase and RT enzymes. In addition, the method of the invention can also use a DNA polymerase having both strong displacement activity and RT activity such as Pyrophage 3173 DNA polymerase.

In some more advantageous embodiments of the method of the invention, the isothermal amplification is LAMP or RT-LAMP.

According to the invention, step a) is performed using at least one pair of oligonucleotide primers for amplifying a target sequence of an HFV nucleic acid, which is located within a gene of the HFV nucleic acid coding for a protein of the viral ribonucleocapsid.

Oligonucleotide refers to a polymer of 5 to 100 nucleotides, preferably from a lower limit of 15 to 20 nucleotides to an upper limit of 45 to 60 nucleotides, wherein the polymer comprise ribonucleotides, deoxyribonucleotides, modified nucleotides or mixtures thereof and may further include modified internucleotide linkages and/or modified 5' and/or 3' termini. Oligonucleotides are usually synthesized using any of a variety of well-known enzymatic or chemical methods. The oligonucleotide primer according to the invention is substantially complementary to the target sequence. Substantially complementary means that the oligonucleotide is at least 80% identical, preferably at least 85%, 90%, 95% and 98% identical to the target sequence. The oligonucleotide may comprise additional sequences (not complementary to the target sequence) at its 5' end. In some embodiments the oligonucleotide comprises a sequence of at least 5, preferably 10 to 15 consecutive nucleotides which is 100% identical to the target sequence. In some more preferred embodiments, the oligonucleotide sequence is 100% identical to the target sequence. Optionally, at least one oligonucleotide of the pair includes a label (detectable moiety).

The oligonuclotides which function as primers are capable of annealing specifically to the target sequence and can be further extended in the presence of a nucleic acid polymerase to specifically amplify the target sequence. At least one oligonucleotide may also function as a probe and further includes a detectable label to detect a target nucleic acid or an amplicon thereof. The detectable label is a moiety that can be detected directly or indirectly by the production of a detectable signal such as colorimetric, fluorescent, chemiluminescent or electrochemoluminescent signal. Directly detectable labels include radioisotopes and fluorophores. Indirectly detectable labels are detected by labeling with additional reagents that enable the detection. Indirectly detectable labels include, for example, chemiluminescent agents, enzymes that produce visible or colored reaction products, and a ligand-detectable ligand binding partner, where a ligand (hapten, antibody, antigen, biotin) may be detected by binding to a labelled ligand-specific binding partner.

The oligonucleotides are designed based on HFV genomic sequences available in sequence databases using general principles for designing amplification primers. The design of the primers may also include specific principles for designing primers for use in specific isothermal amplification methods. Primer design may be performed using any of the various softwares.

According to another preferred embodiment of the method of the invention, the oligonucleotide primers are selected from the group consisting of: the sequences of 5 to 100 nucleotides, preferably 15 to 60 nucleotides having 80% to 100% identity, preferably at least 85%, 90% 95% or 98% identity with any of SEQ ID NO: 2-4, 6-8, 10-12, 14-16, 18-20, 22-24, 26-27 and 29-30 or with a sequence comprising any of SEQ ID NO:2-4, 6-8, 10-12, 14-16, 18-20, 22-24, 26-27 and 29-30 and up to 20 consecutive nucleotides of 5' and/or 3' flanking sequence from said L or NP gene as defined above.

In a more preferred embodiment, the primers are selected from the group consisting of the sequences SEQ ID NO: 31 to 162.

The primer pairs are advantageously selected from the group consisting of: SEQ ID NO: 31-32, 33-34, 35-36, 37-38, 39-40, 41-42, 43-44, 45-46 47-48, 49-50, 51-52, 53-54, 55-56, 57-58, 59-60, 61-62, 63-64, 65-66, 67-68, 69-70, 71-72, 73-74, 75-76, 77-78, 79-80, 81-82, 83-84, 85-86, 87-88, 89-90, 91-92, 93-94, 95-96, 97-98, 99-100, 101-102, 103-104, 105-106, 107-108, 109-110, 111-112, 113-114, 115-116, 117-118, 119-120, 121-122, 123-124, 125-126, 127-128, 129-130, 131-132, 133-134, 135-136, 137-138, 139-140, 141-142, 143-144, 145-146, 147-148, 149-150, 151-152, 153-154, 155-156, 157-158, 159-160, and 161-162 (Table VII).

The primers SEQ ID NO: 31 to 120 are specific for Ebolavirus; SEQ ID NO: 31 to 48 are specific for *Zaire ebolavirus* (Ebola virus or EBOV), SEQ ID NO: 49 to 66 are specific for *Taï Forest ebolavirus* (Tai Forest virus or TAFV); SEQ ID NO: 67 to 84 are specific for *Sudan ebolavirus* (Sudan virus or SUDV); SEQ ID NO: 85 to 102 are specific for *Bundibugyo ebolavirus* (Bundibugyo virus or BDBV); SEQ ID NO: 103 to 120 are specific for *Reston ebolavirus* (Reston virus or RESTV) (Table VII).

The primers SEQ ID NO: 121 to 150 are specific for Marburg virus.

The primers SEQ ID NO: 151 to 162 are specific for Lassa virus.

According to another preferred embodiment of the method of the invention, the amplification reaction is a RT-LAMP amplification which is performed with a combination of two or three primer pairs selected from the group consisting of:
- SEQ ID NO: 31-32 and SEQ ID NO: 33-34, and optionally, SEQ ID NO: 35-36, which are specific for *Zaire ebolavirus;*
- SEQ ID NO: 37-38, and SEQ ID NO: 39-40, and optionally, SEQ ID NO: 41-42, which are specific for *Zaire ebolavirus;*
- SEQ ID NO: 43-44 and SEQ ID NO: 45-46, and optionally, SEQ ID NO: 47-48, which are specific for *Zaire Ebolavirus;*
- SEQ ID NO:49-50 and SEQ ID NO:51-52, and optionally, SEQ ID NO:53-54, which are specific for *Taï Forest ebolavirus;*
- SEQ ID NO:55-56 and SEQ ID NO:57-58, and optionally, SEQ ID NO:59-60, which are specific for *Taï Forest ebolavirus;*
- SEQ ID NO:61-62 and SEQ ID NO:63-64, and optionally, SEQ ID NO:65-66, which are specific for *Taï Forest ebolavirus;*
- SEQ ID NO:67-68 and SEQ ID NO:69-70, and optionally, SEQ ID NO:71-72, which are specific for *Sudan ebolavirus;*
- SEQ ID NO:73-74 and SEQ ID NO:75-76, and optionally, SEQ ID NO:77-78, which are specific for *Sudan Ebolavirus;*
- SEQ ID NO:79-80 and SEQ ID NO:81-82, and optionally, SEQ ID NO:83-84, which are specific for *Sudan ebolavirus;*
- SEQ ID NO:85-86 and SEQ ID NO:87-88, and optionally, SEQ ID NO:89-90, which are specific for *Bundibugyo ebolavirus;*
- SEQ ID NO:91-92 and SEQ ID NO:93-94, and optionally, SEQ ID NO:95-96, which are specific for *Bundibugyo ebolavirus;*
- SEQ ID NO:97-98 and SEQ ID NO:99-100, and optionally, SEQ ID NO:101-102, which are specific for *Bundibugyo ebolavirus;*
- SEQ ID NO:103-104 and SEQ ID NO:105-106, and optionally, SEQ ID NO:107-108, which are specific for *Reston ebolavirus ;*
- SEQ ID NO:109-110 and SEQ ID NO:111-112, and optionally, SEQ ID NO:113-114, which are specific for *Reston ebolavirus;*
- SEQ ID NO:115-116 and SEQ ID NO:117-118, and optionally, SEQ ID NO:119-120, which are specific for *Reston Ebolavirus;*
- SEQ ID NO:121-122 and SEQ ID NO:123-124, and optionally, SEQ ID NO:125-126, which are specific for Marburg virus;
- SEQ ID NO:127-128 and SEQ ID NO:129-130, and optionally, SEQ ID NO:131-132, which are specific for Marburg virus;
- SEQ ID NO:133-134 and SEQ ID NO:135-136, and optionally, SEQ ID NO:137-138, which are specific for Marburg virus;
- SEQ ID NO:139-14 and SEQ ID NO:141-142, and optionally, SEQ ID NO:143-144, which are specific for Marburg virus; SEQ ID NO:145-146 and SEQ ID NO:147-148, and optionally, SEQ ID NO:149-150, which are specific for Marburg virus;
- SEQ ID NO:151-152 and SEQ ID NO:153-154, and optionally, SEQ ID NO:155-156, which are specific for Lassa virus; and
- SEQ ID NO: 157-158 and SEQ ID NO:159-160 and optionally, SEQ ID NO:161-162, which are specific for Lassa virus.

In some more preferred embodiment, the RT-LAMP amplification is performed with the three primer pairs as defined above.

According to another more preferred embodiment, the method of the invention is a method for determining the Ebola virus species in a sample suspected of containing an Ebola virus, wherein the amplification reaction is performed with at least two sets of primers chosen from:
(i) a first set of primers specific for *Zaire ebolavirus* comprising at least one pair of primers selected from the group consisting of SEQ ID NO: 31-32, 33-34, 35-36, 37-38, 39-40, 41-42, 43-44, 45-46, 47-48; preferably two or three pairs of primers selected from the group consisting of: SEQ ID NO: 31-32, 33-34, and eventually SEQ ID NO: 35-36; SEQ ID NO: 37-38, 39-40, and eventually SEQ ID NO: 41-42; SEQ ID NO: 43-44, 45-46, and eventually, SEQ ID NO: 47-48; more preferably, SEQ ID NO: 43-44, 45-46, and eventually, SEQ ID NO: 47-48;
(ii) a second set of primers specific for *Taï Forest ebolavirus* comprising at least one pair of primers selected from the group consisting of SEQ ID NO:49-50, 51-52, 53-54, 55-56, 57-58, 59-60, 61-62, 63-64, 65-66; preferably two or three pairs of primers selected from the group consisting of: SEQ ID NO: 49-50, 51-52, and eventually SEQ ID NO: 53-54; SEQ ID NO: 55-56, 57-58, and eventually SEQ ID NO: 59-60; SEQ ID NO: 61-62, 63-64, and eventually, SEQ ID NO: 65-66;
(iii) a third set of primers specific for *Sudan ebolavirus* comprising at least one pair of primers selected from the group consisting of SEQ IDNO: 67-68, 69-70, 71-72, 73-74, 75-76, 77-78, 79-80, 81-82, 83-84; preferably two or three pairs of primers selected from the group consisting of: SEQ ID NO: 67-68, 69-70, and eventually SEQ ID NO: 71-72; SEQ ID NO: 73-74, 75-76, and eventually SEQ ID NO: 77-78; SEQ ID NO: 79-80, 81-82, and eventually, SEQ ID NO: 83-84;
(iv) a fourth set of primers specific for *Bundibugyo ebolavirus* comprising at least one pair of primers selected from the group consisting of SEQ IDNO:85-86, 87-88, 89-90, 91-92, 93-94, 95-96, 97-98, 99-100, 101-102 ; preferably two or three pairs of primers selected from the group consisting of: SEQ ID NO: 85-86, 87-88, and eventually SEQ ID NO: 89-90; SEQ ID NO: 91-92, 93-94, and eventually SEQ ID NO: 95-96; SEQ ID NO: 97-98, 99-100, and eventually, SEQ ID NO: 101-102; and
(v) a fifth set of primers specific for *Reston ebolavirus* comprising at least one pair of primers selected from the group consisting of SEQ ID NO: 103-104, 105-106, 107-108, 109-110, 111-112, 113-114, 115-116, 117-118, 119-120; preferably two or three pairs of primers selected from the group consisting of: SEQ ID NO: 103-104, 105-106, and eventually SEQ ID NO: 107-108; SEQ ID NO: 109-110, 111-112, and eventually SEQ ID NO: 113-114; SEQ ID NO: 115-116, 117-118, and eventually, SEQ ID NO: 119-120.

The amplification reaction is advantageously performed with the four set of primers (i) to (iv) or the five set of primers (i) to (v) as defined above.

According to another more preferred embodiment, the method is a method for performing the differential detection of HFVs in a sample, wherein the amplification reaction is performed with at least two different sets of primers chosen from:
- a first set of primers specific for Ebola virus comprising one or more of the sets (i) to (v) above;
- a second set of primers specific for Margburg virus comprising at least one pair of primers selected from the group consisting of SEQ ID NO: 121-122, 123-124, 125-126, 127-128, 129-130, 131-132, 133-134, 135-136,137-138, 139-140, 141-142, 143-144, 145-146,147-148, 149-150; preferably two or three pairs of primers selected from the group consisting of: SEQ ID NO: 121-122, 123-124, and eventually SEQ ID NO: 125-126; SEQ ID NO: 127-128, 129-130, and eventually SEQ ID NO: 131-132; SEQ ID NO: 133-134, 135-136, and eventually, SEQ ID NO: 137-138; SEQ ID NO: 139-140, 141-142, and eventually SEQ ID NO: 143-144; SEQ ID NO: 145-146,147-148, and eventually SEQ ID NO: 149-150; and
- a third set of primers specific for Lassa virus comprising at least one pair of primers selected from the group consisting of 151-152, 153-154, 155-156, 157-158, 159-160, 161-162; preferably two or three pairs of primers selected from the group consisting of: SEQ ID NO: 151-152, 153-154, and eventually SEQ ID NO: 155-156; SEQ ID NO: 157-158, 159-160, and eventually SEQ ID NO: 161-162.

The amplification reaction is advantageously performed with the three sets of primers as defined above to perform the differential detection of Ebola virus, Lassa virus and Marburg virus.

The method is useful for performing the differential diagnosis of VHFs, in particular of Ebola virus Disease, Lassa fever and Marburg Hemorrhagic fever.

According to the invention, the amplification product is detected using any of the various methods available for this purpose which are well-known in the art. For example, the detection method is fluorescence, bioluminescence, colorimetry, turbidimetry, immunoenzymatic or electrochemical detection. The detection may be semi-quantitative or quantitative. The detection may also be real-time detection, wherein the signal resulting from the presence of the amplification product is measured during the course of the nucleic acid amplification reaction to monitor the accumulation of specific amplification products. Fluorescence may use DNA intercaling dyes, fluorescent molecular beacon probes or a fluorescence metal indicator such as calcein. Colorimetry may use a colored indicator for alkaline metal ions, such as hydroxy naphthol blue (Goto et al., BioTechniques. 2009 Mar;46(3):167-72) or pH indicators (Tanner et al., BioTechniques, 2015 Feb;58(2):59-68). Turbidity is used when the amplification reaction, such as LAMP, produces large amounts of magnesium pyrophosphate (a white precipitate) and dsDNA, which allow visual inspection of results using a turbidimeter (Mori et al., Biochem. Biophys. Res. Commun., 2001 Nov 23; 289(1):150-4). Electrochemical detection may use a pH meter for direct measurement of released hydrogen ions during the amplification reaction, such as LAMP (Xie S et al., Chem. Commun., 2014 Oct 24; 50(100):15932-5), or integrated electrodes for measuring decreases in current resulting from increasing binding of electrochemically-active DNA-binding redox reporters, such as Methylene Blue, to amplification products (Xie et al., Biosens. Bioelectron. 2014 May 15; 55:324-9). Immunoassays include Enzyme-linked immunosorbent assays (ELISA) and lateral flow immunoassays based on the use of specific probes (Tsai S-M et al., J. Virol. Methods. 2012 Apr; 181(1):117-24; Ravan H and Yazdanparast R., Anal. Biochem., 2013 Aug 15; 439(2):102-8). Bioluminescence detection may be through measurement of bioluminescent output of the coupled conversion of inorganic pyrophosphate produced stoichiometrically during nucleic acid synthesis to ATP by the enzyme ATP sulfurylase (Gandelman et al., PLoS ONE., 2010 Nov 30; 5(11)).

According to another preferred embodiment, the method comprises at least one non-HFV internal control (IC) nucleic acid that is processed as the tested samples and amplified in the same assay reaction mixtures by using amplification oligonucleotide primers specific for a target sequence of the IC nucleic acid sequence.

In some embodiments, the IC target sequence is selected from the group consisting of:
- the sequence from positions 26 to 293, 227 to 498, or 387 to 621 of the CDS of human GAPDH gene of nucleic acid sequence SEQ ID NO: 163 (positions 189 to 1196 of GenBank NM_002046.5.2), corresponding to SEQ ID NO: 164, 165 and 166, respectively;
- the sequence from positions 204 to 517, 584 to 869, or 908 to 1148 of the CDS of *Drosophila melanogaster* Sigma virus G gene of nucleic acid sequence SEQ ID NO: 167 (positions 4262 to 5947 of GenBank GQ375258), corresponding to SEQ ID NO: 168, 169 and 170, respectively.

The IC target sequence SEQ ID NO: 165 is preferably amplified using at least one pair of primers selected from the group consisting of: SEQ ID NO: 171-172, 173-174, 175-176. More preferably, when RT-LAMP is used in the amplification reaction, the IC target sequence is amplified with the pairs SEQ ID NO: 171-172 and 173-174, and optionally SEQ ID NO: 175-176.

The IC target sequence SEQ ID NO: 164 is preferably amplified using at least one pair of primers selected from the group consisting of: SEQ ID NO: 177-178,179-180, 181-182. More preferably, when RT-LAMP is used in the amplification reaction, the IC target sequence is amplified with the pairs SEQ ID NO: 177-178 and 179-180, and optionally SEQ ID NO: 181-182.

The IC target sequence SEQ ID NO: 166 is preferably amplified using at least one pair of primers selected from the group consisting of: SEQ ID NO: 183-184, 185-186, 187-188. More preferably, when RT-LAMP is used in the amplification reaction, the IC target sequence is amplified with the pairs SEQ ID NO: 183-184 and 185-186, and optionally SEQ ID NO: 187-188.

The IC target sequence SEQ ID NO: 169 is preferably amplified using at least one pair of primers selected from the group consisting of: SEQ ID NO: 189-190,191-192 and 193-194. More preferably, when RT-LAMP is used in the amplification reaction, the IC target sequence is amplified with the pairs SEQ ID NO: 189-190 and 191-192, and optionally SEQ ID NO: 193-194.

The IC target sequence SEQ ID NO: 170 is preferably amplified using at least one pair of primers selected from the group consisting of: SEQ ID NO: 195-196, 197-198, 199-200. More preferably, when RT-LAMP is used in the amplification reaction, the IC target sequence is amplified with the pairs SEQ ID NO: 195-196 and 197-198, and optionally SEQ ID NO: 199-200.

The IC target sequence SEQ ID NO: 168 is preferably amplified using at least one pair of primers selected from the group consisting of: SEQ ID NO:201-202, 203-204 and 205-206. More preferably, when RT-LAMP is used in the amplification reaction, the IC target sequence is amplified with the pairs SEQ ID NO: 201-202 and 203-204, and optionally SEQ ID NO: 205-206.

According to another preferred embodiment, the method comprises assaying several samples simultaneously. In some advantageous embodiments, the method comprises assaying multiple samples simultaneously in a high-throughput process.

The method of the invention is useful for the diagnosis of VHFs, in particular the diagnosis of Ebola virus Disease, Lassa fever and Marburg Hemorrhagic fever. The method is also useful for the differential diagnosis of VHFs, in particular the differential diagnosis of Ebola virus Disease, Lassa fever and Marburg Hemorrhagic fever. The method is also useful for HFV species determination, in particular for determining the Ebola virus species present in a sample suspected of containing an Ebola virus.

Another aspect of the present invention is a kit for the detection of a HFV, comprising at least one pair of oligonucleotide primers for amplifying a target sequence of a HFV nucleic acid as defined above. At least one oligonucleotide of the pair may comprise a detectable label. The kit optionally comprises reagents for the isothermal amplification of the target sequence and/or the detection of the amplification product. Reagents available for this purpose are well-known in the art and include the DNA polymerases and RT enzymes described above, buffers for the enzymes, detergents, enhancing agents, nucleic acid binding dyes and probes, preferably labelled probes. In some preferred embodiments of the kit of the invention, the primers, and optional reagents are in lyophilised form to allow ambient storage. The components of the kits are packaged together into any of the various containers suitable for nucleic acid amplification such as plates, slides, wells, dishes, beads, particles, cups, strands, chips, strips and others. The kit optionally includes instructions for performing at least one specific embodiment of the method of the invention. In some advantageous embodiments, the kit comprises micro-well plates or microtubes, preferably in a dried format, *i.e.,* wherein the wells of the plates or microtubes comprise a dried composition containing at least the primers, and preferably further comprising all the reagents for the isothermal amplification of the target sequence and/or the detection of the amplification product amplification. In some other advantageous embodiments, the primers and optional reagents are included into any of the devices available for nucleic acid amplification including devices further integrating nucleic acid extraction and/or amplification product detection capacities such as microfluidic devices or other devices.

Another aspect of the invention is a pair of oligonucleotide primers for amplifying a target sequence of an HFV nucleic acid as defined above. The invention encompasses compositions comprising two or more primer pairs, in particular, compositions comprising primer pairs specific for different types of HFV for the differential diagnosis of VHFs and compositions comprising primer pairs specific for different species of a HFV type for the species determination of a VHF, as defined above. The invention encompasses also an oligonucleotide of the pair of oligonucleotides according to the invention.

Another aspect of the invention is the use of an oligonucleotide primer, primer pair or composition as defined above for the *in vitro* detection of HFVs. The oligonucleotides according to the invention are used as primers and /or probes for the detection of HFV target nucleic acid using standard method which are well-known in the art. In particular the primers may be used in any nucleic amplification technique including the isothermal amplification methods described above and other amplification methods available for this purpose such as Polymerase Chain Reaction (PCR) and RT-PCR.

The practice of the present invention will employ, unless otherwise indicated, conventional techniques, which are within the skill of the art. Such techniques are explained fully in the literature.

For a better understanding of the invention and to show how the same may be carried into effect, there will now be described by way of example a specific mode contemplated by the Inventors with reference to the accompanying drawings in which:
- **Figure 1****:** Evaluation of the analytical sensitivity of the Zaire Ebolavirus RT-LAMP assay. Amplification curves were obtained using 10-fold dilutions of synthetic *Zaire ebolavirus* RNA as template in a real-time *Zaire ebolavirus* qRT-LAMP assay.
- **Figure 2****:** Time-to-result obtained by real-time RT-PCR and real-time RT-LAMP for a panel of 87 plasma RNA extracts from patients with confirmed EVD diagnosis.

In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example: RT-LAMP assay for rapid and accurate detection of HFVs in clinical samples

### 1. Material and methods

Specific target gene regions were selected by multiple sequence alignments of all genome sequences available in the Genbank database for the 5 known Ebola virus species, namely Ebola virus (*Zaire ebolavirus*); Sudan virus (*Sudan ebolavirus*); Taï Forest virus (*Taï Forest ebolavirus,* formerly *Côte d'Ivoire ebolavirus*); Reston virus (*Reston ebolavirus*) and Bundibugyo virus (*Bundibugyo ebolavirus*)*.* Target gene regions were also selected for two additional viral hemorrhagic fever agents, Marburg virus (*Marburg marburgvirus*) and Lassa virus (*Lassa mammarenavirus*), in order to provide additional diagnostic tools for the differential diagnosis of VHFs. In addition, a target gene region was selected for the *Drosophila melanogaster sigma virus,* used as internal control for sample inhibition, extraction efficiency and LAMP procedure. Finally, a human housekeeping gene was selected as control for sample integrity. Primers specifically targeting selected gene regions were designed using the LAMP explorer software (PREMIER BIOSOFT). Primer specificity was verified by BLAST analysis. A total of 15 different primer set, each composed of 6 specific primers, were designed: 3 primer sets targeting Ebola virus; 3 primer sets targeting Sudan virus; 3 primer sets targeting Taï Forest virus; 3 primer sets targeting Reston virus and 3 primer sets targeting Bundibugyo virus **(Table VII).**

A real-time RT-LAMP protocol was developed for real-time detection of target viral species. Reaction conditions (dNTP concentration, MgSO4 concentration, Betain concentration, fluorescent dye concentration, enzyme and buffer composition) were compared individually for optimal amplification and reaction time.

A synthetic *Zaire ebolavirus* RNA transcript was prepared, aliquoted and tested in triplicate using various RT-LAMP master mixes to determine conditions for optimal reaction efficiency:
- Master mix 1: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of Bst 2.0 DNA Polymerase, 5 units of AMV Reverse Transcriptase.
- Master mix 2: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of Bst 2.0 DNA Polymerase, 5 units of WarmStart® RTx Reverse Transcriptase.

- Master mix 3: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of Bst 2.0 WarmStart™ DNA Polymerase, 5 units of AMV Reverse Transcriptase.
- Master mix 4 : 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of Bst 2.0 WarmStart™ DNA Polymerase, 5 units of WarmStart® RTx Reverse Transcriptase.
- Master mix 5 : 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA polymerase, 5 units of AMV Reverse Transcriptase.
- Master mix 6: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA polymerase, 5 units of WarmStart® RTx Reverse Transcriptase.
- Master mix 7: 1X ISO001 nd, 40 pmol of FIP and BIP primers, 5 pmol of F3 and B3 primers, 20 pmol of LF and LB primers, EvaGreen® dye 0.5X, 5 units of AMV reverse transcriptase.
- Master mix 8: 1X Isothermal Mastermix ISO001 (OPTIGENE), 40 pmol of FIP and BIP primers, 5 pmol of F3 and B3 primers, 20 pmol of LF and LB primers, EvaGreen® dye 0.5X, 5 units of WarmStart® RTx Reverse Transcriptase.
- Master mix 9: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA Polymerase, 2.5 units of AMV Reverse Transcriptase.
- Master mix 10: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA Polymerase, 1.25 units of AMV Reverse Transcriptase.
- Master mix 11: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, Syto-9 1.5 µM, 8 units of GspSSD LF DNA Polymerase, 1.25 units of AMV Reverse Transcriptase.
- Master mix 12: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, SYTO® 9 dye 10 µM, 8 units of GspSSD LF DNA Polymerase, 1.25 units of AMV Reverse Transcriptase.
- Master mix 13: 1.4 mM dNTP, 20 mM Tris-HCl (pH 8.8), 10 mM KCl, 10 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA Polymerase, 5 units of AMV Reverse Transcriptase.
- Master mix 14: 1.4 mM dNTP, 20 mM Tris-HCl (pH 8.8), 10 mM KCl, 12 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA Polymerase, 5 units of AMV Reverse Transcriptase.
- Master mix 15: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 0.6 M, 40 pmol of FIP and BIP primers, 5 pmol of F3 and B3 primers, 20 pmol of LF and LB primers, EvaGreen® dye 0.5X, 8 units of GspSSD LF DNA Polymerase, 5 units of AMV Reverse Transcriptase.
- Master mix 16: 1.4 mM dNTP, 20 mM Tris-HCl (pH 8.8), 10 mM KCl, 8 mM MgSO₄, 10 mM (NH₄)₂SO₄, 0.1% Triton™ X-100, Betaine 1.0 M, 40 pmol of FIP and BIP primers, 5 pmol of F3 and B3 primers, 20 pmol of LF and LB primers, EvaGreen® dye 0.5X, 8 units of GspSSD LF DNA Polymerase, 5 units of AMV Reverse Transcriptase.
- Master mix 17: 1.2 mM dNTP, 20 mM Tris-HCl (pH 8.8), 10 mM KCl, 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA Polymerase, 5 units of AMV Reverse Transcriptase.
- Master mix 18: 1.6 mM dNTP, 20 mM Tris-HCl (pH 8.8), 10 mM KCl₃ 8 mM MgSO₄, 10 mM (NH₄)₂SO₄, 0.1% Triton™ X-100, Betaine 0.8 M, 40 pmol of FIP and BIP primers, 5 pmol of F3 and B3 primers, 20 pmol of LF and LB primers, EvaGreen® dye 0.5X, 8 units of GspSSD LF DNA Polymerase, 5 units of AMV Reverse Transcriptase.

All RT-LAMP reactions were carried out in a 25-µL reaction volume containing 20 µL reaction Mastermix and 5 µL of RNA extract. Amplification was performed at a constant temperature of 65°C for 30 minutes. The resulting amplification efficiency for the different RT-LAMP reaction mastermixes were compared using the purified synthetic EBOV RNA.

Following the selection of the most efficient mastermix composition, reaction temperatures (63°C, 65°C and 68°C) were compared individually for optimal amplification and reaction time using the synthetic EBOV RNA transcript tested in triplicate.

Finally, the designed primers sets targeting Zaire Ebola virus were compared individually for optimal amplification and reaction time using a patient plasma RNA extract tested in triplicate.

All reactions were conducted in a LightCycler® 480 (Roche) or a LightCycler® Nano (Roche) or a Genie® III (OptiGene) instrument.

Serial dilutions (10-fold) of *Zaire ebolavirus* synthetic RNA transcripts and genomic RNA from *Zaire ebolavirus* strain H.sapiens-tc/GIN/2014/Gueckedou-C05 were prepared and used to determine the analytical sensitivity of the *Zaire ebolavirus* RT-LAMP assay, as compared to real-time RT-PCR (RealStar® Ebolavirus RT-PCR Kit 1.0 (Altona Diagnostics)).

The analytical specificity of the *Zaire ebolavirus* RT-LAMP assay was verified using a panel of genomic RNA and DNA extracts from closely-related viruses and from pathogens causing symptoms similar to those of EVD.

A monocentric retrospective clinical evaluation of the *Zaire ebolavirus* RT-LAMP assay was conducted within the laboratory facilities of the Macenta Ebola Treatment Center in Guinea. As there is no universally accepted reference test for EVD diagnosis, clinically suspected EVD patients were confirmed based on the results of the reference molecular diagnostic test, the RealStar® Ebolavirus RT-PCR Kit 1.0 (Altona Diagnostics), authorized by the U.S. Food and Drug Administration under an Emergency Use Authorization. Archived EDTA plasma RNA extracts or oral swab RNA extracts, positive for EBOV genome detection by the RealStar Ebolavirus RT-PCR, were used for performance evaluation of the optimized *Zaire ebolavirus* RT-LAMP assay. Negative controls included RNA extracts from febrile patients negative for EBOV genome detection but diagnosed with one of the conditions that can present symptoms similar to EBOV infection. All EBOV-positive and -negative oral swab extracts were obtained from decedents with suspected EVD. The assay was performed blindly on the selected sample extract panel. Obtained results were collected at the end of each testing session using a standard report datasheet, and combined into a final database. Results obtained for the RT-LAMP assay were compared in a 2x2 table with results from the reference Altona RT-PCR assay, in order to estimate indices of sensitivity, specificity, and corresponding 97.5% confidence intervals (97.5% CI). Confidence intervals were calculated using binomial exact methods. Statistical analysis was carried out using STATA/SE version 12.0 (StataCorp, College Station, USA).

### 2. Results

The *L* gene, encoding the RNA-dependent RNA polymerase, was selected as target gene for the detection of the 5 known Ebola virus species and for the detection of Marburg virus. In addition, genes encoding the nucleoprotein were selected as target for the detection of Marburg virus and Lassa virus. For the detection of *Drosophila melanogaster sigma virus,* used as internal control, the G glycoprotein gene was selected as target gene. In addition, LAMP primers targeting the human *gapdh* (glyceraldehyde-3-phosphate dehydrogenase) housekeeping gene were designed as control for sample integrity.

RT-LAMP reaction conditions were optimized using different master mix compositions and different temperatures for the RT-LAMP reaction (Table I).

**Table I: Comparison of RT-LAMP assay efficiency (time-to-result) for different mastermix compositions**

| **Master Mix N°** | **Time to result (min)** |
|---|---|
| 1 | 17.24 ± 0.78 |
| 2 | 16.26 ± 2.02 |
| 3 | 27.94 ± 14.56 |
| 4 | 16.47 ± 1.32 |
| 5 | 12.42 ± 0.55 |
| 6 | 14.27 ± 8.36 |
| 7 | **10.55 ± 0.44** |
| 8 | 11.44 ± 1.83 |
| 9 | 11.94 ± 2.74 |
| 10 | 14.09 ± 2.00 |
| 11 | 11.60 ± 1.38 |
| 12 | 12.19 ± 0.43 |
| 13 | 13.19 ± 1.01 |
| 14 | 16.74 ± 1.74 |
| 15 | 11.70 ± 0.37 |
| 16 | 12.53 ± 0.49 |
| 17 | 14.26 ± 0.23 |
| 18 | 15.51 ± 2.90 |

Among the tested RT-LAMP mastermixes, mastermixes n° 7 and n°11 showed optimal amplification of target RNA as compared to other mastermix compositions. Consequently, mastermix n° 7 was used for all subsequent RT-LAMP reactions.

**Table II: Comparison of RT-LAMP assay efficiency (time-to-result) for different reaction temperatures**

| **Reaction temperature (°C)** | **Time to result (min)** |
|---|---|
| 63 | 12.05 ± 0.62 |
| 65 | 12.03 ± 2.19 |
| 68 | 10.11 ± 0.09 |

A reaction temperature of 68°C was identified as optimal (Table II); however, as little variation of efficiency was observed as compared to amplification at 65°C, a reaction temperature of 65°C was kept for all subsequent RT-LAMP assays.

**Table III: Comparison of RT-LAMP assay efficiency (time-to-result) for different Ebo/a Zaire virus primer sets**

| **Primer Set N°** | **Time to result (min)** |
|---|---|
| 1 | 9.36 ± 0.23 |
| 2 | 8.18 ± 2.02 |
| 3 | 6.36 ± 0.00 |

In addition, primer set n° 3 was identified as best performing for *Zaire ebolavirus* detection (Table III), and was therefore used for the analytical and clinical validation of the *Zaire ebolavirus* RT-LAMP assay.

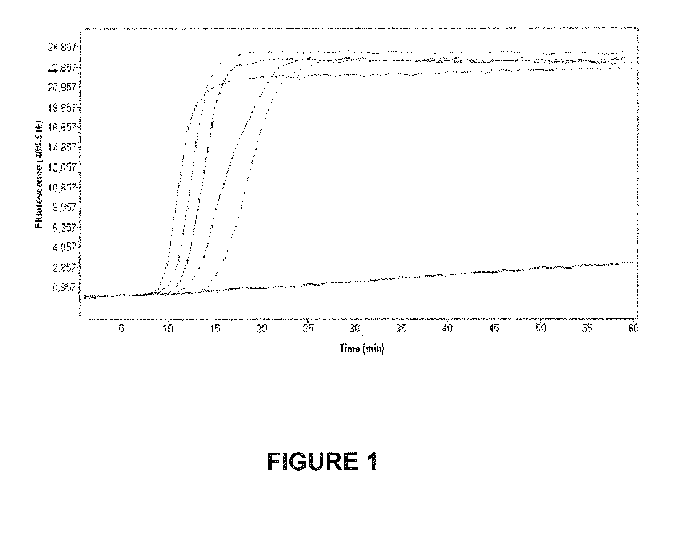

Serial dilutions of *Zaire ebolavirus* synthetic and genomic RNA were used to determine the analytical sensitivity of the *Zaire ebolavirus* RT-LAMP assay. Amplification curves obtained by RT-LAMP for 10-fold serial dilutions of *Zaire ebolavirus* synthetic RNA are shown in **Figure 1****.** The *Zaire ebolavirus* RT-LAMP assay demonstrated a limit of detection of 5 genome copies per reaction, with a time-to-result of less than 15 minutes.

The analytical specificity of the *Zaire ebolavirus* RT-LAMP assay was assessed using a panel of 27 genomic RNA and DNA extracts from closely-related viruses and from pathogens causing symptoms similar to those of EVD (Table IV).

**Table IV: Viral and bacterial strains analyzed by the Zaire ebolavirus RT-LAMP assay**

| **Agent** | **Species** | **Strain** |
|---|---|---|
| | *Adenovirus 2* | Unknown |
| | *Crimean-Congo Hemorrhagic Fever Virus* | Afg09-2990 / BNI |
| | *Crimean-Congo Hemorrhagic Fever Virus* | Kosovo Hoti / BNI |
| | *Chikungunya virus* | A26 Strain |
| | *Chikungunya virus* | Chik/06/21 La Reunion 2006 |
| | *Cytomegalovirus* | Unknown |
| | *Dengue virus 1* | VR344 (Thai 1958 strain) |
| | *Dengue virus 2* | VR345 (TH-36 strain) |
| | *Dengue virus 3* | VR216 (H87 strain) |
| | *Dengue virus 4* | VR217 (H241 strain) |
| | *Influenza A virus H3N2* | Flu/A/Panama/2007/1999 |
| | *Lassa virus* | Lib 1580/121 / BNI |
| | *Lassa virus* | Josiah /BNI |
| | *Marburg Virus* | Musoke /BNI |
| | *Measles Virus* | Schwartz |
| | *Rift Valley Fever Virus* | Strain ZH548 |
| | *Yellow fever virus 17D* | RKI #142/94/1 |
| | *Yellow fever virus Asibi* | AY640589.1 /RKI |
| | *Varicella-zoster virus* | Unknown |
| | *West Nile virus* | WN-143 |
| | *West Nile virus* | WN-56-2 Camargue 2002 |
| | *Zika virus* | MR766 |
| | *Zika virus* | 7630 Côte d'lvoire 1980 |
| Bacteria | *Bacillus subtilis* | 52.65 |
| | *Listeria monocytogenes* | CIP82.110T |
| | *Leptospira interrogans icterohaemorrhagiae* | CIP6.1204 |
| | *Neisseria meningitidis* | CIP73.10T |

None of the tested viral and bacterial RNA and DNA extracts were detected by the *Zaire Ebolavirus* RT-LAMP assay, confirming the specificity of the assay.

The RT-LAMP assay was evaluated using a panel of RNA extracts from 174 patient plasma samples including 87 samples from acute and convalescent phase patients with confirmed EVD diagnosis and 87 samples from febrile patients who presented with symptoms compatible for EVD. Patients with symptoms compatible to EVD included 12 (13.7%) patients diagnosed with typhoid fever and 11 (12.6%) patients diagnosed with malaria.

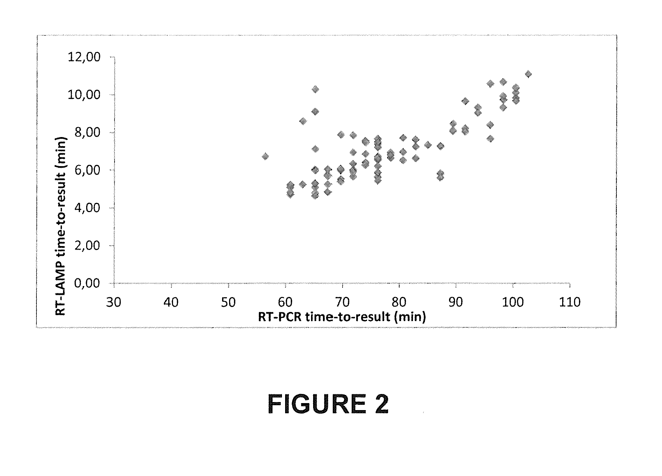

All 87 EBOV-positive RNA extracts were found positive by RT-LAMP. A comparison of the time-to-results observed between the reference RT-PCR and the RT-LAMP assay is shown for each sample from patients with confirmed EVD diagnosis in **Figure 2****.**

The time-to-result ranged from 4.66 to 11.09 minutes for the real-time RT-LAMP assay and from 56.40 to 102.60 minutes for the reference real-time RT-PCR assay. Among the 87 extracts from febrile patients negative for EBOV genome detection by the reference real-time RT-PCR, 3 were found positive by the RT-LAMP assay. Presence of *Zaire ebolavirus* genome was confirmed by testing the extracts again in duplicate using two different *Zaire ebolavirus* RT-LAMP primer sets. These samples were therefore classified as true positives for *Zaire ebolavirus* genome detection (false-negative for RT-PCR). When compared to the results of the reference *Zaire ebolavirus* genome detection method (real-time RT-PCR), the RT-LAMP assay demonstrated a clinical sensitivity of 100.0% (one-sided 97.5% CI = 96.0 to 100.0 %) and a clinical specificity of 100.0 % (one-sided 97.5% CI = 95.7 to 100.0 %) on plasma RNA extracts (Table V).

**Table V: Overall clinical performances of the Zaire ebolavirus RT-LAMP assay on patient plasma RNA extracts**

| Gold standard | Parameter | New test RT-LAMP |
|---|---|---|
| Reference test (RT-PCR) | No. of samples | 174 |
| | True positive | 90* |
| | False negative | 0 |
| | True negative | 84 |
| | False positive | 0 |
| | Sensitivity | 100.0% |
| | Sensitivity (97.5% CI) | (96.0 to 100.0 %)§ |
| | Specificity | 100.0% |
| | Specificity (97.5% CI) | (95.7 to 100.0 %)§ |

| | | |
|---|---|---|
| * Three samples with false negative results for the reference RT-PCR were classified as true positives after retesting § one-sided 97.5 % CI | | |

In addition, a panel of 50 oral swab RNA extracts from decedents, 27 of which were positive by the reference real-time RT-PCR, were tested by RT-LAMP. All 27 EBOV-positive RNA extracts were found positive by RT-LAMP. The time-to-result ranged from 6.26 to 13.45 minutes for the real-time RT-LAMP assay and from 60.80 to 93.80 minutes for the reference real-time RT-PCR assay. Among the 23 swab extracts negative for EBOV genome detection by the reference real-time RT-PCR, 2 were found positive by the RT-LAMP assay. Presence of *Zaire ebolavirus* genome was confirmed by testing the extracts again in duplicate using two different *Zaire ebolavirus* RT-LAMP primer sets. These samples were therefore also classified as true positives for Zaire ebolavirus genome detection. When compared to the results of the reference *Zaire ebolavirus* genome detection method (real-time RT-PCR), the RT-LAMP assay demonstrated a clinical sensitivity of 100.0% (one-sided 97.5% CI = (88.1 to 100.0 %) and a clinical specificity of 100.0 % (one-sided 97.5% CI = 83.9% to 100.0 %) on oral swab RNA extracts (Table VI).

**Table VI: Overall clinical performances of the Zaire ebolavirus RT-LAMP assay on RNA extracts from oral swabs collected from decedents**

| Gold standard | Parameter | New test RT-LAMP |
|---|---|---|
| Reference test (RT-PCR) | No. of samples | 50 |
| | True positive | 29* |
| | False negative | 0 |
| | True negative | 21 |
| | False positive | 0 |
| | **Sensitivity** | **100.0%** |
| | Sensitivity (97.5% CI) | (88.1 to 100.0 %)§ |
| | **Specificity** | **100.0%** |
| | Specificity (97.5% Cl) | (83.9 to 100.0 %)§ |

| | | |
|---|---|---|
| * Two samples with false negative results tor the reference RT-PCR were classified as true positives after retesting § one-sided 97.5 % CI | | |

### 3. Conclusions

In conclusion, the developed RT-LAMP method demonstrated equivalent analytical sensitivity and at least a 5 times faster time-to-result (15 minutes as compared to 80 minutes) as compared to the currently used reference real-time RT-PCR assay as well as other published Ebola virus qRT-PCR assays (16,17), thereby showing great potential for point-of-care applications. Moreover, the RT-LAMP assay demonstrated superior clinical performances as compared to the RT-PCR assay, probably due to a higher tolerance to inhibitors, making it highly suitable for accurate and reliable diagnostic applications.

The developed system, a highly accurate, fast, and easy-to-use molecular diagnostic test will be applicable not only in all well-equipped hospitals and field laboratories but also directly at the point-of-care in decentralized healthcare facilities in high-burden areas. Put together, this easily interpretable, rapid turn-around field tool will provide critical information to impact clinical management including triage, referral, treatment decisions, and decision to discharge. In addition to medical, national defense, and homeland security applications, the proposed technology is applicable in pharmaceutical, industrial, veterinary and environmental testing.

**Table VII: RT-LAMP assays primer list (SEQ ID NO: 31 to 162)**

| **Target species (Accession Nr)** | **Target gene** | **Target Region** | **Oligo Name** | **Sequence 5' to 3'** | **SEQ ID NO:** | **Primer position** |
|---|---|---|---|---|---|---|
| *Zaire ebolavirus* | *L* | | EBOV-ZAIRE-1-F3 | ATCCGACTCGCAATGTTC | 31 | 1703-1720 |
| | | | EBOV-ZAIRE-1-B3 | ACACCATAGCAACGGTTG | 32 | 1973-1956 |
| (NC_002549) | | SEQ ID NO: 3 | EBOV-ZAIRE-1-FIP | TTGCTCACGTTCCGTAACTACC-AACACTTTGTGAAGCTCTGT | 33 | 1803-1782, 1722-1741 |
| | | | EBOV-ZAIRE-1-BIP | TCAAGCATCATGGCACCACA-AATCAGTTACAAAGCTACTCCC | 34 | 1821-1840, 1897-1876 |
| | | | EBOV-ZAIRE-1-LoopF | GCTTTAGCAAGACCATCAGC | 35 | 1763-1744 |
| | | | EBOV-ZAIRE-1-LoopB | TGGTGAACATGCCACAGTTA | 36 | 1854-1873 |
| | *L* | | EBOV-ZAIRE-2-F3 | GAATGTAGGTAGAACCTTCGG | 37 | 1671-1691 |
| | | | EBOV-ZAIRE-2-B3 | ACACCATAGCAACGGTTG | 38 | 1973-1956 |
| | | | EBOV-ZAIRE-2-FIP | TTGCTCACGT7CCGTAACTACC-TTGTGAAGCTCTGTTAGCTG | 39 | 1803-1782, 1728-1747 |
| | | SEQ ID NO: 4 | EBOV-ZAIRE-2-BIP | TCAAGCATCATGGCACCACA-AATCAGTTACAAAGCTACTCCC | 40 | 1821-1840, 1897-1876 |
| | | | EBOV-ZAIRE-2-LoopF | GGAAATGCTTTAGCAAGACCAT | 41 | 1769-1748 |
| | | | EBOV-ZAIRE-2-LoopB | TGGTGAACATGCCACAGTTA | 42 | 1854-1873 |
| | *L* | | EBOV-ZAIRE-3-F3 | GAATGTAGGTAGAACCTTCGG | 43 | 1671-1691 |
| | | | EBOV-ZAIRE-3-B3 | CTCTAACTGTGGCATGTTCA | 44 | 1876-1857 |
| | | SEQ ID NO: 2 | EBOV-ZAIRE-3-FIP | TGCTTTAGCAAGACCATCAGCT-CTTATCCGACTCGCAATGT | 45 | 1764-1743, 1700-1718 |
| | | | EBOV-ZAIRE-3-BIP | GGTAGTTACGGAACGTGAGCAA-CCAAAATCATCACTTGTGTGG | 46 | 1782-1803, 1856-1836 |
| | | | EBOV-ZAIRE-3-LoopF | ACAGAGCTTCACAAAGTGTTTG | 47 | 1741-1720 |
| | | | EBOZAIRE-3-LoopB | ATTGCATCAAGCATCATGGC | 48 | 1815-1834 |
| *Taï Forest ebolavirus* | *L* | | EBOV-TAI-1-F3 | TCTCGCACAGAATTGTACTG | 49 | 5270-5289 |
| | | | EBOV-TAI-1-B3 | TGCTTCAATGCTGTGTTCT | 50 | 5598-5580 |
| | | SEQ ID NO: 8 | EBOV-TAI-1-FIP | CTATTCCCGTGAAGCGGCAATA-GACAACAGAGATTGTAAGGCT | 51 | 5428-5407, 5349-5369 |
| (NC_014372) | | | EBOV-TAI-1-BIP | GCTCGATGAAGTCCTTTGGGAA-GTAATGCACCCGAACCTT | 52 | 5448-5469, 5527-5510 |
| | | | EBOV-TAI-1-LoopF | TGTGGTGTCTGGTATTGCC | 53 | 5385-5403 |
| | | | EBOV-TAI-1-LoopB | TCTGCTATAACACTTGCCGAA | 54 | 5485-5505 |
| | *L* | | EBOV-TAI-2-F3 | GCCTGATCAATCACGACTT | 55 | 3531-3549 |
| | | | EBOV-TAI-2-B3 | TTACTCGTGCTTCTATGAAGG | 56 | 3745-3725 |
| | | SEQ ID NO: 6 | EBOV-TAI-2-FIP | GGCTGGCTGCCCAATCTTA-GGACAATTGGAGATGGCAT | 57 | 3618-3600, 3554-3572 |
| | | | EBOV-TAI-2-BIP | AAATGCCCTTCAGCAGCCTTA-GCTCCACCTTGAGTAACC | 58 | 3628-3648, 3701-3684 |
| | | | EBOV-TAI-2-LoopF | TCCTCTGTTCGAGATCCGATA | 59 | 3599-3579 |
| | | | EBOV-TAI-2-LoopB | CGTGAAGCAATTGAGTTGACAT | 60 | 3649-3670 |
| | *L* | | EBOV-TAI-3-F3 | AGAACTGCCATTACTGTCTTC | 61 | 4654-4674 |
| | | | EBOV-TAI-3-B3 | GATCGACTTCTCCAGTATGC | 62 | 4940-4921 |
| | | SEQ ID NO: 7 | EBOV-TAI-3-FIP | AACAATGCGATGACGTGGTGTA-TTCCACTGTGGGTCATCT | 63 | 4799-4778, 4718-4735 |
| | | | EBOV-TAI-3-BIP | CAACATGAGTCCTCCCACGATC-AATGTCTCCACTCGACTGT | 64 | 4801-4822, 4862-4844 |
| | | | EBOV-TAI-3-LoopF | ACTGTTGATCGGACTAGGACT | 65 | 4773-4753 |
| | | | EBOV-TAI-3-LoopB | GTTTGTGCTGCAGAAGCC | 66 | 4825-4842 |
| *Sudan ebolavirus* | *L* | | EBOV-SUD-1-F3 | CCTCTGCGAAGCATTACTT | 67 | 1728-1746 |
| | | | EBOV-SUD-1-B3 | CATGTAACATTGCGGAATTAGG | 68 | 2025-2004 |
| | | SEQ ID NO: 11 | EBOV-SUD-1-FIP | TCATCACTTGTATGGTGCCAGG-CAAGCAATATGATGGTTGTCAC | 69 | 1853-1832, 1772-1793 |
| (NC_006432) | | | EBOV-SUD-1-BIP | GCCACAGTTCGTGGAAGTAGTT-TGAAGGGAGCTGTGAATTC | 70 | 1867-1888, 1948-1930 |
| | | | EBOV-SUD-1-LoopF | ATGCTTGGTGAAGGAGGC | 71 | 1831-1814 |
| | | | EBOV-SUD-1-LoopB | AATACAATCTGGCCTTCAGGT | 72 | 1907-1927 |
| | *L* | | EBOV-SUD-2-F3 | AATTGCGATCAGCAGTCAT | 73 | 2204-2222 |
| | | SEQ ID NO: 12 | EBOV-SUD-2-B3 | TGCTGTCTTGAGTGATTGAG | 74 | 2460-2441 |
| | | | EBOV-SUD-2-FIP | GCCAAGCTAGCAGCCACT-CTAGAATCTAGTCCGAATGAGC | 75 | 2333-2316, 2263-2284 |
| | | | EBOV-SUD-2-BIP | GTCACAAGTGCCTGTGGGAT-GATAAAGCCTGAGTGTACGAA | 76 | 2338-235, 2400-2380 |
| | | | EBOV-SUD-2-LoopF | GCTGCATTGTCTTCTGCG | 77 | 2312-2295 |
| | | | EBOV-SUD-2-LoopB | ATTCCTCAAGCCTGATGAGAC | 78 | 2358-2378 |
| | *L* | | EBOV-SUD-3-F3 | AACGAATGAGGTTGTTGACA | 79 | 528-547 |
| | | | EBOV-SUD-3-B3 | AGTCTCCTGCGTTATACAGA | 80 | 847-828 |
| | | | EBOV-SUD-3-FIP | TGTGTGTCCTTGAATAGCCTCC-AACACAGCTAATGTTCCACA | 81 | 681-660, 604-623 |
| | | SEQ ID NO: 10 | EBOV-SUD-3-BIP | GTCTCTACAGCCGAGGTCCTTA-GGCTAACTCAGCAATCAGAA | 82 | 694-715, 774-755 |
| | | | EBOV-SUD-3-LoopF | TTAGGTTGGTACCAGTCAGTTG | 83 | 650-629 |
| | | | EBOV-SUD-3-LoopB | AAGGATCTTGTCACAAGTCGTT | 84 | 724-745 |
| *Bundibugyo ebolavirus* | *L* | | EBOV-BUN-1-F3 | ATTGCCACAATCACTCAAGA | 85 | 2433-2452 |
| | | | EBOV-BUN-1-B3 | TTGACCTAGATCGGTTCCTT | 86 | 2661-2642 |
| | | | EBOV-BUN-1-FIP | TGCCGTTCCTATACTAGCCAGA-CTGCTACCAGGATTGCTC | 87 | 2529-2508,2453-2470 |
| (NC_014373) | | SEQ ID NO: 15 | EBOV-BUN-1-BIP | TATACCCTTGCCGGGTGGTT-CAAGGTGGTGGTATTGGAG | 88 | 2561-2580,2632-2614 |
| | | | EBOV-BUN-1-LoopF | CCTGAAGGTCATCAAAGATTGC | 89 | 2503-2482 |
| | | | EBOV-BUN-1-LoopB | GCCGCATTCCATACATTCTTC | 90 | 2581-2601 |
| | *L* | | EBOV-BUN-2-F3 | TGTACCTTACATCGGGTCTC | 91 | 3570-3589 |
| | | | EBOV-BUN-2-B3 | GCTGTATTGGTCATTATACCGA | 92 | 3828-3807 |
| | | | EBOV-BUN-2-FIP | GCTTCACGTAAGGCAGCAGA-GAACAGAGGACAAGATTGGG | 93 | 3656-3637, 3590-3609 |
| | | SEQ ID NO: 16 | EBOV-BUN-2-BIP | CCAAGGTGGTGCCAATAGTGAT-AAGGATCTCCTGCACACT | 94 | 3690-3711, 3768-3751 |
| | | | EBOV-BUN-2-LoopF | TTAGGCTTGATTGCTGGCT | 95 | 3629-3611 |
| | | | EBOV-BUN-2-LoopB | TTGTAGAGGCACGAGTAAACC | 96 | 3728-3748 |
| | *L* | | EBOV-BUN-3-F3 | ACCGTTGTCATTGTTGTCA | 97 | 579-597 |
| | | | EBOV-BUN-3-B3 | TAGAGATTGTTTCAGGTTGTGG | 98 | 823-802 |
| | | | EBOV-BUN-3-FIP | GTGTGACCTTGAACCGCTTCT-GATTCCTCATGCAGCTAAGG | 99 | 677-657, 612-631 |
| | | SEQ ID NO:14 | EBOV-BUN-3-BIP | TGTCAGTTTCCACTGCAGATGT-GCTGCAATGAGTGTGGTAT | 100 | 686-707, 764-746 |
| | | | EBOV-BUN-3-LoopF | TGAAGATTGATGCGTGATACCA | 101 | 655-634 |
| | | | EBOV-BUN-3-LoopB | AAGGACATCATAACCTGTCGTT | 102 | 721-742 |
| *Reston ebolavirus* | *L* | | EBOV-RES-1-F3 | TTACTTGGCTCCATTATTCGG | 103 | 5922-5942 |
| | | | EBOV-RES-1-B3 | GTAGCATACTGTGCGATGT | 104 | 6140-6122 |
| | | SEQ ID NO: 20 | EBOV-RES-1-FIP | ATGGGCCGATCTCCTGTTAGTA-ACCGATTACATCAAGTGCC | 105 | 6042-6021, 5961-5979 |
| (NC_004161) | | | EBOV-RES-1-BIP | TCACAAGGCATGTCTTGGTGTT-GACTCAACCAGTACACGC | 106 | 6048-6069, 6121-6104 |
| | | | EBOV-RES-1-LoopF | AGGTACCATTCACTTGACCG | 107 | 5999-5980 |
| | | | EBOV-RES-1-LoopB | AAGCACAAGTCCAGCGAG | 108 | 6086-6103 |
| | *L* | | EBOV-RES-2-F3 | AGGCAATACTATCCTATGGACT | 109 | 4422-4443 |
| | | | EBOV-RES-2-B3 | CCTTCAAGCGGATATACTATCC | 110 | 4742-4721 |
| | | SEQ ID NO: 19 | EBOV-RES-2-FIP | TTAATCGGGACATCACACTGGC-ACCAGCTGCACAACTTAC | 111 | 4564-4543, 4484-4501 |
| | | | EBOV-RES-2-BIP | TGGCGGGACATCTGGAGA-CGATTGCTGTCCGAAGAA | 112 | 4596-4613, 4660-4643 |
| | | | EBOV-RES-2-LoopF | CACGGAGTGCTCGATGAG | 113 | 4519-4502 |
| | | | EBOV-RES-2-LoopB | CTGATGCTGCTCGACTGT | 114 | 4625-4642 |
| | *L* | | EBOV-RES-3-F3 | ACATGATGGTAGTAACTGAACG | 115 | 1775-1796 |
| | | | EBOV-RES-3-B3 | TTAACATTGTGAGGCGGATT | 116 | 2060-2041 |
| | | SEQ ID NO: 18 | EBOV-RES-3-FIP | ACTCCCTCGAACGGTAGCA-CCTTCTTCATCAGGCATCAT | 117 | 1881-1863, 1812-1831 |
| | | | EBOV-RES-3-BIP | CGCTATGAGTTCACTGCACCAT-AAGACATTACGCACACCAT | 118 | 1921-1942,1985-1967 |
| | | | EBOV-RES-3-LoopF | TCATCACTGGTGTGGTGC | 119 | 1850-1833 |
| | | | EBOV-RES-3-LoopB | TGAGTACTGCAACCATTGCT | 120 | 1947-1966 |
| *Marburg virus* | *L* | | MARV-L-1-F3 | CGAAGACAGTGATGGAGC | 121 | 786-803 |
| | | | MARV-L-1-B3 | CACTCAGCCTTATTGTCTGAA | 122 | 1049-1029 |
| (NC_001608) | | SEQ ID NO: 22 | MARV-L-1-FIP | CGTCCTCCAGGTACATGGC-CTCCTCTTGATGTGTTAGGTG | 123 | 880-862, 812-832 |
| | | | MARV-L-1-BIP | TTAGAACCCTTGTGTGTCAGCT-ATCATCTGTGATTGGAACCAG | 124 | 901-922, 980-960 |
| | | | MARV-L-1-LoopF | AATTTGGTCTCCCTGGTTGTAT | 125 | 858-837 |
| | | | MARV-L-1-LoopB | CACATGGCATCTTTACACCAAG | 126 | 932-953 |
| | *L* | | MARV-L-2-F3 | AGCAATCAATTCTGTCGGTT | 127 | 5746-5765 |
| | | | MARV-L-2-B3 | ACAACATTCTCGGTATTACCC | 128 | 5980-5960 |
| | | SEQ ID NO: 23 | MARV-L-2-FIP | CCCTTCGGCCAAACAAATTGC-GTCTCATCAATGCATTACAAGC | 129 | 5853-5833, 5776-5797 |
| | | | MARV-L-2-BIP | AAGGAAGTGGTGCTCGGTTAC-GTTGTGAATCCGTAGCCAA | 130 | 5855-5875, 5935-5917 |
| | | | MARV-L-2-LoopF | TTTACCTGGAGGCAAAAGATCA | 131 | 5823-5802 |
| | | | MARV-L-2-LoopB | TTGAAGTGGAAGGAAACGGAT | 132 | 5878-5898 |
| | *L* | | MARV-L-3-F3 | AGACAAGCAATTCCTTACTGG | 133 | 6442-6462 |
| | | | MARV-L-3-B3 | TCTCAAGTCTAGTAGAGGTGAA | 134 | 6684-6663 |
| | | | MARV-L-3-FIP | ACGATGGCAGAAAGATGACTCT-ATCCTGCTTCATTACACGAG | 135 | 6555-6534, 6485-6504 |
| | | | MARV-L-3-BIP | TCCATTCAGTGAAGGAAAGGCT-AAATGTTGTTTGCCTTGACG | 136 | 6564-6585, 6632-6613 |
| | | SEQ ID NO: 24 | MARV-L-3-LoopF | AGGGAAACCCAAAGTTAGGAAA | 137 | 6528-6507 |
| | | | MARV-L-3-LoopB | TTCACAAGGTCCAGTCTTATGT | 138 | 6590-6611 |
| | *NP* | | MARV-NP-1-F3 | AACAACCTCGAACATGGAC | 139 | 862-880 |
| | | | MARV-NP-1-B3 | GACGGCTAGTGTCTGACT | 140 | 1128-1111 |
| | | SEQ ID NO: 26 | MARV-NP-1-FIP | GTTCGCCAACATTGACACCAG-ATCCTCAGCTTTCAGCAATT | 141 | 961-941, 884-903 |
| | | | MARV-NP-1-BIP | CTACGAGAGGCGGCACATG-CCTCGGCAATAGCTTGAAT | 142 | 973-991,1051-1033 |
| | | | MARV-NP-1-LoopF. | AATGTACTGCCGTGTGCT | 143 | 938-921 |
| | | | MARV-NP-1-LoopB | GGCGACATGAACATCAGGA | 144 | 1013-1031 |
| | *NP* | | MARV-NP-2-F3 | CGGCAGTATTGGTGATGTAA | 145 | 1749-1768 |
| | | | MARV-NP-2-B3 | AATCTGCTTGAAGCTCCTTAG | 146 | 2038-2018 |
| | | | MARV-NP-2-FIP | AGGCGACAATTCATAGGCTTCC-CCTTCTTCACCATCTGCTC | 147 | 1860-1839, 1798-1816 |
| | | SEQ ID NO: 27 | MARV-NP-2-BIP | AGCAGAATTGGCCACAAAGAGT-GAGGATTTGTTTGCAGAAGATC | 148 | 1886-1907, 1966-1845 |
| | | | MARV-NP-2-LoopF | CTCATCCTTGTGTCTTCCTGAG | 149 | 1838-1817 |
| | | | MARV-NP-2-LoopB | GGTGACAAAGAAGGGTAGAACT | 150 | 1908-1929 |
| *Lassa virus* | *NP* | | LASV-NP-1-F3 | TCATCTGAGGTTAGAGTCCC | 151 | 293-274 |
| | | | LASV-NP-1-B3 | GAATTATCTGGTTACTGCTCCA | 152 | 55-76 |
| (NC_004296) | | | LASV-NP-1-FIP | CGCAAGGAGAGAAGGGATGAC-ACAAGATTGTTGACCGCTT | 153 | 163-183, 233-215 |
| | | SEQ ID NO: 29 | LASV-NP-1-BIP | GACTTCGGAGAAGTCAAGTCCA-ACATCAAACTACAGGTGGTG | 154 | 141-120, 77-96 |
| | | | LASV-NP-1-LoopF | AACGGTTGAGGGACCTAAATC | 155 | 194-214 |
| | | | LASV-NP-1-LoopB | AAAGAGCCTGGGCATCTTT | 156 | 115-97 |
| | *NP* | | LASV-NP-2-F3 | CCTGAACTTGGTTGATAGAGG | 157 | 1223-1203 |
| | | | LASV-NP-2-B3 | AAAGCTGACAGCAACAATTC | 158 | 1036-1055 |
| | | SEQ ID NO: 30 | LASV-NP-2-FIP | GGATGCAATGCTGCAACTTGA-TTCAGGTCTTCCTTCAATGTC | 159 | 1122-1142, 1185-1165 |
| | | | LASV-NP-2-BIP | TTGAGGGTCATCAGCTGAGAAT-GTAAATCCCTGCAGTCGG | 160 | 1100-1121, 1058-1075 |
| | | | LASV-NP-2-LoopF | CCCAAATGCTAAGACCTGGA | 161 | 1143-1162 |
| | | | LASV-NP-2-LoopB | TAAGCCCAGCGGTAAACC | 162 | 1096-1079 |
| *Homo sapiens* | *GAPDH* | | HUM-GAPDH-1-F3 | TCTTCCAGGAGCGAGATC | 171 | 227-244 |
| | | | HUM-GAPDH-1-B3 | GTCATGGATGACCTTGGC | 172 | 498-481 |
| | | | HUM-GAPDH-1-FIP | GCAAATGAGCCCCAGCCTTGAGTACGTCGTGGAGTC | 173 | 337-320, 276-293 |
| (NM_002046.5) | | SEQ ID NO: 165 | HUM-GAPDH-1-BIP | TGTTCGTCATGGGTGTGAACCGAGGCATTGCTGATGATCT | 174 | 389-409, 452-434 |
| | | | HUM-GAPDH-1-LoopF | CTCCATGGTGGTGAAGACG | 175 | 318-300 |
| | | | HUM-GAPDH-1- LoopB | TGAGAAGTATGACAACAGCCTC | 176 | 411-432 |
| | *GAPDH* | | HUM-GAPDH-2-F3 | ACGGATTTGGTCGTATTGG | 177 | 26-44 |
| | | | HUM-GAPDH-2-B3 | GACTCCACGACGTACTCA | 178 | 293-276 |
| | | SEQ ID NO: 164 | HUM-GAPDH-2-FIP | TGGAATTTGCCATGGGTGGAATGTTGCCATCAATGACCC | 179 | 170-150, 90-107 |
| | | | HUM-GAPDH-2-BIP | GCACCGTCAAGGCTGAGAAGATCTCGCTCCTGGAAGA | 180 | 173-191, 244-227 |
| | | | HUM-GAPDH-2-LoopF | ACCATGTAGTTGAGGTCAATGA a | 181 | 131-110 |
| | | | HUM-GAPDH-2- LoopB | ATCAATGGAAATCCCATCACCA | 182 | 205-226 |
| | *GAPDH* | | HUM-GAPDH-3-F3 | CATGTTCGTCATGGGTGT | 183 | 387-404 |
| | | | HUM-GAPDH-3-B3 | GATGATGTTCTGGAGAGCC | 184 | 621-603 |
| | | | HUM-GAPDH-3-FIP | GGTGCTAAGCAGTTGGTGGTATGAGAAGTATGACAACAGCC | 185 | 476-457, 410-430 |
| | | SEQ ID NO: 166 | HUM-GAPDH-3-BIP | GCCAAGGTCATCCATGACAACTCAGTGATGGCATGGACTG | 186 | 481-502, 547-530 |
| | | | HUM-GAPDH-3-LoopF | AGGCATTGCTGATGATCTTGA | 187 | 451-431 |
| | | | HUM-GAPDH-3- LoopB | GGTATCGTGGAAGGACTCATG | 188 | 505-525 |
| *Drosophila melanogaster sigma virus* | G | | SIGV-G-1-F3 | GGACCATTCATGACGATATGAT | 189 | 584-605 |
| | | | SIGV-G-1-B3 | ACTTGGAATGTCACAGAGTTAG | 190 | 869-848 |
| | | SEQ ID NO: 169 | SIGV-G-1-FIP | GACTTCCAGCATCGGCTCC-CTGGGTGGCAACAAAGTAT | 191 | 717-699, 644-662 |
| | | | SIGV-G-1-BIP | GGCTCCATCCACATCGAGG-CCTATCTCACCACAGAAGGA | 192 | 718-736, 794-775 |
| (GQ375258) | | | SIGV-G-1-LoopF | CTCTCCATACAGCCGAGATG | 193 | 696-677 |
| | | | SIGV-G-1- LoopB | CAGGGACAAGAACCTCACTTTA | 194 | 741-762 |
| | G | | SIGV-G-2-F3 | AAACTGCCGTTGTGGAAA | 195 | 908-925 |
| | | | SIGV-G-2-B3 | GTGTTCAGTGTTACTGTGTCTA | 196 | 1148-1127 |
| | | | SIGV-G-2-FIP | AGAGGTCGGAAGCCCAGAT-AGCTCACTGTCAACATGATG | 197 | 1023-1005, 947-966 |
| | | SEQ ID NO: 170 | SIGV-G-2-BIP | TCAAGTGCAGGAGGGACCT-GTATTGGTACATTCCGACACT | 198 | 1047-1065,1125-1105 |
| | | | SIGV-G-2-LoopF | GAGATGGTCTCTTGACACTTCA | 199 | 995-974 |
| | | | SIGV-G-2- LoopB | GAGAGAAGGGTGTCTTGTACC | 200 | 1081-1102 |
| | G | | SIGV-G-3-F3 | CAAGAAGCACCAGATCCTG | 201 | 204-222 |
| | | | SIGV-G-3-B3 | CCAACATTACAGGATGGTCTT | 202 | 517-497 |
| | | SEQ ID NO: 168 | SIGV-G-3-FIP | GCGGAGAGATGGAGTGTGAAAT-ATGCCATAAGGTGAAGTATGAG | 203 | 361-340, 282-303 |
| | | | SIGV-G-3-BIP | AGAGCACCAATTGGGCACC-GAGTTCCAAGAACAGTCCTC | 204 | 399-417, 461-442 |
| | | | SIGV-G-3-LoopF | TACCATGGCATATCACAGATGG | 205 | 326-305 |
| | | | SIGV-G-3- LoopB | ATGTCTCGCTGAGCTTTCC | 206 | 419-437 |

## Claims

1. A method of detecting a Hemorrhagic Fever Virus (HFV) in a sample, comprising:
a) subjecting said sample to an isothermal nucleic acid amplification reaction using at least one pair of oligonucleotide primers for amplifying a target sequence of a HFV nucleic acid, wherein the target sequence is located within a gene of the HFV nucleic acid which codes for a protein of the viral ribonucleocapsid, and
b) detecting the presence of an amplification product for said target sequence.

2. The method according to claim 1, wherein the HFV is selected from the group consisting of: Ebola virus, Lassa virus and Marburg virus.

3. The method according to claim 2, wherein the Ebola virus is chosen from Ebola-Zaire, Ebola-Taï Forest, Ebola-Sudan, Ebola-Bundibugyo and Ebola-Reston.

4. The method according to any one of claims 1 to 3, wherein the target sequence is located within the L or NP gene.

5. The method according to claim 4, wherein the target sequence is located within a region of the L or NP gene selected from the group consisting of: a sequence of any of SEQ ID NO: 2-4, 6-8, 10-12, 14-16, 18-20, 22-24, 26-27 and 29-30 and a sequence comprising any of SEQ ID NO:2-4, 6-8, 10-12, 14-16, 18-20, 22-24, 26-27 and 29-30 and up to 20 consecutive nucleotides of 5' and/or 3' flanking sequence from said L or NP gene.

6. The method according to any one of claims 1 to 5, wherein the oligonucleotide primers are selected from the group consisting of: the sequences of 5 to 100 nucleotides, preferably 15 to 60 nucleotides having 80% to 100% identity, preferably at least 85%, 90%, 95% or 98% identity with any of SEQ ID NO: 2-4, 6-8, 10-12, 14-16, 18-20, 22-24, 26-27 and 29-30.

7. The method according to claim 6, wherein the primers are selected from the group consisting of the sequences SEQ ID NO: 31 to 162.

8. The method according to any one of claims 1 to 7, wherein the isothermal amplification is Loop-mediated Amplification (LAMP) or Reverse-transcription-Loop-mediated Amplification (RT-LAMP).

9. The method according to claim 8, which is performed with a combination of two or three primer pairs selected from the group consisting of: SEQ ID NO: 31-32 and SEQ ID NO: 33-34, and optionally, SEQ ID NO: 35-36; SEQ ID NO: 37-38, and SEQ ID NO: 39-40, and optionally, SEQ ID NO: 41-42; SEQ ID NO: 43-44 and SEQ ID NO: 45-46, and optionally, SEQ ID NO: 47-48; SEQ ID NO:49-50 and SEQ ID NO:51-52, and optionally, SEQ ID NO:53-54; SEQ ID NO:55-56 and SEQ ID NO:57-58, and optionally, SEQ ID NO:59-60; SEQ ID NO:61-62 and SEQ ID NO:63-64, and optionally, SEQ ID NO:65-66; SEQ ID NO:67-68 and SEQ ID NO:69-70, and optionally, SEQ ID NO:71-72; SEQ ID NO:73-74 and SEQ ID NO:75-76, and optionally, SEQ ID NO:77-78; SEQ ID NO:79-80 and SEQ ID NO:81-82, and optionally, SEQ ID NO:83-84; SEQ ID NO:85-86 and SEQ ID NO:87-88, and optionally, SEQ ID NO:89_90; SEQ ID NO:91-92 and SEQ ID NO:93-94, and optionally, SEQ ID NO:95-96; SEQ ID NO:97-98 and SEQ ID NO:99-10, and optionally, SEQ ID NO:101-102; SEQ ID NO:103-104and SEQ ID NO:105-106, and optionally, SEQ ID NO:107-108; SEQ ID NO:109-110 and SEQ ID NO:111-112, and optionally, SEQ ID NO:113-114; SEQ ID NO:115-116 and SEQ ID NO:117-118, and optionally, SEQ ID NO:119-120; SEQ ID NO:121-122 and SEQ ID NO:123-124, and optionally, SEQ ID NO:125-126; SEQ ID NO:127-128 and SEQ ID NO:129-130, and optionally, SEQ ID NO:131-132; SEQ ID NO:133-134 and SEQ ID NO:135-136, and optionally, SEQ ID NO:137-138; SEQ ID NO:139-140 and SEQ ID NO:141-142, and optionally, SEQ ID NO:143-144; SEQ ID NO:145-146 and SEQ ID NO:147-148, and optionally, SEQ ID NO:149-150; SEQ ID NO:151-152 and SEQ ID NO:153-154, and optionally, SEQ ID NO:155-156; SEQ ID NO:157-158 and SEQ ID NO:159-160 and optionally, SEQ ID NO:161-162.

10. The method according to any one of claims 1 to 9, wherein the detection in step b) is fluorescence, bioluminescence, colorimetry, turbidimetry, immunoenzymatic or electrochemical detection.

11. The method according to any one of claims 1 to 10, which comprises assaying multiple samples simultaneously or multiple target sequences simultaneously on the same sample.

12. The method according to claim 11, which is for the differential diagnosis of Viral Hemorrhagic fevers or HFV subtype or species determination.

13. A kit for the detection of an HFV, comprising at least one pair of oligonucleotide primers for amplifying a target sequence of an HFV nucleic acid as defined in any one of claims 6, 7 and 9, and optionally reagents for the isothermal amplification of the target sequence and/or the detection of the amplification product.

14. The kit according to claim 13, wherein the primers and optional reagents are in lyophilised form.

15. A pair of oligonucleotide primers as defined in claim 6 or claim 7.
